(19) Europäisches Patentamt
European Patent Office
Office européen des brevets

(11) **EP 4 806 514 A2**

(12) **EUROPEAN PATENT APPLICATION**

(43) Date of publication:
**16.09.2026 Bulletin 2026/38**

(21) Application number: **26193757.7**

(22) Date of filing: **20.09.2024**

(51) International Patent Classification (IPC):
***A61P 35/00*** *(2006.01)*

(52) Cooperative Patent Classification (CPC):
**A61P 35/00; A61K 47/68031; A61K 47/68037; A61K 47/6849; A61K 47/6851; A61K 47/6889; C07K 16/28;** A61K 2039/505; C07K 2317/24; C07K 2317/33; C07K 2317/565; C07K 2317/567; C07K 2317/73; C07K 2317/77; C07K 2317/92

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:
**22.09.2023 PCT/CN2023/120835**
**18.12.2023 PCT/CN2023/139628**
**22.12.2023 PCT/CN2023/141205**
**18.04.2024 PCT/CN2024/088670**
**02.09.2024 PCT/CN2024/116347**

(62) Document number(s) of the earlier application(s) in accordance with Art. 76 EPC:
**24867586.0 / 4 780 856**

(71) Applicant: **Lepu Biopharma Co., Ltd.**
**Shanghai 201112 (CN)**

(72) Inventors:
- **WU, Qi**
  **Shanghai 201112 (CN)**
- **ZENG, Peng**
  **Shanghai 201112 (CN)**
- **YANG, Yuanyuan**
  **Shanghai 201112 (CN)**
- **CUI, Feifei**
  **Shanghai 201112 (CN)**
- **JIANG, Wenqing**
  **Shanghai 201112 (CN)**
- **FANG, Lei**
  **Shanghai 201112 (CN)**

(74) Representative: **Gleiss Große Schrell und Partner mbB**
**Leitzstraße 45**
**70469 Stuttgart (DE)**

Remarks:
•The complete document including Reference Table(s) and the Sequence Listing(s) can be downloaded from the EPO website
•This application was filed on 22.07.2026 as a divisional application to the application mentioned under INID code 62.

# (54) ANTI-CDH17 ANTIBODIES AND USES THEREOF

(57) Antibodies having highly selective specificity to the human CDH17 protein are identified that are useful for treating cancer. The antibodies can be used to prepare antibody-drug conjugates, which exhibit efficient *in vitro* and *in vivo* tumor cell killings.

EP 4 806 514 A2

## Description

## BACKGROUND

Gastrointestinal (GI) cancers, including colorectal cancer (CRC), gastric cancer (GC), pancreatic cancer (PC), esophageal cancer (EC), liver cancer, biliary cancer and neuroendocrine tumors (NETs), are the leading cause of cancer mortality worldwide, with significant associated morbidity. Colorectal cancer (CRC) alone accounts for approximately 10% of all cancer diagnoses and is the second leading cause of cancer death worldwide. In China, liver and stomach cancers are among the most lethal malignancies in the world, accounting for more than half of all diagnosed cases and causing more than 1.42 million deaths worldwide each year, yet there is no effective therapy. If caught early, radical approaches can lead to better outcomes, compared to less than 15% in advanced disease. Unfortunately, and very often, the majority of GI cancers is asymptomatic and diagnosed at very advanced stages when presented to the clinic. Therefore, in addition to early detection, research into different approaches to treating GI cancer at both early and late stages is needed to improve outcomes.

Cadherin 17 (CDH17), also known as liver-intestine cadherin (LI-cadherin), is a distinctive member of the cadherin superfamily. It is very similar to cadherin 16 (CDH16) and they form a subgroup called 7D-domain cadherins. Unlike classical cadherins with five cadherin domains and a cytoplasmic tail of more than 100 aa, CDH17 has a long extracellular domain (seven cadherin domains) and a short cytoplasmic tail (20 aa residues). Under physiological conditions, the expression of CDH17 in humans and mice is mainly restricted to epithelial cells in the intestine tissues and pancreatic ducts, but not in vital organs such as the liver, stomach, heart, lung and brain. Functionally, CDH17 is involved in intercellular adhesion to maintain tissue integrity and water absorption by regulating the intercellular cleft in a $Ca^{2+}$-dependent manner. Pathophysiologically, the expression of CDH17 has been extensively studied in various cancers of the digestive system. Its expression is upregulated in gastric cancer (GC), colorectal cancer (CRC), esophageal cancer (EC), pancreatic cancer (PC) and neuroendocrine tumors (NETs). Thus, CDH17 is considered as a cancer biomarker for prognosis and an oncogene for cancer intervention.

The distinct expression pattern of CDH17 in GI cancer has enabled the development of therapeutics that use CDH17 to specifically target GI cancer cells. Currently, multiple active clinical studies in GI cancers are evaluating these CDH17-specific drugs. Several types of modalities targeting CDH17 including bispecific Ab (BsAb), chimeric antigen receptor T cell therapy (CAR-T) are currently under clinical or preclinical investigation. BI905711 is a tetravalent bispecific antibody that cross-links TRAILR2 with CDH17. These cross-linking drives CDH17-dependent TRAILR2 oligomerization, leading to caspase activation and eventual apoptosis, inhibiting GI tumor growth. BI905711 exhibited manageable safety in patients (pts) with advanced GI cancers in Phase Ia/b study (NCT04137289). Stable disease (SD) was 27% (13 pts achieved SD) and 8 pts were progression-free for ≥4 months (PFS4). In pts with CRC, 6 pts achieved SD and 3 had PFS4. Among non-CRC pts, 7 pts achieved SD (PDAC: n = 6) and 5 had PFS4 (PDAC: n = 4). Other CDH17-based T cell engagers including ARB202 from Arbele also showed promising anti-tumor efficacy in preclinical studies and the Phase I clinical trial is currently enrolling patients in Australia and Hong Kong (NCT05411133). CHM-2101 is an optimized 3rd generation CAR-T cell therapy that is the first to target CDH17. Preclinical evidence of CHM-2101 have demonstrated a unique potency for solid tumors with complete eradication of tumor cells with no relapse. Preclinical studies also demonstrated no toxicity to normal tissues. Overall, exploring different drug types targeting CDH17 will hopefully bring more choices for GI cancer treatment.

## SUMMARY

The present disclosure provides anti-CDH17 antibodies or antigen-binding fragments thereof that exhibit efficient binding activity to human CDH17 protein or CDH17 expressing tumor cells, having different epitopes. Antibodies of the CDH17 membrane-distal binders also exhibit efficient internalization rate on CDH17 expressing cells and subsequently demonstrate efficient *in vitro* and *in vivo* tumor cell killing, particularly when constructed as antibody-drug conjugates (ADCs).

The present disclosure provides an antibody or antigen-binding fragment thereof having specificity to a human Cadherin 17 (CDH17) protein, wherein the antibody or antigen-binding fragment thereof comprises a heavy chain variable region (VH) comprising heavy chain complementarity determining regions HCDR1, HCDR2, and HCDR3, and a light chain variable region (VL) comprising light chain complementarity determining regions LCDR1, LCDR2, and LCDR3, wherein the HCDR1, HCDR2, HCDR3, LCDR1, LCDR2, and LCDR3 respectively, comprise the amino acid sequences of (a) SEQ ID NO:1-6; (b) SEQ ID NO:9-14; (c) SEQ ID NO:17-21 and any one of SEQ ID NO:22 and 217-221; (d) SEQ ID NO:25, SEQ ID NO:26 or 245, SEQ ID NO:27, any one of SEQ ID NO:28, 246 and 247, SEQ ID NO:29 and any one of SEQ ID NO:30 and 222-230; (e) SEQ ID NO:33-38; (f) SEQ ID NO:41-46; (g) SEQ ID NO:49, 50, 51, any one of SEQ ID NO:52 and 239-241, any one of SEQ ID NO:53 and 242-244, and SEQ ID NO:54; (h) SEQ ID NO:57-62; (i) SEQ ID NO:65-70; (j) SEQ ID NO:73-78; (k) SEQ ID NO:81-86; (l) SEQ ID NO:89-94; (m) SEQ ID NO:97-102; or (n) SEQ ID NO:105-110.

In certain embodiments, the HCDR1 comprises the amino acid sequence of SEQ ID NO:17, the HCDR2 comprises the amino acid sequence of SEQ ID NO:18, the HCDR3 comprise the amino acid sequence of SEQ ID NO:19, the LCDR1 comprises the amino acid sequence of SEQ ID NO:20, the LCDR2 comprises the amino acid sequence of SEQ ID NO:21, and the LCDR3 comprises an amino acid sequence selected from the group consisting of SEQ ID NO:217-221.

In certain embodiments, the HCDR1 comprises the amino acid sequence of SEQ ID NO:25, the HCDR2 comprises the amino acid sequence of SEQ ID NO:245, the HCDR3 comprise the amino acid sequence of SEQ ID NO:27, the LCDR1 comprises the amino acid sequence of SEQ ID NO:246 or 247, the LCDR2 comprises the amino acid sequence of SEQ ID NO:29, and the LCDR3 comprises an amino acid sequence selected from the group consisting of SEQ ID NO:222-230.

In certain embodiments, the HCDR1 comprises the amino acid sequence of SEQ ID NO:25, the HCDR2 comprises the amino acid sequence of SEQ ID NO:26, the HCDR3 comprise the amino acid sequence of SEQ ID NO:27, the LCDR1 comprises the amino acid sequence of SEQ ID NO:28, the LCDR2 comprises the amino acid sequence of SEQ ID NO:29, and the LCDR3 comprises an amino acid sequence selected from the group consisting of SEQ ID NO:30.

In certain embodiments, the HCDR1 comprises the amino acid sequence of SEQ ID NO:49, the HCDR2 comprises the amino acid sequence of SEQ ID NO:50, the HCDR3 comprise the amino acid sequence of SEQ ID NO:51, the LCDR1 comprises an amino acid sequence selected from the group consisting of SEQ ID NO:239-241, the LCDR2 comprises an amino acid sequence selected from the group consisting of SEQ ID NO:53 and 242-244, and the LCDR3 comprises the amino acid sequence of SEQ ID NO:54.

In certain embodiments, the HCDR1 comprises the amino acid sequence of SEQ ID NO:49, the HCDR2 comprises the amino acid sequence of SEQ ID NO:50, the HCDR3 comprise the amino acid sequence of SEQ ID NO:51, the LCDR1 comprises an amino acid sequence selected from the group consisting of SEQ ID NO:52 and 239-241, the LCDR2 comprises an amino acid sequence selected from the group consisting of SEQ ID NO:242-244, and the LCDR3 comprises the amino acid sequence of SEQ ID NO:54.

In certain embodiments, the antibody is a chimeric antibody or a humanized antibody.

In certain embodiments, (a) the VH comprises the amino acid sequence of SEQ ID NO: 7, and the VL comprises the amino acid sequence of SEQ ID NO: 8;

(b) the VH comprises the amino acid sequence of SEQ ID NO: 15, and the VL comprises the amino acid sequence of SEQ ID NO: 16;

(c) the VH comprises an amino acid sequence selected from the group consisting of SEQ ID NO: 23 and 125-132, and the VL comprises an amino acid sequence selected from the group consisting of SEQ ID NO: 24, 134-137 and 203-207;

(d) the VH comprises an amino acid sequence selected from the group consisting of SEQ ID NO: 31, 113-119 and 248, and the VL comprises an amino acid sequence selected from the group consisting of SEQ ID NO: 32, 121-123, 201-202, 208-216, and 249-250;

(e) the VH comprises an amino acid sequence selected from the group consisting of SEQ ID NO: 39 and 139-140, and the VL comprises an amino acid sequence selected from the group consisting of SEQ ID NO: 40, 142-145 and 147;

(f) the VH comprises the amino acid sequence of SEQ ID NO: 47, and the VL comprises the amino acid sequence of SEQ ID NO: 48;

(g) the VH comprises an amino acid sequence selected from the group consisting of SEQ ID NO: 55, 149-152, and 154, and the VL comprises an amino acid sequence selected from the group consisting of SEQ ID NO: 56, 156-158, 160, and 232-238;

(h) the VH comprises the amino acid sequence of SEQ ID NO: 63, and the VL comprises the amino acid sequence of SEQ ID NO: 64;

(i) the VH comprises the amino acid sequence of SEQ ID NO: 71, and the VL comprises the amino acid sequence of SEQ ID NO: 72;

(j) the VH comprises the amino acid sequence of SEQ ID NO: 79, and the VL comprises the amino acid sequence of SEQ ID NO: 80;

(k) the VH comprises the amino acid sequence of SEQ ID NO: 87, and the VL comprises the amino acid sequence of SEQ ID NO: 88;

(l) the VH comprises an amino acid sequence selected from the group consisting of SEQ ID NO: 95 and 162-165, and the VL comprises an amino acid sequence selected from the group consisting of SEQ ID NO: 96, 167-169 and 171; (m) the VH comprises an amino acid sequence selected from the group consisting of SEQ ID NO: 103 and 173-176, and the VL comprises an amino acid sequence selected from the group consisting of SEQ ID NO: 104, 178-181 and 183-184; or

(n) the VH comprises an amino acid sequence selected from the group consisting of SEQ ID NO: 111, 186-189 and 191, and the VL comprises an amino acid sequence selected from the group consisting of SEQ ID NO: 112, 193-196 and 198-199.

In certain embodiments, the antibody or antigen-binding fragment thereof provided herein comprises a VH and a VL that respectively comprise the amino acid sequences selected from the group consisting of (a) SEQ ID NO: 113 and 121; (b) SEQ ID NO: 113 and 122; (c) SEQ ID NO: 113 and 123; (d) SEQ ID NO: 114 and 121; (e) SEQ ID NO: 114 and 122; (f) SEQ ID NO: 114 and 123; (g) SEQ ID NO: 115 and 121; (h) SEQ ID NO: 115 and 122; (i) SEQ ID NO: 115 and 123; (j) SEQ ID NO: 116 and 121; (k) SEQ ID NO: 116 and 122; (l) SEQ ID NO: 116 and 123; (m) SEQ ID NO: 117 and 121; (n) SEQ ID NO: 117 and 122; (o) SEQ ID NO: 118 and 121; (p) SEQ ID NO: 119 and 122; (q) SEQ ID NO: 113 and 201; (r) SEQ ID NO: 114 and 201; (s) SEQ ID NO: 115 and 201; (t) SEQ ID NO: 116 and 201; (u) SEQ ID NO: 113 and 202; (v) SEQ ID NO: 114 and 202; (w) SEQ ID NO: 115 and 202; (x) SEQ ID NO:116 and 208; (y) SEQ ID NO:116 and 209; (z) SEQ ID NO:116 and 210; (aa) SEQ ID NO:116 and 211; (ab) SEQ ID NO:116 and 212; (ac) SEQ ID NO:116 and 213; (ad) SEQ ID NO:116 and 214; (ae) SEQ ID NO:116 and 215; (af) SEQ ID NO:116 and 216; (ag) SEQ ID NO:248 and 123; (ah) SEQ ID NO:116 and 249; (ai) SEQ ID NO:248 and 249; or (aj) SEQ ID NO:249 and 250.

In certain embodiments, the antibody or antigen-binding fragment thereof provided herein comprises a VH and a VL that respectively comprise the amino acid sequences selected from the group consisting of (a) SEQ ID NO: 125 and 134; (b) SEQ ID NO: 125 and 135; (c) SEQ ID NO: 125 and 136; (d) SEQ ID NO: 126 and 134; (e) SEQ ID NO: 126 and 135; (f) SEQ ID NO: 126 and 136; (g) SEQ ID NO: 127 and 134; (h) SEQ ID NO: 127 and 135; (i) SEQ ID NO: 127 and 136; (j) SEQ ID NO: 128 and 134; (k) SEQ ID NO: 128 and 135; (l) SEQ ID NO: 128 and 136; (m) SEQ ID NO: 129 and 134; (n) SEQ ID NO: 129 and 135; (o) SEQ ID NO: 129 and 136; (p) SEQ ID NO: 130 and 134; (q) SEQ ID NO: 130 and 135; (r) SEQ ID NO: 130 and 136; (s) SEQ ID NO: 131 and 134; (t) SEQ ID NO: 125 and 137; (u) SEQ ID NO: 126 and 137; (v) SEQ ID NO: 127 and 137; (w) SEQ ID NO: 128 and 137; (x) SEQ ID NO: 129 and 137; (y) SEQ ID NO: 132 and 137; (z) SEQ ID NO:131 and 203; (aa) SEQ ID NO:131 and 204; (ab) SEQ ID NO:131 and 205; (ac) SEQ ID NO:131 and 206; or (ad) SEQ ID NO:131 and 207.

In certain embodiments, the antibody or antigen-binding fragment thereof provided herein comprises a VH and a VL that respectively comprise the amino acid sequences selected from the group consisting of (a) SEQ ID NO: 139 and 142; (b) SEQ ID NO: 139 and 143; (c) SEQ ID NO: 139 and 144; (d) SEQ ID NO: 139 and 145; (e) SEQ ID NO: 139 and 147; (f) SEQ ID NO: 140 and 142; (g) SEQ ID NO: 140 and 143; (h) SEQ ID NO: 140 and 144; (i) SEQ ID NO: 140 and 145; or (j) SEQ ID NO: 140 and 147.

In certain embodiments, the antibody or antigen-binding fragment thereof provided herein comprises a VH and a VL that respectively comprise the amino acid sequences selected from the group consisting of (a) SEQ ID NO: 149 and 156; (b) SEQ ID NO: 149 and 157; (c) SEQ ID NO: 149 and 158; (d) SEQ ID NO: 150 and 156; (e) SEQ ID NO: 150 and 157; (f) SEQ ID NO: 150 and 158; (g) SEQ ID NO: 151 and 156; (h) SEQ ID NO: 151 and 157; (i) SEQ ID NO: 151 and 158; (j) SEQ ID NO: 152 and 156; (k) SEQ ID NO: 149 and 160; (l) SEQ ID NO: 152 and 160; (m) SEQ ID NO: 154 and 156; (n) SEQ ID NO: 154 and 160; (o) SEQ ID NO:152 and 232; (p) SEQ ID NO:152 and 233; (q) SEQ ID NO:152 and 234; (r) SEQ ID NO:152 and 235; (s) SEQ ID NO:152 and 236; (t) SEQ ID NO:152 and 237 or (u) SEQ ID NO:152 and 238.

In certain embodiments, the antibody or antigen-binding fragment thereof provided herein comprises a VH and a VL that respectively comprise the amino acid sequences selected from the group consisting of (a) SEQ ID NO: 162 and 167; (b) SEQ ID NO: 162 and 168; (c) SEQ ID NO: 162 and 169; (d) SEQ ID NO: 163 and 167; (e) SEQ ID NO: 163 and 168; (f) SEQ ID NO: 163 and 169; (g) SEQ ID NO: 164 and 167; (h) SEQ ID NO: 164 and 168; (i) SEQ ID NO: 164 and 169; (j) SEQ ID NO: 165 and 167; (k) SEQ ID NO: 162 and 171; or (l) SEQ ID NO: 165 and 171.

In certain embodiments, the antibody or antigen-binding fragment thereof provided herein comprises a VH and a VL that respectively comprise the amino acid sequences selected from the group consisting of (a) SEQ ID NO: 173 and 178; (b) SEQ ID NO: 173 and 179; (c) SEQ ID NO: 173 and 180; (d) SEQ ID NO: 173 and 181; (e) SEQ ID NO: 174 and 178; (f) SEQ ID NO: 174 and 179; (g) SEQ ID NO: 174 and 180; (h) SEQ ID NO: 174 and 181; (i) SEQ ID NO: 175 and 178; (j) SEQ ID NO: 175 and 179; (k) SEQ ID NO: 175 and 180; (l) SEQ ID NO: 175 and 181; (m) SEQ ID NO: 176 and 178; (n) SEQ ID NO: 176 and 179; (o) SEQ ID NO: 176 and 180; (p) SEQ ID NO: 173 and 183; (q) SEQ ID NO: 176 and 183; or (r) SEQ ID NO: 176 and 184.

In certain embodiments, the antibody or antigen-binding fragment thereof provided herein comprises a VH and a VL that respectively comprise the amino acid sequences selected from the group consisting of (a) SEQ ID NO: 186 and 193; (b) SEQ ID NO: 186 and 194; (c) SEQ ID NO: 186 and 195; (d) SEQ ID NO: 186 and 196; (e) SEQ ID NO: 187 and 193; (f) SEQ ID NO: 187 and 194; (g) SEQ ID NO: 187 and 195; (h) SEQ ID NO: 188 and 193; (i) SEQ ID NO: 188 and 194; (j) SEQ ID NO: 189 and 193; (k) SEQ ID NO: 191 and 198; or (l) SEQ ID NO: 191 and 199.

In one aspect, the present disclosure provides an antibody or antigen-binding fragment thereof having specificity to a human CDH17 protein, wherein the antibody or antigen-binding fragment thereof comprises a heavy chain variable region comprising heavy chain complementarity determining regions HCDR1, HCDR2, and HCDR3, and a light chain variable region comprising light chain complementarity determining regions LCDR1, LCDR2, and LCDR3, wherein:

the set of HCDR1, HCDR2, and HCDR3 is selected from Table 2A, or CDR sets derived from Table 2A with one, two, or three amino acid addition, deletion and/or substitution in one or more of the CDRs, and

the set of LCDR1, LCDR2, and LCDR3 are selected from Table 2B or CDR sets derived from Table 2B with one, two, or three amino acid addition, deletion and/or substitution in one or more of the CDRs.

In one aspect, the present disclosure provides an antibody or antigen-binding fragment thereof having specificity to a human CDH17 protein, wherein the antibody or antigen-binding fragment thereof compete with the antibody or antigen-binding fragment thereof provided herein.

In certain embodiments, the antibody or an antigen-binding fragment thereof is selected from the group consisting of a full length antibody, Fab, Fab', F(ab')2, Fd, Fvs, single-chain Fvs (scFv), single-chain antibodies, disulfide-linked Fvs (sdFv), a nanobody, a domain antibody, an isolated CDR and a bivalent domain antibody.

In certain embodiments, the antibody or antigen-binding fragment thereof further comprising a heavy chain constant region, a light chain constant region, an Fc region, or the combination thereof.

In certain embodiments, the light chain constant region is a kappa or lambda chain constant region. In certain embodiments, the isotype is IgG1, IgG2, IgG3 or IgG4.

In one aspect, the present disclosure provides an antibody-drug conjugate comprising the antibody or antigen-binding fragment thereof provided herein, and a drug which is optionally connected to the antibody or antigen-binding fragment thereof through a linker.

In certain embodiments, the linker is connected to the antibody or antigen-binding fragment thereof provided herein via chemical conjugation or enzymatic conjugation.

In certain embodiments, the linker is a cleavable linker, or a non-cleavable linker.

In certain embodiments, the cleavable linker is a chemically sensitive linker or enzyme-cleavable linker; wherein the non-cleavable linker comprises thioether or maleimidecaproyl (MC).

In certain embodiments, the chemically sensitive linker is a pH-sensitive linker or glutathione-sensitive disulfide linker; wherein the enzyme-cleavable linker is a peptide based linker or β-glucuronide linker.

In certain embodiments, the linker comprises succinimidyl-4-(N-maleimidomethyl) cyclohexane-1-carboxylate (SMCC), sulfo-SMCC, p-carboxycyclo hexylmethylmaleimide, maleimide-caproyl (MC)-Valine-citrulline (VC)- para-aminobenzyloxycarbamoyl (PABC), CL2A, maleimide-caproyl (MC)- glycine-glycine-phenylalanine-glycine (GGFG), MC, or maleimide propoyl (MP)-PEG8-Valine-alanine (VA)-PABC.

In certain embodiments, the drug is selected from the group consisting of a cytotoxin, a therapeutic peptide and polypeptide.

In certain embodiments, the drug is selected from the group consisting of auristatins, maytansinoids, benzodiazepines, tubulysins, duocarmycin, camptothecin, calicheamicins, exatecans, irinotecans (SN38), doxorubicin, anthracycline, the pyrrolobenzodiazepenes (PBD), TLR agonist, STING agonists, pseudomonas aeruginosa exotoxin PE38, diphtheria toxin, staphylococcus aureus enterotoxin A/E-120, antibacterial antibiotic, shigatoxin, ricin, and urease.

In certain embodiments, the drug is monomethyl auristatin E (MMAE), monomethyl auristatin F (MMAF), maytansine, mertansine (DM1), ravtansine (DM4), tublysin A, DX-8951f, DXd, 7-ethyl-10-hydroxycamptothecin (SN-38), DGN462, Amberstatin269, anthramycin, SG3199 / SCX, IRDye®700DX, TLR7/8 agonist, diABZI STING agonist-2, or any derivative thereof.

In certain embodiments, the drug and the linker, collectively, is ozogamicin, vedotin, mafodotin, emtansine, soravtansine, ravtansine, mertansine, deruxtecan, govitecan, or tesirine.

In certain embodiments, the drug and linker, collectively, comprise

(a) vedotin having Formula I:

(Mc-vc-PABC-MMAE);

Formula I

(b) deruxtecan having Formula II:

Formula II (Mc-GGFG-DXd);

(c) ozogamicin having Formula III:

Formula III ;

(d) mafodotin having Formula IV:

Formula IV ;

(e) emtansine having Formula V:

Formula V ;

(f) govitecan having Formula VI:

Formula VI ;

or

(g) tesirine having Formula VII:

Formula VII .

In one aspect, the present disclosure provides a composition comprising the antibody or antigen-binding fragment thereof or the antibody-drug conjugate provided herein, and a pharmaceutically acceptable carrier.

In one aspect, the present disclosure provides an isolated cell comprising one or more polynucleotide encoding the antibody or antigen-binding fragment thereof.

In one aspect, the present disclosure provides a polynucleotide encoding one or more chains of the antibody or antigen-binding fragment thereof provided herein.

In one aspect, the present disclosure provides a method of treating cancer in a patient in need thereof, comprising administering to the patient the antibody or antigen-binding fragment thereof, the antibody-drug conjugate, or the composition as provided herein.

In one aspect, the present disclosure provides use of the antibody or antigen-binding fragment thereof, the antibody-drug conjugate, or the composition as provided herein in the manufacture of a medicament for treating cancer in a patient in need thereof.

In one aspect, the present disclosure provides the antibody or antigen-binding fragment thereof, the antibody-drug conjugate, or the composition provided herein for use in the treatment of cancer in a patient in need thereof.

In certain embodiments, the cancer is selected from the group consisting of bladder cancer, breast cancer, colorectal cancer, endometrial cancer, esophageal cancer, head and neck cancer, kidney cancer, leukemia, liver cancer, lung cancer, lymphoma, melanoma, pancreatic cancer, prostate cancer, and thyroid cancer.

In certain embodiments, the method further comprising administering to the patient an additional therapy for treating said cancer. In certain embodiments, said additional therapy an immunotherapy, a chemotherapy or a radiotherapy.

In one aspect, the present disclosure provides a method of detecting expression of CDH17 in a sample, comprising contacting the sample with the antibody or antigen-binding fragment thereof provided herein under conditions for the antibody or antigen-binding fragment thereof to bind to the CDH17, and detecting the binding which indicates expression of CDH17 in the sample.

## BRIEF DESCRIPTION OF THE DRAWINGS

**FIG. 1A**-**1B** show the binding activity of the CDH17 chimeric antibodies to human CDH17 protein.

**FIG. 2A**-**2B** show the binding activity of the CDH17 chimeric antibodies to human CDH16 protein.

**FIG. 3A**-**3D** show the binding activity of the CDH17 chimeric antibodies to human CDH17 overexpressing HEK293 cells (**3A**-**3B**) and CDH17 negative HEK293 cells (**3C**-**3D**).

**FIG. 4A-4F** show the binding activity of the CDH17 chimeric antibodies to CDH17 positive AsPC-1 cells (**4A-4B**), HCT-8 cells (**4C-4D**) and AGS cells (**4E-4F**), respectively.

**FIG. 5A**-**5B** show the binding activity of the CDH17 chimeric antibodies to cynomolgus CDH17 overexpressing HEK293 cells.

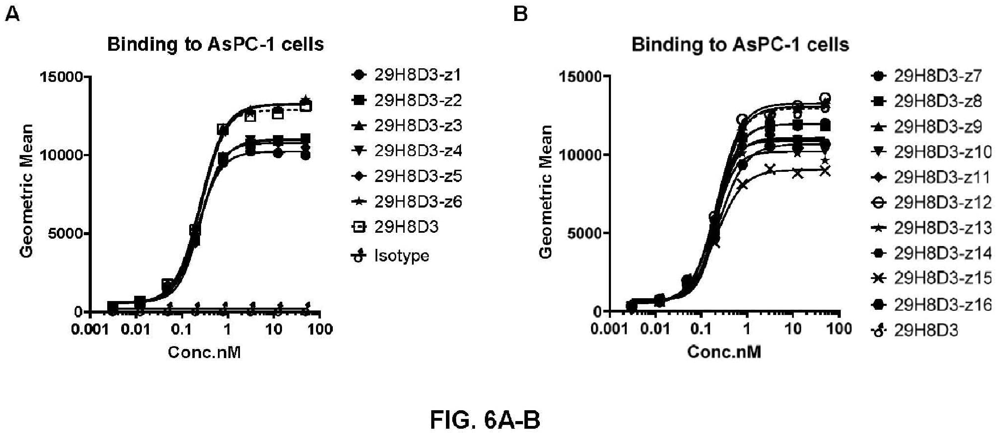

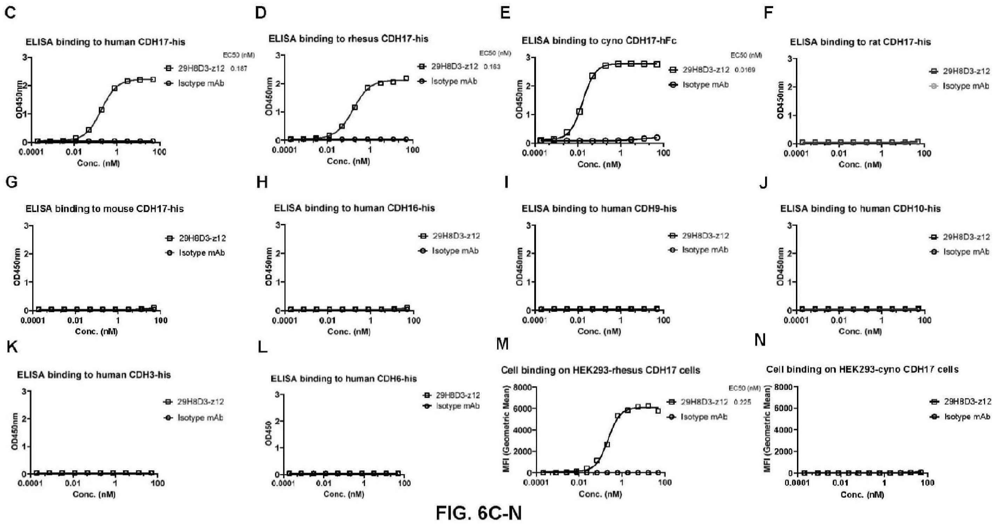

**FIG. 6A**-**6B** show the binding activity of the 29H8D3 humanized antibodies to human CDH17 expressing AsPC-1 cells**. FIG. 6C**-**6G** show the binding activity of humanized 29H8D3-z12 to recombinant human (**FIG. 6C**), rhesus (**FIG. 6D**), cyno (**FIG. 6E**), rat (**FIG. 6F**) and mouse (**FIG. 6G**) CDH17 proteins**. FIG. 6H**-**6L** show the binding activity of humanized 29H8D3-z12 to recombinant human CDH16 (**FIG. 6H**), CDH9 (**FIG. 6I**), CDH10 (**FIG. 6J**), CDH3 (**FIG. 6K**) and CDH6 (**FIG. 6L**) proteins**. FIG. 6M-6N** show the binding activity of humanized 29H8D3-z12 to rhesus (**FIG. 6M**) and cyno (**FIG. 6N**) CDH17 expressed on HEK-293 cells.

**FIG. 7A-7B** show the binding activity of the 29D2D7 humanized antibodies to human CDH17 expressing HEK293 cells.

**FIG. 8** shows the binding activity of the 61C7F12 humanized antibodies to human CDH17 expressing HEK293 cells.

**FIG. 9** shows the binding activity of the 69E3H11 humanized antibodies to human CDH17 expressing HEK293 cells.

**FIG. 10A-10B** show the binding activity of the 143H10E4 humanized antibodies to human CDH17 expressing HCT-8 cells.

**FIG. 11A-11B** show the binding activity of the 152A1D12 humanized antibodies to human CDH17 expressing AGS cells.

**FIG. 12A-12B** show the binding activity of the 155B11C6 humanized antibodies to CDH17 expressing AGS cells.

**FIG. 13A-13D** show the binding activity of the PTM site removed 29D2D7 humanized antibodies (**13A**-**13B**) and 29H8D3 humanized antibodies (**13C**-**13E**) to human CDH17 expressing HCT-8 cells**. FIG. 13F-13I** show the binding activity of the affinity matured 69E3H11 humanized antibodies to human CDH17 expressing LoVo (**13F**), HCT-8 (**13G** and **13I**) and AsPC1 (**13H**) cells.

**FIG. 14A-14B** show internalization of the CDH17 chimeric antibodies on human CDH17 positive AsPC-1 cells (**14A)** and LoVo cells (**14B**).

**FIG. 15A-15E** show internalization of the CDH17 humanized antibodies and PTM removed CDH17 humanized antibodies on human CDH17 positive HCT-8 cells.

**FIG. 16A-16G** show the chemical structure of linker-payload compounds vedotin (**16A**), deruxtecan (**16B**), ozogamicin (**16C**), mafodotin (**16D**), emtansine (**16E**), govitecan (**16F**) and tesirine (**16G**) used in the ADCs.

**FIG. 17A-17C** show the indirect tumor cell killing of CDH17 antibodies mediated by Mc-vc-PABC-MMAE-labeled anti-human IgG secondary antibody against AGS cells.

**FIG. 18A-18C** show *in vitro* tumor cell killing of Mc-vc-PABC-MMAE-labeled CDH17 ADCs against CDH17 positive AsPC1 (**18A**), LoVo (**18B**) and AGS (**18C**) cells.

**FIG. 19A-19D** show the binding activity of Mc-GGFG-DXd-labeled CDH17 ADCs and the parental CDH17 mAbs to CDH17 expressing tumor cells including AsPC1(**19A**), AGS (**19B**), KATO III (**19C**) and HT29 (**19D**) cells.

**FIG. 20A-20F** show the internalization rate (**20A**, **20C-20F**) and percentage (**20B**) of Mc-GGFG-DXd-labeled ADCs and mAbs on CDH17 expressing HCT-8 (**20A**), AGS (**20C**), AsPC1 (**20D**), SW480-hCDH17 (**20E**) cells and CDH17 negative SW480 (**20F**) cells.

**FIG. 21A-21D** show *in vitro* tumor cell killing of Mc-GGFG-DXd-labeled CDH17 ADCs against CDH17 positive HCT-8 (**21A**), SW620-hCDH17(**21B**), MDA-MB-468-hCDH17 (**21C**) and SW480-hCDH17 (**21D**) cells.

**FIG. 22A-22I** show *in vitro* tumor cell killing of Mc-GGFG-DXd-labeled CDH17 ADCs against CDH17 positive HCT-8 (**22A**), AGS (**22B**), MDA-MB-468-hCDH17 (**22C**), SW480-hCDH17 (**22D**), SK-CO-1 (**22E**), SW620-hCDH17(**22F**), LS1034 (**22G**), Caco2 (**22H**), and CDH17 negative RKO (**22I**) cells.

**FIG. 23A-23C** show *in vitro* bystander killing of Mc-GGFG-DXd-labeled CDH17 ADCs in coculture systems composed of CDH17 positive SW480-hCDH17 cells labelled with GFP and CDH17 negative RKO cells labelled with RFP (**23A**), or SW480 cells (**23B**), or MDA-MB-468 cells (**23C**).

**FIG. 24A-24B** show *in vivo* tumor growth inhibition of the Mc-vc-PABC-MMAE-labeled CDH17 ADCs in LoVo CDX mice model.

**FIG. 25A** shows *in vivo* tumor growth inhibition of the Mc-GGFG-DXd-labeled CDH17 ADCs in LS1034 CDX mice model**. FIG. 25B** shows the body weight change of the mice after treatment in LS1034 CDX mice model.

**FIG. 26A-26B** show *in vivo* tumor growth inhibition (**26A**) and body weight changes (**26B**) of the GGFG-DXd-labeled CDH17 ADCs in four CRC PDX mice models.

**FIG. 27** show *in vivo* dose-dependent tumor growth inhibition of the GGFG-DXd-labeled CDH17 ADCs in three CRC PDX mice models.

## DETAILED DESCRIPTION

### *Definitions*

It is to be noted that the term "a" or "an" entity refers to one or more of that entity; for example, "an antibody," is understood to represent one or more antibodies. As such, the terms "a" (or "an"), "one or more," and "at least one" can be used interchangeably herein.

As used herein, an "antibody" or "antigen-binding moiety" refers to a polypeptide or a polypeptide complex that specifically recognizes and binds to an antigen. An antibody can be a whole antibody and any antigen binding fragment or a single chain thereof. Thus the term "antibody" includes any protein or peptide containing molecule that comprises at least a portion of an immunoglobulin molecule having biological activity of binding to the antigen. Examples of such include, but are not limited to a complementarity determining region (CDR) of a heavy or light chain or a ligand binding portion thereof, a heavy chain or light chain variable region, a heavy chain or light chain constant region, a framework (FR) region, or any portion thereof, or at least one portion of a binding protein.

A full-length antibody comprises two heavy chains and two light chains. The variable regions of the light and heavy chains are responsible for antigen binding. The variable domains of the heavy chain and light chain may be referred to as "VH" and "VL", respectively. The variable regions in both chains generally contain three highly variable loops called the complementarity determining regions (CDRs) (light chain (LC) CDRs including LC-CDR1, LC-CDR2, and LC-CDR3, heavy chain (HC) CDRs including HC-CDR1, HC-CDR2, and HC-CDR3). CDR boundaries for the antibodies and antigen-binding fragments disclosed herein may be defined or identified by the conventions of Kabat, Chothia, or Al-Lazikani (Al-Lazikani 1997; Chothia 1985; Chothia 1987; Chothia 1989; Kabat 1987; Kabat 1991). The three CDRs of the heavy or light chains are interposed between flanking stretches known as framework regions (FRs), which are more highly conserved than the CDRs and form a scaffold to support the hypervariable loops. The constant regions of the heavy and light chains are not involved in antigen binding, but exhibit various effector functions. Antibodies are assigned to classes based on the amino acid sequence of the constant region of their heavy chain. The five major classes or isotypes of antibodies are IgA, IgD, IgE, IgG, and IgM, which are characterized by the presence of $\alpha$, $\delta$, $\varepsilon$, $\gamma$, and $\mu$ heavy chains, respectively. Several of the major antibody classes are divided into subclasses such as IgG1 ($\gamma$1 heavy chain), IgG2 ($\gamma$2 heavy chain), IgG3 ($\gamma$3 heavy chain), IgG4 ($\gamma$4 heavy chain), IgA1 ($\alpha$1 heavy chain), or IgA2 ($\alpha$2 heavy chain).

The term "half antibody" as used herein refers to one immunoglobulin heavy chain associated with one immunoglobulin light chain. One skilled in the art will readily appreciate that a half-antibody may encompass a fragment thereof and may also have an antigen binding domain consisting of a single variable domain*, e.g., orig*inating from a camelidae.

The term "single chain half antibody" as used herein refers to a single chain polypeptide comprising a VL domain, optionally a CL domain, a tether, a VH domain, optionally a CH1 domain, a hinge domain, a CH2 domain and a CH3 domain, wherein said domains are positioned relative to each other in an N-terminal to C-terminal direction as follows: VL-tether-VH-hinge-CH2-CH3, VL-tether-VH-partial hinge-CH2-CH3, VL-tether-VH- hinge variant -CH2-CH3, or VL-CL-tether-VH-CH1-hinge-CH2-CH3.

The expression "single domain antibodies" (sdAbs) or "single variable domain (SVD) antibodies" generally refers to antibodies in which a single variable domain (VH or VL) can confer antigen binding. In other words, the single variable domain does not need to interact with another variable domain in order to recognize the target antigen. Examples of single domain antibodies include those derived from camelids (lamas and camels) and cartilaginous fish (*e.g., nurs*e sharks) and those derived from recombinant methods from humans and mouse antibodies (Nature (1989) 341:544-546; Dev Comp

Immunol (2006) 30:43-56; Trend Biochem Sci (2001) 26:230-235; Trends Biotechnol (2003):21:484-490; WO 2005/035572; WO 03/035694; Febs Lett (1994) 339:285-290; WO00/29004; WO 02/051870). When the sdAb contains only a heavy chain, it can be exchangably used with "VHH" or "single heavy chain variable domain antibody" or "nanobody".

The terms "antibody fragment" or "antigen-binding fragment", as used herein, is a portion of an antibody such as $F(ab')_2$, $F(ab)_2$, Fab', Fab, Fv, scFv and the like. Regardless of structure, an antibody fragment binds with the same antigen that is recognized by the intact antibody. The term "antibody fragment" includes aptamers, spiegelmers, and diabodies. The term "antibody fragment" also includes any synthetic or genetically engineered protein that acts like an antibody by binding to a specific antigen to form a complex.

A "Fab" with regard to an antibody refers to a monovalent antigen-binding fragment of the antibody consisting of a single light chain (both variable and constant regions) bound to the variable region and first constant region of a single heavy chain by a disulfide bond. Fab can be obtained by papain digestion of an antibody at the residues proximal to the N-terminus of the disulfide bond between the heavy chains of the hinge region.

A "Fab'" refers to a Fab fragment that includes a portion of the hinge region, which can be obtained by pepsin digestion of an antibody at the residues proximal to the C-terminus of the disulfide bond between the heavy chains of the hinge region and thus is different from Fab in a small number of residues (including one or more cysteines) in the hinge region.

A "$F(ab)_2$" refers to a dimer of Fab' that comprises two light chains and part of the two heavy chains.

"Fv" with regard to an antibody refers to the smallest fragment of the antibody to bear the complete antigen binding site. A Fv fragment consists of the variable region of a single light chain bound to the variable region of a single heavy chain. A "dsFv" refers to a disulfide-stabilized Fv fragment that the linkage between the variable region of a single light chain and the variable region of a single heavy chain is a disulfide bond.

A "single-chain variable fragment" or "scFv" refers to a fusion protein of the variable regions of the heavy ($V_H$) and light chains ($V_L$) of immunoglobulins. In some aspects, the regions are connected with a short linker peptide of ten to about 25 amino acids. The linker can be rich in glycine for flexibility, as well as serine or threonine for solubility, and can either connect the N-terminus of the $V_H$ with the C-terminus of the $V_L$, or vice versa. This protein retains the specificity of the original immunoglobulin, despite removal of the constant regions and the introduction of the linker. ScFv molecules are known in the art and are described, *e.g., in* US patent 5,892,019.

As used herein, the term "diabody molecule" refers to a complex of two or more polypeptide chains or proteins, each comprising at least one VL and one VH domain or fragment thereof, wherein both domains are comprised within a single polypeptide chain. In certain embodiments "diabody molecule" includes molecules comprising an Fc or a hinge-Fc domain. Said polypeptide chains in the complex may be the same or different, i.e., the diabody molecule may be a homo-multimer or a hetero-multimer. In specific aspects, "diabody molecule" includes dimers or tetramers or said polypeptide chains containing both a VL and VH domain. The individual polypeptide chains comprising the multimeric proteins may be covalently joined to at least one other peptide of the multimer by interchain disulfide bonds.

A "domain antibody" refers to an antibody fragment containing only the variable region of a heavy chain or the variable region of a light chain. In certain embodiments, two or more VH domains are covalently joined with a peptide linker to form a bivalent or multivalent domain antibody. The two VH domains of a bivalent domain antibody may target the same or different antigens.

The term antibody encompasses various broad classes of polypeptides that can be distinguished biochemically. Those skilled in the art will appreciate that heavy chains are classified as gamma, mu, alpha, delta, or epsilon (y, $\mu$, $\alpha$, $\delta$, $\varepsilon$) with some subclasses among them *(e.g., $\gamma$l-*$\gamma$4). It is the nature of this chain that determines the "class" of the antibody as IgG, IgM, IgA IgG, or IgE, respectively. The immunoglobulin subclasses (isotypes) *e.g., $IgG_1$,* $IgG_2$, $IgG_3$, $IgG_4$, $IgG_5$, etc. are well characterized and are known to confer functional specialization. Modified versions of each of these classes and isotypes are readily discernable to the skilled artisan in view of the instant disclosure and, accordingly, are within the scope of the instant disclosure. All immunoglobulin classes are clearly within the scope of the present disclosure, the following discussion will generally be directed to the IgG class of immunoglobulin molecules. With regard to IgG, a standard immunoglobulin molecule comprises two identical light chain polypeptides of molecular weight approximately 23,000 Daltons, and two identical heavy chain polypeptides of molecular weight 53,000-70,000. The four chains are typically joined by disulfide bonds in a "Y" configuration wherein the light chains bracket the heavy chains starting at the mouth of the "Y" and continuing through the variable region.

Antibodies, antigen-binding moieties, variants, or derivatives thereof of the disclosure include, but are not limited to, polyclonal, monoclonal, multispecific, human, humanized, primatized, or chimeric antibodies, single chain antibodies, epitope-binding fragments*, e.g.,* Fab*,* Fab' and $F(ab')_2$, Fd, Fvs, single-chain Fvs (scFv), single-chain antibodies, disulfide-linked Fvs (sdFv), fragments comprising either a VK or VH domain, fragments produced by a Fab expression library, and anti-idiotypic (anti-Id) antibodies (including*, e.g., anti*-Id antibodies to LIGHT antibodies disclosed herein). Immunoglobulin or antibody molecules of the disclosure can be of any type (*e.g., IgG,* IgE, IgM, IgD, IgA, and IgY), class (*e.g., IgGl*, IgG2, IgG3, IgG4, IgAl and IgA2) or subclass of immunoglobulin molecule.

As used herein, the term "derivative" in the context of polypeptides or proteins refers to a polypeptide or protein that comprises an amino acid sequence which has been altered by the introduction of amino acid residue substitutions, deletions or additions. The term "derivative" as used herein also refers to a polypeptide or protein which has been modified, i.e, by the covalent attachment of any type of molecule to the polypeptide or protein. For example, but not by way of limitation, an antibody may be modified*, e.g., by g*lycosylation, acetylation, pegylation, phosphorylation, amidation, derivatization by known protecting/blocking groups, proteolytic cleavage, linkage to a cellular an antigen or other protein, etc. A derivative polypeptide or protein may be produced by chemical modifications using techniques known to those of skill in the art, including, but not limited to specific chemical cleavage, acetylation, formylation, metabolic synthesis of tunicamycin, etc. Further, a derivative polypeptide or protein derivative possesses a similar or identical function as the polypeptide or protein from which it was derived.

As used herein, the term "derivative" in the context of a non-proteinaceous derivative refers to a second organic or inorganic molecule that is formed based upon the structure of a first organic or inorganic molecule. A derivative of an organic molecule includes, but is not limited to, a molecule modified, *e.g., by the* addition or deletion of a hydroxyl, methyl, ethyl, carboxyl or amine group. An organic molecule may also be esterified, alkylated and/or phosphorylated.

Light chains are classified as either kappa or lambda (K, $\lambda$). Each heavy chain class may be bound with either a kappa or lambda light chain. In general, the light and heavy chains are covalently bonded to each other, and the "tail" portions of the two heavy chains are bonded to each other by covalent disulfide linkages or non-covalent linkages when the immunoglobulins are generated either by hybridomas, B cells or genetically engineered host cells. In the heavy chain, the amino acid sequences run from an N-terminus at the forked ends of the Y configuration to the C-terminus at the bottom of each chain.

Both the light and heavy chains are divided into regions of structural and functional homology. The terms "constant" and "variable" are used functionally. In this regard, it will be appreciated that the variable domains of both the light (VK) and heavy (VH) chain portions determine antigen recognition and specificity. Conversely, the constant domains of the light chain (CK) and the heavy chain (CH1, CH2 or CH3) confer important biological properties such as secretion, transplacental mobility, Fc receptor binding, complement binding, and the like. By convention the numbering of the constant region domains increases as they become more distal from the antigen-binding site or amino- terminus of the antibody. The N-terminal portion is a variable region and at the C-terminal portion is a constant region; the CH3 and CK domains actually comprise the carboxy-terminus of the heavy and light chain, respectively.

As indicated above, the variable region allows the antibody to selectively recognize and specifically bind epitopes on antigens. That is, the VK domain and VH domain, or subset of the complementarity determining regions (CDRs), of an antibody combine to form the variable region that defines a three dimensional antigen-binding site. This quaternary antibody structure forms the antigen-binding site present at the end of each arm of the Y. More specifically, the antigen-binding site is defined by three CDRs on each of the VH and VK chains *(i.e.* CDR-H1, CDR-H2, CDR-H3, CDR-L1, CDR-L2 and CDR-L3). In some instances, *e.g., certain* immunoglobulin molecules derived from camelid species or engineered based on camelid immunoglobulins, a complete immunoglobulin molecule may consist of heavy chains only, with no light chains. *See, e.g.,* Hamers-Casterman et al., Nature 363:446-448 (1993).

In naturally occurring antibodies, the six "complementarity determining regions" or "CDRs" present in each antigen-binding domain are short, non-contiguous sequences of amino acids that are specifically positioned to form the antigen-binding domain as the antibody assumes its three dimensional configuration in an aqueous environment. The remainder of the amino acids in the antigen-binding domains, referred to as "framework" regions, show less inter-molecular variability. The framework regions largely adopt a $\beta$-sheet conformation and the CDRs form loops which connect, and in some cases form part of, the $\beta$ -sheet structure. Thus, framework regions act to form a scaffold that provides for positioning the CDRs in correct orientation by inter-chain, non-covalent interactions. The antigen-binding domain formed by the positioned CDRs defines a surface complementary to the epitope on the immunoreactive antigen. This complementary surface promotes the non-covalent binding of the antibody to its cognate epitope. The amino acids comprising the CDRs and the framework regions, respectively, can be readily identified for any given heavy or light chain variable region by one of ordinary skill in the art, since they have been precisely defined (*see* "Sequences of Proteins of Immunological Interest," Kabat, E., et al., U.S. Department of Health and Human Services, (1983); and Chothia and Lesk, J. Mol. Biol., 196:901-917 (1987)).

In the case where there are two or more definitions of a term which is used and/or accepted within the art, the definition of the term as used herein is intended to include all such meanings unless explicitly stated to the contrary. A specific example is the use of the term "complementarity determining region" ("CDR") to describe the non-contiguous antigen combining sites found within the variable region of both heavy and light chain polypeptides. This particular region has been described by Kabat et al., U.S. Dept. of Health and Human Services, "Sequences of Proteins of Immunological Interest" (1983) and by Chothia et al., J. Mol. Biol. 196:901-917 (1987), which are incorporated herein by reference in their entireties. The CDR definitions according to Kabat and Chothia include overlapping or subsets of amino acid residues when compared against each other. Nevertheless, application of either definition to refer to a CDR of an antibody or variants thereof is intended to be within the scope of the term as defined and used herein. The appropriate amino acid

residues which encompass the CDRs as defined by each of the above cited references are set forth in the table below as a comparison. The exact residue numbers which encompass a particular CDR will vary depending on the sequence and size of the CDR. Those skilled in the art can routinely determine which residues comprise a particular CDR given the variable region amino acid sequence of the antibody.

| | Kabat | Chothia |
|---|---|---|
| CDR-H1 | 31-35 | 26-32 |
| CDR-H2 | 50-65 | 52-58 |
| CDR-H3 | 95-102 | 95-102 |
| CDR-L1 | 24-34 | 26-32 |
| CDR-L2 | 50-56 | 50-52 |
| CDR-L3 | 89-97 | 91-96 |

Kaba*t et al*. also defined a numbering system for variable domain sequences that is applicable to any antibody. One of ordinary skill in the art can unambiguously assign this system of "Kabat numbering" to any variable domain sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983).

In addition to table above, the Kabat number system describes the CDR regions as follows: CDR-H1 begins at approximately amino acid 31 (*i.e.*, approximately 9 residues after the first cysteine residue), includes approximately 5-7 amino acids, and ends at the next tryptophan residue. CDR-H2 begins at the fifteenth residue after the end of CDR-H1, includes approximately 16-19 amino acids, and ends at the next arginine or lysine residue. CDR-H3 begins at approximately the thirty third amino acid residue after the end of CDR-H2; includes 3-25 amino acids; and ends at the sequence W-G-X-G, where X is any amino acid. CDR-L1 begins at approximately residue 24 (*i.e.*, following a cysteine residue); includes approximately 10-17 residues; and ends at the next tryptophan residue. CDR-L2 begins at approximately the sixteenth residue after the end of CDR-L1 and includes approximately 7 residues. CDR-L3 begins at approximately the thirty third residue after the end of CDR-L2 (*i.e.*, following a cysteine residue); includes approximately 7-11 residues and ends at the sequence F or W-G-X-G, where X is any amino acid.

Antibodies disclosed herein may be from any animal origin including birds and mammals. Preferably, the antibodies are human, murine, donkey, rabbit, goat, guinea pig, camel, llama, horse, or chicken antibodies. In another embodiment, the variable region may be condricthoid in origin (*e.g., from* sharks).

As used herein, the term "heavy chain constant region" includes amino acid sequences derived from an immunoglobulin heavy chain. A polypeptide comprising a heavy chain constant region comprises at least one of: a CH1 domain, a hinge (*e.g., uppe*r, middle, and/or lower hinge region) domain, a CH2 domain, a CH3 domain, or a variant or fragment thereof. For example, an antigen-binding polypeptide for use in the disclosure may comprise a polypeptide chain comprising a CH1 domain; a polypeptide chain comprising a CH1 domain, at least a portion of a hinge domain, and a CH2 domain; a polypeptide chain comprising a CH1 domain and a CH3 domain; a polypeptide chain comprising a CH1 domain, at least a portion of a hinge domain, and a CH3 domain, or a polypeptide chain comprising a CH1 domain, at least a portion of a hinge domain, a CH2 domain, and a CH3 domain. In another embodiment, a polypeptide of the disclosure comprises a polypeptide chain comprising a CH3 domain. Further, an antibody for use in the disclosure may lack at least a portion of a CH2 domain (*e.g., all* or part of a CH2 domain). As set forth above, it will be understood by one of ordinary skill in the art that the heavy chain constant region may be modified such that they vary in amino acid sequence from the naturally occurring immunoglobulin molecule.

The heavy chain constant region of an antibody disclosed herein may be derived from different immunoglobulin molecules. For example, a heavy chain constant region of a polypeptide may comprise a CH1 domain derived from an $IgG_l$ molecule and a hinge region derived from an $IgG_3$ molecule. In another example, a heavy chain constant region can comprise a hinge region derived, in part, from an $IgG_l$ molecule and, in part, from an $IgG_3$ molecule. In another example, a heavy chain portion can comprise a chimeric hinge derived, in part, from an $IgG_l$ molecule and, in part, from an $IgG_4$ molecule.

As used herein, the term "light chain constant region" includes amino acid sequences derived from antibody light chain. Preferably, the light chain constant region comprises at least one of a constant kappa domain or constant lambda domain.

A "light chain-heavy chain pair" refers to the collection of a light chain and heavy chain that can form a dimer through a disulfide bond between the CL domain of the light chain and the CH1 domain of the heavy chain.

As previously indicated, the subunit structures and three dimensional configuration of the constant regions of the various immunoglobulin classes are well known. As used herein, the term "VH domain" includes the amino terminal variable domain of an immunoglobulin heavy chain and the term "CH1 domain" includes the first (most amino terminal) constant region domain of an immunoglobulin heavy chain. The CH1 domain is adjacent to the VH domain and is amino terminal to the hinge region of an immunoglobulin heavy chain molecule.

The "CH1 domain" (also referred to as "C1" of "H1" domain) usually extends from about amino acid 118 to about amino acid 215 (EU numbering system).

As used herein, the term "hinge region" includes the portion of a heavy chain molecule that joins the CH1 domain to the CH2 domain, a region in IgG corresponding to Glu216 to Pro230 of human IgG1, EU numbering system (Burton, Molec. Immunol.22:161-206 (1985)). Hinge regions of other IgG isotypes may be aligned with the IgG1 sequence by placing the first and last cysteine residues forming inter-heavy chain S-S bonds in the same positions. This hinge region is flexible, thus allowing the two N-terminal antigen-binding regions to move independently. Hinge regions can be subdivided into three distinct domains: upper, middle, and lower hinge domains (Roux et al., J. Immunol 161:4083 (1998)).

As used herein the term "CH2 domain" includes the portion of a heavy chain molecule that extends*, e.g., from* about residue 244 to residue 360 of an antibody using conventional numbering schemes (residues 244 to 360, Kabat numbering system; and residues 231-340, EU numbering system; *see* Kabat et al., U.S. Dept. of Health and Human Services, "Sequences of Proteins of Immunological Interest" (1983). The CH2 domain is unique in that it is not closely paired with another domain. Rather, two N-linked branched carbohydrate chains are interposed between the two CH2 domains of an intact native IgG molecule. It is also well documented that the CH3 domain extends from the CH2 domain to the C-terminal of the IgG molecule and comprises approximately 108 residues.

The "CH3 domain" (also referred to as "C3" domain) comprises the stretch of residues C-terminal to a CH2 domain in an Fc region (*i.e.* from about amino acid residue 341 to the C-terminal end of an antibody sequence, typically at amino acid residue 446 or 447 of an IgG, EU numbering system).

The term "Fc region", "Fc domain" or "fragment crystallizable region" herein is used to define a C-terminal region of an immunoglobulin heavy chain, including native-sequence Fc regions and variant Fc regions. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. Suitable native-sequence Fc regions for use in the antibodies described herein include human IgG1, IgG2 (IgG2A, IgG2B), IgG3 and IgG4.

As used herein the term "disulfide bond" includes the covalent bond formed between two sulfur atoms. The amino acid cysteine comprises a thiol group that can form a disulfide bond or bridge with a second thiol group. In most naturally occurring IgG molecules, the CH1 and CK regions are linked by a disulfide bond and the two heavy chains are linked by two disulfide bonds at positions corresponding to 239 and 242 using the Kabat numbering system (position 226 or 229, EU numbering system).

As used herein, the term "chimeric antibody" will be held to mean any antibody wherein the immunoreactive region or site is obtained or derived from a first species and the constant region (which may be intact, partial or modified in accordance with the instant disclosure) is obtained from a second species. In certain embodiments the target binding region or site will be from a non-human source (*e.g., mous*e or primate) and the constant region is human.

"Humanized antibody" is used herein to describe an antibody that comprises heavy and light chain variable region sequences from a non-human species (*e.g., a mo*use) but in which at least a portion of the VH and/or VL sequence has been altered to be more "human-like," i.e., more similar to human germline variable sequences. A "humanized antibody" is an antibody or a variant, derivative, analog, or fragment thereof, which immunospecifically binds to an antigen of interest and which comprises a framework (FR) region having substantially the amino acid sequence of a human antibody and a complementary determining region (CDR) having substantially the amino acid sequence of a non-human antibody. As used herein, the term "substantially" in the context of a CDR refers to a CDR having an amino acid sequence at least 80%, at least 85%, at least 90%, at least 95%, at least 98%, or at least 99% identical to the amino acid sequence of a non-human antibody CDR. A humanized antibody comprises substantially all of at least one, and typically two, variable domains (Fab, Fab', $F(ab')_2$, Fv) in which all or substantially all of the CDR regions correspond to those of a non-human immunoglobulin (i.e., donor antibody) and all or substantially all of the framework regions are those of a human immunoglobulin consensus sequence. In an embodiment, a humanized antibody also comprises at least a portion of an immunoglobulin constant region (Fc), typically that of a human immunoglobulin. In some embodiments, a humanized antibody contains the light chain as well as at least the variable domain of a heavy chain. The antibody also may include the CH1, hinge, CH2, CH3, and CH4 regions of the heavy chain. In some embodiments, a humanized antibody only contains a humanized light chain. In some embodiments, a humanized antibody only contains a humanized heavy chain. In specific

embodiments, a humanized antibody only contains a humanized variable domain of a light chain and/or humanized heavy chain.

The term "epitope" as used herein refers to the specific group of atoms or amino acids on an antigen to which an antibody or antibody moiety binds. Two antibodies or antibody moieties may bind the same epitope within an antigen if they exhibit competitive binding for the antigen.

An "antibody-drug conjugate (ADC)" is typically composed of a monoclonal antibody (mAbs) covalently attached to a cytotoxic drug (payload) via a linker. It combines both the advantages of highly specific targeting ability and highly potent killing effect to achieve accurate and efficient elimination of cancer cells, which has become one of the hotspots for the research and development of anticancer drugs. The cytotoxic drug and the linker form a structure of drug linker compound.

By "specifically binds" or "has specificity to," it is generally meant that an antibody binds to an epitope via its antigen-binding domain, and that the binding entails some complementarity between the antigen-binding domain and the epitope. According to this definition, an antibody is said to "specifically bind" to an epitope when it binds to that epitope, via its antigen-binding domain more readily than it would bind to a random, unrelated epitope. The term "specificity" is used herein to qualify the relative affinity by which a certain antibody binds to a certain epitope. For example, antibody "A" may be deemed to have a higher specificity for a given epitope than antibody "B," or antibody "A" may be said to bind to epitope "C" with a higher specificity than it has for related epitope "D."

"Percent (%) sequence identity" with respect to amino acid sequence (or nucleic acid sequence) is defined as the percentage of amino acid (or nucleic acid) residues in a candidate sequence that are identical to the amino acid (or nucleic acid) residues in a reference sequence, after aligning the sequences and, if necessary, introducing gaps, to achieve the maximum correspondence. Conservative substitution of the amino acid residues may or may not be considered as identical residues. Alignment for purposes of determining percent amino acid (or nucleic acid) sequence identity can be achieved, for example, using publicly available tools such as BLASTN, BLASTp (available on the website of U.S. National Center for Biotechnology Information (NCBI), see also, Altschul S. F. et al, J. Mol. Biol., 215:403-410 (1990); Stephen F. et al, Nucleic Acids Res., 25:3389-3402 (1997)), ClustalW2 (available on the website of European Bioinformatics Institute, see also, Higgins D. G. et al, Methods in Enzymology, 266:383-402 (1996); Larkin M. A. et al, Bioinformatics (Oxford, England), 23(21): 2947-8 (2007)), and ALIGN or Megalign (DNASTAR) software. Those skilled in the art may use the default parameters provided by the tool, or may customize the parameters as appropriate for the alignment, such as for example, by selecting a suitable algorithm.

As used herein, the terms "treat" or "treatment" refer to both therapeutic treatment and prophylactic or preventative measures, wherein the object is to prevent or slow down (lessen) an undesired physiological change or disorder, such as the progression of cancer. Beneficial or desired clinical results include, but are not limited to, alleviation of symptoms, diminishment of extent of disease, stabilized (*i.e.*, not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

By "subject" or "individual" or "animal" or "patient" or "mammal," is meant any subject, particularly a mammalian subject, for whom diagnosis, prognosis, or therapy is desired. Mammalian subjects include humans, domestic animals, farm animals, and zoo, sport, or pet animals such as dogs, cats, guinea pigs, rabbits, rats, mice, horses, cattle, cows, and so on.

As used herein, phrases such as "to a patient in need of treatment" or "a subject in need of treatment" includes subjects, such as mammalian subjects, that would benefit from administration of an antibody or composition of the present disclosure used*, e.g., for* detection, for a diagnostic procedure and/or for treatment.

***Anti-CDH17 Antibodies***

Through trials and errors, the instant inventors were able to identify new antibodies that can bind to the human CDH17 protein potently and specifically. Chimeric antibodies were including 16C18, 20C3E8, 29D2D7, 29H8D3, 61C7F12, 67A11B11, 69E3H11, 95F2C2F12, 103G6G1, 120B10C5, 134C7B1, 143H10E4, 152A1D12 and 155B11C6 obtained (**Table 1**).

In accordance with one embodiment of the present disclosure, provided are antibody or antigen-binding fragments that include the heavy chain and light chain variable domains with the CDR regions. The CDRs are summarized in **Tables 2A-2B** (Kabat numbering).

In some embodiments, the VH CDR1, CDR2, and CDR3 are selected from any set of VH CDR1, CDR2, and CDR3 shown in **Table 2A**, and the VL CDR1, CDR2, and CDR3 are selected from any set of VL CDR1, CDR2, and CDR3 shown i**n Tables 2B, 15B and 15D**. In some embodiments, the VH CDR1, CDR2, and CDR3 and the VL CDR1, CDR2, and CDR3 are selected from those derived from the same antigen-binding moieties.

In some embodiments, at least one, or two, or three, or four, or five, or six of the VH CDR1, CDR2, and CDR3 and the VL CDR1, CDR2, and CDR3 of the above are modified by one, two or three amino acid additions, deletions, substitutions, or the combinations thereof.

The CDRs, heavy chain variable regions, light chain variable regions or single heavy chain variable domains of the present disclosure can be further modified. In some embodiments, the modified heavy chain variable region, light chain variable region or single heavy chain variable domain retains at least about 70%, 75%, 80%, 85%, 90%, 95%, 98% or 99% sequence identity and is still capable of binding to the target site.

In some embodiments, the modification is substitution at no more than one hot spot position from each of the CDRs. In some embodiments, the modification is substitution at one, two or three such hot spot positions. In one embodiment, the modification is substitution at one of the hot spot positions. Such substitutions, in some embodiments, are conservative substitutions.

The antibodies or antigen-binding fragment thereof comprises a heavy chain variable region comprising an amino acid sequence selected from the group consisting of SEQ ID NO: 7, 15, 23, 31, 39, 47, 55, 63, 71, 79, 87, 95, 103, 111, or a peptide having at least 70%, 75%, 80%, 85%, 90%, 95%, 98% or 99% sequence identity to SEQ ID NO: 7, 15, 23, 31, 39, 47, 55, 63, 71, 79, 87, 95, 103, 111.

The antibodies or antigen-binding fragment thereof comprises a light chain variable region comprising an amino acid sequence selected from the group consisting of SEQ ID NO: 8, 16, 24, 32, 40, 48, 56, 64, 72, 80, 88, 96, 104, 112, or a peptide having at least 70%, 75%, 80%, 85%, 90%, 95%, 98% or 99% sequence identity to SEQ ID NO: 8, 16, 24, 32, 40, 48, 56, 64, 72, 80, 88, 96, 104, 112.

Post-translational modification (PTM) removal can be made to the antibodies in, for example, CDRs or framework regions by amino acid substitutions in order to remove potential PTM sites resulting in deamidation, isomerization, glycosylation, oxidation and unpaired cysteine, and to increase developability including long-term stability, manufacturability, and reduce heterogeneity of the antibodies provided herein. Alternatively, affinity maturation can be made to the antibodies in, for example, CDRs by amino acid substitutions in order to improve the binding affinity of the antibodies which may bring enhanced activities. According to the present disclosure, exemplary light chain CDRs in 29D2D7-z19, 29H8D3-z12 and 69E3H11-z12 undergoing PTM site removal or affinity maturation are listed in **Table 15B and 15D**, and the sequences of the variable region of the light chain so generated are listed in **Table 15A and 15C** in **Example 4**.

The antibodies or antigen-binding fragment thereof comprises (a) a heavy chain variable region comprising an amino acid sequence of SEQ ID NO: 7, a peptide having at least 70%, 75%, 80%, 85%, 90%, 95%, 98% or 99% sequence identity thereof, or a humanized version thereof, and a light chain variable region comprising an amino acid sequence of SEQ ID NO: 8, a peptide having at least 70%, 75%, 80%, 85%, 90%, 95%, 98% or 99% sequence identity thereof, or a humanized version thereof;

(b) a heavy chain variable region comprising an amino acid sequence of SEQ ID NO: 15, a peptide having at least 70%, 75%, 80%, 85%, 90%, 95%, 98% or 99% sequence identity thereof, or a humanized version thereof, and a light chain variable region comprising an amino acid sequence of SEQ ID NO: 16, a peptide having at least 70%, 75%, 80%, 85%, 90%, 95%, 98% or 99% sequence identity thereof, or a humanized version thereof;

(c) a heavy chain variable region comprising an amino acid sequence of SEQ ID NO: 23, a peptide having at least 70%, 75%, 80%, 85%, 90%, 95%, 98% or 99% sequence identity thereof, or a humanized version thereof, and a light chain variable region comprising an amino acid sequence of SEQ ID NO: 24, a peptide having at least 70%, 75%, 80%, 85%, 90%, 95%, 98% or 99% sequence identity thereof, or a humanized version thereof;

(d) a heavy chain variable region comprising an amino acid sequence of SEQ ID NO: 31, a peptide having at least 70%, 75%, 80%, 85%, 90%, 95%, 98% or 99% sequence identity thereof, or a humanized version thereof, and a light chain variable region comprising an amino acid sequence of SEQ ID NO: 32, a peptide having at least 70%, 75%, 80%, 85%, 90%, 95%, 98% or 99% sequence identity thereof, or a humanized version thereof;

(e) a heavy chain variable region comprising an amino acid sequence of SEQ ID NO: 39, a peptide having at least 70%, 75%, 80%, 85%, 90%, 95%, 98% or 99% sequence identity thereof, or a humanized version thereof, and a light chain variable region comprising an amino acid sequence of SEQ ID NO: 40, a peptide having at least 70%, 75%, 80%, 85%, 90%, 95%, 98% or 99% sequence identity thereof, or a humanized version thereof;

(f) a heavy chain variable region comprising an amino acid sequence of SEQ ID NO: 47, a peptide having at least 70%, 75%, 80%, 85%, 90%, 95%, 98% or 99% sequence identity thereof, or a humanized version thereof, and a light chain variable region comprising an amino acid sequence of SEQ ID NO: 48, a peptide having at least 70%, 75%, 80%, 85%, 90%, 95%, 98% or 99% sequence identity thereof, or a humanized version thereof;

(g) a heavy chain variable region comprising an amino acid sequence of SEQ ID NO: 55, a peptide having at least 70%, 75%, 80%, 85%, 90%, 95%, 98% or 99% sequence identity thereof, or a humanized version thereof, and a light chain variable region comprising an amino acid sequence of SEQ ID NO: 56, a peptide having at least 70%, 75%, 80%, 85%, 90%, 95%, 98% or 99% sequence identity thereof, or a humanized version thereof;

(h) a heavy chain variable region comprising an amino acid sequence of SEQ ID NO: 63, a peptide having at least 70%,

75%, 80%, 85%, 90%, 95%, 98% or 99% sequence identity thereof, or a humanized version thereof, or a humanized version thereof, and a light chain variable region comprising an amino acid sequence of SEQ ID NO: 64, a peptide having at least 70%, 75%, 80%, 85%, 90%, 95%, 98% or 99% sequence identity thereof, or a humanized version thereof;

(i) a heavy chain variable region comprising an amino acid sequence of SEQ ID NO: 71, a peptide having at least 70%, 75%, 80%, 85%, 90%, 95%, 98% or 99% sequence identity thereof, or a humanized version thereof, and a light chain variable region comprising an amino acid sequence of SEQ ID NO: 72, a peptide having at least 70%, 75%, 80%, 85%, 90%, 95%, 98% or 99% sequence identity thereof, or a humanized version thereof;

(j) a heavy chain variable region comprising an amino acid sequence of SEQ ID NO: 79, a peptide having at least 70%, 75%, 80%, 85%, 90%, 95%, 98% or 99% sequence identity thereof, or a humanized version thereof, and a light chain variable region comprising an amino acid sequence of SEQ ID NO: 80, a peptide having at least 70%, 75%, 80%, 85%, 90%, 95%, 98% or 99% sequence identity thereof, or a humanized version thereof;

(k) a heavy chain variable region comprising an amino acid sequence of SEQ ID NO: 87, a peptide having at least 70%, 75%, 80%, 85%, 90%, 95%, 98% or 99% sequence identity thereof, or a humanized version thereof, and a light chain variable region comprising an amino acid sequence of SEQ ID NO: 88, a peptide having at least 70%, 75%, 80%, 85%, 90%, 95%, 98% or 99% sequence identity thereof, or a humanized version thereof;

(l) a heavy chain variable region comprising an amino acid sequence of SEQ ID NO: 95, a peptide having at least 70%, 75%, 80%, 85%, 90%, 95%, 98% or 99% sequence identity thereof, or a humanized version thereof, and a light chain variable region comprising an amino acid sequence of SEQ ID NO: 96, a peptide having at least 70%, 75%, 80%, 85%, 90%, 95%, 98% or 99% sequence identity thereof, or a humanized version thereof;

(m) a heavy chain variable region comprising an amino acid sequence of SEQ ID NO: 103, a peptide having at least 70%, 75%, 80%, 85%, 90%, 95%, 98% or 99% sequence identity thereof, or a humanized version thereof, and a light chain variable region comprising an amino acid sequence of SEQ ID NO: 104, a peptide having at least 70%, 75%, 80%, 85%, 90%, 95%, 98% or 99% sequence identity thereof, or a humanized version thereof;

(n) a heavy chain variable region comprising an amino acid sequence of SEQ ID NO: 111, a peptide having at least 70%, 75%, 80%, 85%, 90%, 95%, 98% or 99% sequence identity thereof, or a humanized version thereof, and a light chain variable region comprising an amino acid sequence of SEQ ID NO: 112, a peptide having at least 70%, 75%, 80%, 85%, 90%, 95%, 98% or 99% sequence identity thereof, or a humanized version thereof; or

(o) a heavy chain variable region comprising an amino acid sequence of SEQ ID NO: 131 or a peptide having at least 70%, 75%, 80%, 85%, 90%, 95%, 98% or 99% sequence identity thereof, and a light chain variable region comprising an amino acid sequence selected from the group consisting of SEQ ID NO: 203-207 or a peptide having at least 70%, 75%, 80%, 85%, 90%, 95%, 98% or 99% sequence identity thereof;

(p) a heavy chain variable region comprising an amino acid sequence of SEQ ID NO: 116 or 248 or a peptide having at least 70%, 75%, 80%, 85%, 90%, 95%, 98% or 99% sequence identity thereof, and a light chain variable region comprising an amino acid sequence selected from the group consisting of SEQ ID NO: 208-216 and 249-250 or a peptide having at least 70%, 75%, 80%, 85%, 90%, 95%, 98% or 99% sequence identity thereof; or

(q) a heavy chain variable region comprising an amino acid sequence of SEQ ID NO: 152 or a peptide having at least 70%, 75%, 80%, 85%, 90%, 95%, 98% or 99% sequence identity thereof, and a light chain variable region comprising an amino acid sequence selected from the group consisting of SEQ ID NO: 232-238 or a peptide having at least 70%, 75%, 80%, 85%, 90%, 95%, 98% or 99% sequence identity thereof.

The antibody or an antigen-binding fragment thereof provided herein is selected from the group consisting of a full length antibody, a diabody, a scFv, an scFv dimer, a BsFv, a dsFv, a (dsFv)2, a dsFv-dsFv', an Fv fragment, a Fab, a Fab', a F(ab')2, a ds diabody, a nanobody, a domain antibody, an isolated CDR and a bivalent domain antibody.

According to specific embodiments, the antibodies are humanized. Humanized forms of non-human (***e.g., muri***ne) antibodies are chimeric molecules of immunoglobulins, immunoglobulin chains or fragments thereof (such as Fv, Fab, Fab', $F(ab')_2$ or other antigen-binding subsequences of antibodies) which contain minimal sequence derived from non-human immunoglobulin. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin (Jones et al., Nature, 321: 522-525 (1986); Riechmann et al., Nature, 332: 323-329 (1988); and Presta, Curr. Op. Struct. Biol., 2: 593-596 (1992)).

Methods for humanizing non-human antibodies are well known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers (Jones et al., Nature, 321: 522-525 (1986); Riechmann et al., Nature 332: 323-327 (1988); Verhoeyen et al., Science, 239: 1534-1536 (1988)), by substituting rodent CDRs or CDR sequences for the corresponding sequences of a human antibody. Accordingly, such humanized antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

A "conservative amino acid substitution" is one in which the amino acid residue is replaced with an amino acid residue having a similar side chain. Families of amino acid residues having similar side chains have been defined in the art, including basic side chains (*e.g., lysi*ne, arginine, histidine), acidic side chains (*e.g., aspa*rtic acid, glutamic acid), uncharged polar side chains (*e.g., glyc*ine, asparagine, glutamine, serine, threonine, tyrosine, cysteine), nonpolar side chains (*e.g., alan*ine, valine, leucine, isoleucine, proline, phenylalanine, methionine, tryptophan), beta-branched side chains (*e.g., thre*onine, valine, isoleucine) and aromatic side chains (*e.g., tyro*sine, phenylalanine, tryptophan, histidine). Thus, a nonessential amino acid residue in an immunoglobulin polypeptide is preferably replaced with another amino acid residue from the same side chain family. In another embodiment, a string of amino acids can be replaced with a structurally similar string that differs in order and/or composition of side chain family members.

Non-limiting examples of conservative amino acid substitutions are provided in the table below, where a similarity score of 0 or higher indicates conservative substitution between the two amino acids.

***Amino Acid Similarity Matrix***

| | C | G | P | S | A | T | D | E | N | Q | H | K | R | V | M | I | L | F | Y | W |
|---|---|---|---|---|---|---|---|---|---|---|---|---|---|---|---|---|---|---|---|---|
| **W** | -8 | -7 | -6 | -2 | -6 | -5 | -7 | -7 | -4 | -5 | -3 | -3 | 2 | -6 | -4 | -5 | -2 | 0 | 0 | 17 |
| **Y** | 0 | -5 | -5 | -3 | -3 | -3 | -4 | -4 | -2 | -4 | 0 | -4 | -5 | -2 | -2 | -1 | -1 | 7 | 10 | |
| **F** | -4 | -5 | -5 | -3 | -4 | -3 | -6 | -5 | -4 | -5 | -2 | -5 | -4 | -1 | 0 | 1 | 2 | 9 | | |
| **L** | -6 | -4 | -3 | -3 | -2 | -2 | -4 | -3 | -3 | -2 | -2 | -3 | -3 | 2 | 4 | 2 | 6 | | | |
| **I** | -2 | -3 | -2 | -1 | -1 | 0 | -2 | -2 | -2 | -2 | -2 | -2 | -2 | 4 | 2 | 5 | | | | |
| **M** | -5 | -3 | -2 | -2 | -1 | -1 | -3 | -2 | 0 | -1 | -2 | 0 | 0 | 2 | 6 | | | | | |
| **V** | -2 | -1 | -1 | -1 | 0 | 0 | -2 | -2 | -2 | -2 | -2 | -2 | -2 | 4 | | | | | | |
| **R** | -4 | -3 | 0 | 0 | -2 | -1 | -1 | -1 | 0 | 1 | 2 | 3 | 6 | | | | | | | |
| **K** | -5 | -2 | -1 | 0 | -1 | 0 | 0 | 0 | 1 | 1 | 0 | 5 | | | | | | | | |
| **H** | -3 | -2 | 0 | -1 | -1 | -1 | 1 | 1 | 2 | 3 | 6 | | | | | | | | | |
| **Q** | -5 | -1 | 0 | -1 | 0 | -1 | 2 | 2 | 1 | 4 | | | | | | | | | | |
| **N** | -4 | 0 | -1 | 1 | 0 | 0 | 2 | 1 | 2 | | | | | | | | | | | |
| **E** | -5 | 0 | -1 | 0 | 0 | 0 | 3 | 4 | | | | | | | | | | | | |
| **D** | -5 | 1 | -1 | 0 | 0 | 0 | 4 | | | | | | | | | | | | | |
| **T** | -2 | 0 | 0 | 1 | 1 | 3 | | | | | | | | | | | | | | |
| **A** | -2 | 1 | 1 | 1 | 2 | | | | | | | | | | | | | | | |
| **S** | 0 | 1 | 1 | 1 | | | | | | | | | | | | | | | | |
| **P** | -3 | -1 | 6 | | | | | | | | | | | | | | | | | |
| **G** | -3 | 5 | | | | | | | | | | | | | | | | | | |
| **C** | 12 | | | | | | | | | | | | | | | | | | | |

***Conservative Amino Acid Substitutions***

| For Amino Acid | Substitution With |
|---|---|
| Alanine | D-Ala, Gly, Aib, β-Ala, L-Cys, D-Cys |
| Arginine | D-Arg, Lys, D-Lys, Orn D-Orn |
| Asparagine | D-Asn, Asp, D-Asp, Glu, D-Glu Gln, D-Gln |
| Aspartic Acid | D-Asp, D-Asn, Asn, Glu, D-Glu, Gln, D-Gln |
| Cysteine | D-Cys, S-Me-Cys, Met, D-Met, Thr, D-Thr, L-Ser, D-Ser |
| Glutamine | D-Gln, Asn, D-Asn, Glu, D-Glu, Asp, D-Asp |
| Glutamic Acid | D-Glu, D-Asp, Asp, Asn, D-Asn, Gln, D-Gln |
| Glycine | Ala, D-Ala, Pro, D-Pro, Aib, β-Ala |
| Isoleucine | D-Ile, Val, D-Val, Leu, D-Leu, Met, D-Met |
| Leucine | Val, D-Val, Met, D-Met, D-Ile, D-Leu, Ile |
| Lysine | D-Lys, Arg, D-Arg, Orn, D-Orn |
| Methionine | D-Met, S-Me-Cys, Ile, D-Ile, Leu, D-Leu, Val, D-Val |
| Phenylalanine | D-Phe, Tyr, D-Tyr, His, D-His, Trp, D-Trp |
| Proline | D-Pro |
| Serine | D-Ser, Thr, D-Thr, allo-Thr, L-Cys, D-Cys |
| Threonine | D-Thr, Ser, D-Ser, allo-Thr, Met, D-Met, Val, D-Val |
| Tyrosine | D-Tyr, Phe, D-Phe, His, D-His, Trp, D-Trp |
| Valine | D-Val, Leu, D-Leu, Ile, D-Ile, Met, D-Met |

In certain embodiments, the humanized antibody or antigen-binding fragment provided herein comprises no more than 10, 9, 8, 7, 6, 5, 4, 3, 2, or 1 amino acid residue substitutions in each of the human FR sequences, or no more than 10, 9, 8, 7, 6, 5, 4, 3, 2, or 1 amino acid residue substitutions in all the FRs of a heavy or a light chain variable domain. In some embodiments, such change in amino acid residue could be present in heavy chain FR regions only, in light chain FR regions only, or in both chains.

In certain embodiments, the one or more amino acid residues are mutated, for example, back-mutated to the corresponding residue found in the non-human parent antibody (*e.g., in t*he mouse framework region) from which the CDR sequences are derived. Suitable positions for mutations can be selected by a skilled person following principles known in the art. For example, a position for mutation can be selected where: 1) the residue in the framework of the human germline sequence is rare (*e.g., in l*ess than 20%or less than 10% in human variable region sequence) ; 2) the position is immediately adjacent to one or more of the 3 CDR's in the primary sequence of the human germline chain, as it is likely to interact with residues in the CDRs; or 3) the position is close to CDRs in a 3-dimensional model, and therefore can have a good probability of interacting with amino acids in the CDR. The residue at the selected position can be mutated back to the corresponding residue in the parent antibody, or to a residue which is neither the corresponding residue in human germline sequence nor in parent antibody, but to a residue typical of human sequences, i.e., that occurs more frequently at that position in the known human sequences belonging to the same subgroup as the human germline sequence (see U.S. Pat. No. 5,693,762).

Back mutations can be introduced into the human germline framework sequence, if needed. In certain embodiments, the humanized antibody 29H8D3 may contain one or more back mutations selected from the group consisting of: A24V, V37M, M48I, R67K, V68A, M70L, T72A, T74K, M81I, R84N, R87T, D89E and Y95F, in the heavy chain framework of human germline sequence *IGHV1-18*01*. The humanized antibody 29H8D3 may contain one or more back mutations selected from the group consisting of: Y41F, L51V, D75A and Q105A in the light chain framework of human germline sequence *IGKV2-28*01*. Specific variable region sequences with the back-mutations can be found in **Table 7A**. In certain embodiments, the heavy chain variable region with one or more back mutations comprises an amino acid sequence selected from the group consisting of SEQ ID NO: 114-119. In certain embodiments, the light chain variable region with one or more back mutations comprises an amino acid sequence selected from the group consisting of SEQ ID NO: 122-123 and 201-202. Specific combinations of the heavy chain variable region and light chain variable region can be found in **Table 7B**.

In certain embodiments, the humanized antibody 29D2D7 may contain one or more back mutations selected from the group consisting of: R38K, M48I, R67K, V68A, M70L, T72A, T74K, R87T and D89E, in the heavy chain framework

of human germline sequence *IGHV1-18*01*. The humanized antibody 29D2D7 may contain one or more back mutations selected from the group consisting of: V3G, T5S and P49S, in the light chain framework of human germline sequence *IGKV4-1*01*. Specific variable region sequences with the back-mutations can be found in **Table 8A**. In certain embodiments, the heavy chain variable region with one or more back mutations comprises an amino acid sequence selected from the group consisting of SEQ ID NO: 126-132. In certain embodiments, the light chain variable region with one or more back mutations comprises an amino acid sequence selected from the group consisting of SEQ ID NO: 135-137. Specific combinations of the heavy chain variable region and light chain variable region can be found in **Table 8B**.

In certain embodiments, the humanized antibody 61C7F12 may contain S49A back mutation in the heavy chain framework of human germline sequence *IGHV3-21*01*. The humanized antibody 61C7F12 may contain one or more back mutations selected from the group consisting of: D1Q, Q3V, A44S, L48W, E71S, F72Y and T73S, in the light chain framework of human germline sequence *IGKV1-9*01*. Specific variable region sequences with the back-mutations can be found in **Table 9A**. In certain embodiments, the heavy chain variable region with one or more back mutations comprises an amino acid sequence of SEQ ID NO: 140. In certain embodiments, the light chain variable region with one or more back mutations comprises an amino acid sequence selected from the group consisting of SEQ ID NO: 143-145. Specific combinations of the heavy chain variable region and light chain variable region can be found in **Table 9B**.

In certain embodiments, the humanized antibody 69E3H11 may contain one or more back mutations selected from the group consisting of: R38K, M48I, R67K, V68A, I70L, R72V, T74K and R98L, in the heavy chain framework of human germline sequence *IGHV1-3*01*; or M70L, V79A and R87T in the heavy chain framework of human germline sequence *IGHV1-46*01*.The humanized antibody 69E3H11 may contain one or more back mutations selected from the group consisting of: K50Y, and F72Y, in the light chain framework of human germline sequence *IGKV6-21*01*. Specific variable region sequences with the back-mutations can be found in **Table 10A**. In certain embodiments, the heavy chain variable region with one or more back mutations comprises an amino acid sequence selected from the group consisting of SEQ ID NO: 150-152 and 154. In certain embodiments, the light chain variable region with one or more back mutations comprises an amino acid sequence selected from the group consisting of SEQ ID NO: 157-158. Specific combinations of the heavy chain variable region and light chain variable region can be found in **Table 10B**.

In certain embodiments, the humanized antibody 143H10E4 may contain one or more back mutations selected from the group consisting of: G27S, S30T, M48I, R67K, V68A, I70L, S84N and Y95F, in the heavy chain framework of human germline sequence *IGHV1-69*02*. The humanized antibody 143H10E4 may contain one or more back mutations selected from the group consisting of: L47P, L48W, K50Y, F72Y and T73S, in the light chain framework of human germline sequence *IGKV6-21*01*. Specific variable region sequences with the back-mutations can be found in **Table 11A**. In certain embodiments, the heavy chain variable region with one or more back mutations comprises an amino acid sequence selected from the group consisting of SEQ ID NO: 163-165. In certain embodiments, the light chain variable region with one or more back mutations comprises an amino acid sequence selected from the group consisting of SEQ ID NO: 167-169. Specific combinations of the heavy chain variable region and light chain variable region can be found in **Table 11B**.

In certain embodiments, the humanized antibody 152A1D12 may contain one or more back mutations selected from the group consisting of: R38K, M48I, R67K, V68A, I70L and R72V, in the heavy chain framework of human germline sequence *IGHV1-3*01*. The humanized antibody 152A1D12 may contain one or more back mutations selected from the group consisting of: L46R, L47W and F71Y in the hybrid light chain framework of the human germline sequences generated from a combination of the frameworks of *IGKV1-13*02* and *IGKV3-11*01*; or L47W and I58V, in the light chain framework of human germline sequence *IGKV3-11*01*. Specific variable region sequences with the back-mutations can be found in **Table 12A**. In certain embodiments, the heavy chain variable region with one or more back mutations comprises an amino acid sequence selected from the group consisting of SEQ ID NO: 174-176. In certain embodiments, the light chain variable region with one or more back mutations comprises an amino acid sequence selected from the group consisting of SEQ ID NO: 179-181 and 184. Specific combinations of the heavy chain variable region and light chain variable region can be found in **Table 12B**.

In certain embodiments, the humanized antibody 155B11C6 may contain one or more back mutations selected from the group consisting of: R38K, M48I, R67K, V68A, M70L, R72A and T74K, in the heavy chain framework of human germline sequence *IGHV1-2*02;* and M70L, R72A, T74K, S85G and R87T in the heavy chain framework of human germline sequence *IGHV1-46*01*. The humanized antibody 155B11C6 may contain one or more back mutations selected from the group consisting of: A44S, L47P, L48W, I59V, D71S and F72Y, in the light chain framework of human germline sequence *IGKV3-20*01*, and L47P, L48W, K50Y and F72Y in the light chain framework of human germline sequence *IGKV6-21*01*. Specific variable region sequences with the back-mutations can be found in **Table 13A**. In certain embodiments, the heavy chain variable region with one or more back mutations comprises an amino acid sequence selected from the group consisting of SEQ ID NO: 187-189 and 191. In certain embodiments, the light chain variable region with one or more back mutations comprises an amino acid sequence selected from the group consisting of SEQ ID NO: 194-196 and 199. Specific combinations of the heavy chain variable region and light chain variable region can be found in **Table 13B**.

In one embodiment, provided is an antibody or antigen binding fragment thereof that competes with 16C18 in

binding to the human CDH17 protein. In one embodiment, provided is an antibody or antigen binding fragment thereof that competes with 20C3E8 in binding to the human CDH17 protein. In one embodiment, provided is an antibody or antigen binding fragment thereof that competes with 29D2D7 in binding to the human CDH17 protein. In one embodiment, provided is an antibody or antigen binding fragment thereof that competes with 29H8D3 in binding to the human CDH17 protein. In one embodiment, provided is an antibody or antigen binding fragment thereof that competes with 61C7F12 in binding to the human CDH17 protein. In one embodiment, provided is an antibody or antigen binding fragment thereof that competes with 67A11B11 in binding to the human CDH17 protein. In one embodiment, provided is an antibody or antigen binding fragment thereof that competes with 69E3H11 in binding to the human CDH17 protein. In one embodiment, provided is an antibody or antigen binding fragment thereof that competes with 95F2C2F12 in binding to the human CDH17 protein. In one embodiment, provided is an antibody or antigen binding fragment thereof that competes with 103G6G1 in binding to the human CDH17 protein. In one embodiment, provided is an antibody or antigen binding fragment thereof that competes with 120B10C5 in binding to the human CDH17 protein. In one embodiment, provided is an antibody or antigen binding fragment thereof that competes with 143H10E4 in binding to the human CDH17 protein. In one embodiment, provided is an antibody or antigen binding fragment thereof that competes with 152A1D12 in binding to the human CDH17 protein. In one embodiment, provided is an antibody or antigen binding fragment thereof that competes with 155B11C6 in binding to the human CDH17 protein.

In one embodiment, provided is an antibody or antigen binding fragment thereof that binds to the same amino acid residues on the human CDH17 protein as 16C18. In one embodiment, provided is an antibody or antigen binding fragment thereof that binds to the same amino acid residues on the human CDH17 protein as 20C3E8. In one embodiment, provided is an antibody or antigen binding fragment thereof that binds to the same amino acid residues on the human CDH17 protein as 29D2D7. In one embodiment, provided is an antibody or antigen binding fragment thereof that binds to the same amino acid residues on the human CDH17 protein as 29H8D3. In one embodiment, provided is an antibody or antigen binding fragment thereof that binds to the same amino acid residues on the human CDH17 protein as 61C7F12. In one embodiment, provided is an antibody or antigen binding fragment thereof that binds to the same amino acid residues on the human CDH17 protein as 67A11B11. In one embodiment, provided is an antibody or antigen binding fragment thereof that binds to the same amino acid residues on the human CDH17 protein as 69E3H11. In one embodiment, provided is an antibody or antigen binding fragment thereof that binds to the same amino acid residues on the human CDH17 protein as 95F2C2F12. In one embodiment, provided is an antibody or antigen binding fragment thereof that binds to the same amino acid residues on the human CDH17 protein as 103G6G1. In one embodiment, provided is an antibody or antigen binding fragment thereof that binds to the same amino acid residues on the human CDH17 protein as 120B10C5. In one embodiment, provided is an antibody or antigen binding fragment thereof that binds to the same amino acid residues on the human CDH17 protein as 143H10E4. In one embodiment, provided is an antibody or antigen binding fragment thereof that binds to the same amino acid residues on the human CDH17 protein as 152A1D12. In one embodiment, provided is an antibody or antigen binding fragment thereof that binds to the same amino acid residues on the human CDH17 protein as 155B11C6.

In one embodiment, provided is an antibody or antigen binding fragment thereof that binds to the EC1 domain of the human CDH17 protein (see **Table A** for the domain regions). In one embodiment, provided is an antibody or antigen binding fragment thereof that binds to the EC2 domain of the human CDH17 protein. In one embodiment, provided is an antibody or antigen binding fragment thereof that binds to the EC3 domain of the human CDH17 protein. In one embodiment, provided is an antibody or antigen binding fragment thereof that binds to the EC4 domain of the human CDH17 protein. In one embodiment, provided is an antibody or antigen binding fragment thereof that binds to the EC5 domain of the human CDH17 protein. In one embodiment, provided is an antibody or antigen binding fragment thereof that binds to the EC6 domain of the human CDH17 protein. In one embodiment, provided is an antibody or antigen binding fragment thereof that binds to the EC7 domain of the human CDH17 protein.

In one embodiment, provided is an antibody or antigen binding fragment thereof that binds to the EC1-EC2 domain of the human CDH17 protein. In one embodiment, provided is an antibody or antigen binding fragment thereof that binds to the EC2-EC3 domain of the human CDH17 protein. In one embodiment, provided is an antibody or antigen binding fragment thereof that binds to the EC3-EC4 domain of the human CDH17 protein. In one embodiment, provided is an antibody or antigen binding fragment thereof that binds to the EC4-EC5 domain of the human CDH17 protein. In one embodiment, provided is an antibody or antigen binding fragment thereof that binds to the EC5-EC6 domain of the human CDH17 protein. In one embodiment, provided is an antibody or antigen binding fragment thereof that binds to the EC6-EC7 domain of the human CDH17 protein.

***Table A. CDH17 Domains***

| CDH17 Sequence (SEQ ID NO:231) | Residue |
|---|---|
| MILQAHLHSLCLLMLYLATGYG**QEGKFSGPLKPMTFSIYEGQEPSQIIFQ** | 50 |
| **FKANPPAVTFELTGETDNIFVIEREGLLYYNRALDRETRSTHNLQVAALD** | 100 |

(continued)

| CDH17 Sequence (SEQ ID NO:231) | Residue |
|---|---|
| EC1 (Q23-Q128) | |
| **ANGIIVEGPVPITIKVKDINDNRPTFLQ**SKYEGSVBQNSRPGKPFLYVNA | 150 |
| TDLDDPATPNGQLYYQIVIQLPMINNVMYFQINNKTGAISLTREGSQELN | 200 |
| EC2 (S129-P244) | |
| PAKNPSYNLVISVKDMGGQSENSFSDTTSVDIIVTENIWKAPKP**VEMVEN** | 250 |
| **STDPHPIKITQVRWNDPGAQYSLVDKEKLPRFPFSIDQEGDIYVTQPLDR** | 300 |
| EC3 (V245-C340) | |
| **EEKDAYVFYAVAKDEYGKPLSYPLEIHVKVKDINDNPPTC**PSPVTVFEVQ | 350 |
| ENERLGNSIGTLTAHDRDEENTANSFLNYRIVEQTPKLPMDGLFLIQTYA | 400 |
| EC4 (P341-F449) | |
| GMLQLAKQSLKKQDTPQYNLTIEVSDKDFKTLCFVQINVIDINDQIPIF**E** | 450 |
| **KSDYGNLTLAEDTNIGSTILTIQATDADEPFTGSSKILYHIIKGDSEGRL** | 500 |
| EC5 (E450-F566) | |
| **GVDTDPHTNTGYVIIKKPLDFETAAVSNIVFKAENPEPLVFGVKYNASSF** | 550 |
| **AKFTLIVTDVNEAPQF**SQHVEFQAKVSEDVAIGTKVGNVTAKDPEGLDISY | 600 |
| SLRGDTRGWLKIDHVTGEIFSVAPLDREAGSPYRVQVVATEVGGSSLSSV | 650 |
| EC6 (S567-L667) | |
| SEFHLILMDVNDNPPRL**AKDYTGLFFCHPLSAPGSLIFEATDDDQHLFRG** | 700 |
| **PHFTFSLGSGSLQNDWEVSKINGTHARLSTRHTEFEEREYVVLIRINDGG** | 750 |
| EC7 (A668-M787) | |
| **RPPLEGIVSLPVTFCSCVEGSCFRPAGHQTGIPTVGM**AVGILLTTLLVIG | 800 |
| IILAVVFIRIKKDKGKDNVESAQASEVKPLRS | 832 |

In certain embodiments, the humanized light and heavy chains of the present disclosure are substantially non-immunogenic in humans and retain substantially the same affinity as or even higher affinity than the parent antibody to CDH17.

It will also be understood by one of ordinary skill in the art that antibodies as disclosed herein may be modified such that they vary in amino acid sequence from the naturally occurring binding polypeptide from which they were derived. For example, a polypeptide or amino acid sequence derived from a designated protein may be similar*, e.g., have* a certain percent identity to the starting sequence*, e.g.,* it may be 60%, 70%, 75%, 80%, 85%, 90%, 95%, 98%, or 99% identical to the starting sequence.

Antibodies may be produced by a process of affinity maturation in which a modified antibody is generated that has an improvement in the affinity of the antibody for antigen, compared to an unmodified parent antibody. Affinity-matured antibodies may be produced by procedures known in the art*, e.g.,* Marks et al., Rio/Technology 10:779-783 (1992); Barbas et al. Proc Nat. Acad. Sci. USA 91 :3809-3813 (1994); Schier et al. Gene 169: 147-155 (1995); Yelton et al. J. Immunol. 155: 1994-2004 (1995); Jackson et al., J. Immunol. 154(7):331 0-15 9 (1995); and Hawkins et al, J. Mol. Biol. 226:889-896 (1992).

In some embodiments, one or more amino acid modifications may be introduced into the Fc domain, thereby generating an Fc domain variant. The Fc domain variant may comprise a human Fc domain sequence (*e.g., der*ived from human IgG1, IgG2, IgG3 or IgG4 Fc region) comprising an amino acid modification (*e.g., a su*bstitution) at one or more amino acid positions. In some embodiments, the Fc domain variant change one or more functional and/or pharmacokinetic properties of the antibody.

The Fc region can also be engineered to enhance or eliminate effector function. IgG antibodies can induce direct anti-tumor effects by way of indirect anti-tumor effects via the Fc-mediated effector functions that engage other immune cells or killer mechanisms. "Effector functions" or "antibody effector functions" as used herein refer to biological activities attributable to the binding of Fc region of an antibody to its effectors such as C1 complex and Fc receptor (FcγRIIa or FcγRIIIa). Exemplary effector functions include: complement dependent cytotoxicity (CDC) induced by interaction of antibodies and C1q on the C1 complex; antibody-dependent cell-mediated cytotoxicity (ADCC) induced by binding of Fc region of an antibody to Fc receptor on an effector cell; and antibody dependent cell mediated phagocytosis (ADCP) , where nonspecific cytotoxic cells that express Fcγ receptors (FcγRs) recognize bound antibody on a target cell and subsequently cause phagocytosis of the target cell.

In some embodiments, the Fc regions provided herein are maintained or improved with effector function, such as

ADCC and/or CDC.

Among the four IgG subclasses, IgG1 and IgG3 induce the strongest Fc-effector functions. However, since IgG1 has the longest half-life and is more stable than IgG3, most therapeutic antibodies with Fc-mediated functions are of IgG1 isotype.

IgG2 and IgG4 isotypes have significantly lower binding affinity to FcγRs. Recent evidence suggests that the IgG2 isotype is not completely devoid of effector function, whereas the IgG4 isotype can undergo in vivo Fab arm exchange leading to bispecific antibody and off-target effects.

***Antibody-drug conjugates***

In certain embodiments, the antibody comprises an amino acid sequence or one or more moieties not normally associated with an antibody. Exemplary modifications are described in more detail below. For example, an antibody of the disclosure may comprise a flexible linker sequence, or may be modified to add a functional moiety (*e.g., PEG,* a drug, a toxin, or a label).

Antibodies, variants, or derivatives thereof of the disclosure include derivatives that are modified, *i.e.*, by the covalent attachment of any type of molecule to the antibody such that covalent attachment does not prevent the antibody from binding to the epitope. For example, but not by way of limitation, the antibodies can be modified*, e.g., by g*lycosylation, acetylation, pegylation, phosphorylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the antibodies may contain one or more non-classical amino acids.

In some embodiments, the antibodies may be conjugated to therapeutic agents, prodrugs, peptides, proteins, enzymes, viruses, lipids, biological response modifiers, pharmaceutical agents, or PEG.

The antibodies may be conjugated or fused to a therapeutic agent, which may include detectable labels such as radioactive labels, an immunomodulator, a hormone, an enzyme, an oligonucleotide, a photoactive therapeutic or diagnostic agent, a cytotoxic agent, which may be a drug or a toxin, an ultrasound enhancing agent, a non-radioactive label, a combination thereof and other such agents known in the art.

The antibodies or antigen-binding fragments thereof provided herein can be conjugated or connected to one or more drug linker compounds, thereby forming an antibody-drug conjugate (ADC). The drug linker compound includes a linker and a payload (drug).

ADCs derived from the instantly disclosed new antibodies have been tested in the accompanying experimental examples and exhibited superior properties. In Example 6, the *in vitro* tumor cell killing activities of ADCs with vc-MMAE were measured. All ADCs with antibodies that bound to the distal domains (e.g., EC1, EC2, EC3, and EC4) exhibited excellent tumor cell killing activities, consistent with these antibodies' internalization efficiency.

Surprisingly, the ADCs can even have greater internalization efficiency than their naked antibody counterpart. For instance, when 29H8D3-z12 was conjugated to GGFG-DXd, the resulting ADC (29H8D3-z12-DXd) demonstrated higher internalization rates to CDH17-expressing tumor cells than the naked antibody (Example 8).

With such excellent internalization efficiency and cytotoxic activities, the tested ADC exhibited efficient bystander killing (Example 8) and in vivo anti-tumor efficacy (Examples 9 and 10), including to tumors with driver-gene mutations or resistant to chemotherapeutic drugs (Example 11).

The drug linker compound can be connected to the antibodies via a cysteine residue or a lysine residue on the antibodies. The ADC can be constructed via chemical approach such as stochastic conjugation on pre-existing lysine or cysteine residues via appropriate coupling reactions, such as amide coupling (of lysine) and sulfhydryl coupling (of cysteine). An active carboxylic acid ester (when available in the linker) is used to connect payloads to lysine residues on the antibody. The primary amine in Lys easily reacts with N-hydroxysuccinimide (NHS) esters introduced into the drug-linker, forming a stable amide. A typical IgG1 antibody molecule has roughly 90 Lys residues, of which approximately 30 can be modified for conjugation, implying that between 1 and 30 payloads can be covalently coupled to the antibody. As for cysteine, after reduction, the disulfide bond could transform to expose free cysteine residues which are accessible for coupling reactions, such as Michael additions, disulfide formation, and a-halo carbonyl alkylations.

In certain embodiments, the drug linker compound is connected to the antibodies provided herein via free thiols of the cysteine residues on the antibodies.

The ADC can also be constructed via enzymatic approach such as site-specific conjugation. The site-specific conjugation includes introduction of engineered reactive cysteine residues, disulfide re-bridging, unnatural amino acids, enzyme-assisted ligation, or glycan remodeling, glycoconjugation or click chemistry. Details of the methods of conjugation can be found in the art, such as Fu et al., Signal Transduction and Targeted Therapy 7:93 (2022).

In certain embodiments, the drug-antibody ratio (DAR) can be more than 2, more than 4, more than 6, more than 8, more than 10, more than 16, more than 20, or more than 30. In certain embodiments, the drug-antibody ratio (DAR) can be from 1-30, from 1-20, from 1-10, from 1-8, such as, 4-8, or 2-4. In certain embodiments, the drug-antibody ratio (DAR)

can be 2, 4, 6, 8, 10, 16, 20, or 30. In certain embodiments, the DAR is homogenous.

The linker contained in the drug-linker compound can be non-cleavable linker and cleavable linker. The cleavable linker mainly includes enzyme-cleavable linkers and chemically sensitive linkers.

Non-cleavable linkers consist of stable bonds that resist proteolytic degradation and ensure greater plasma stability. The mechanism of action of non-cleavable linkers is based on the internalization of the ADC complex followed by degradation of the mAb component in the lysosome, resulting in the release of a cytotoxic drug that kills tumor cells. They do not unleash cytotoxic agents at off-target sites and thus do not harm healthy cells. Non-cleavable linkers are divided into two groups, namely thioether or maleimidocaproyl (MC). Examples of the non-cleavable linkers include, but not limited to, 4-maleimidomethyl cyclohexane-1-carboxylate (MCC), succinimidyl-4-(N-maleimidomethyl) cyclohexane-1-carboxylate (SMCC), maleimidecaproyl (MC), and p-carboxycyclo hexylmethylmaleimide.

Cleavable linkers mainly include chemically sensitive linkers those are usually cleaved by environmental differences (such as redox potential, pH) and enzyme-cleavable linkers which are cleaved by specific enzymes in response to extracellular and intracellular environments.

Chemically sensitive linkers include, but not limited to, types of pH-sensitive linkers, and glutathione-sensitive disulfide linkers. (Khongorzul et al., Mol Cancer Res; 18(1) (2020)).

PH-sensitive linkers are a group of linkers that are sensitive to the acidic environment but are stable in the alkaline environment such as systemic circulation, such as hydrozone based linker. One successful example of ADC design using pH-sensitive linker is the IMMU-110 which is composed of a humanized anti-CD74 mAb conjugated to doxorubicin via acid-labile hydrazone.

Glutathione-sensitive disulfide linkers utilize difference in reduction potential in the cytoplasm in contrast to plasma. A high concentration of glutathione can be found in cancer cells than normal cells. Glutathione-sensitive linkers are stable in the blood flow and particularly cleaved by the elevated intracellular concentration of glutathione in the tumor cell, releasing the active drugs at the tumor sites from the nontoxic prodrugs (see *supra*).

Enzyme-cleavable linkers include, but not limited to, peptide based linkers, β-glucuronide based linkers and phosphate based linkers.

Peptide based linkers, also known as protease-sensitive linkers, are the most commonly used ADC linkers. These linkers can be cleaved by specific proteases extracellularly and/or intracellularly. For intracellular cleavage, as tumor cells exhibit high expression of lysosomal proteases like cathepsin B compared with the normal cells, therefore, proteases-sensitive peptide linker ADCs are selectively bound to and transformed into cancerous cells through receptor mediated endocytosis. The peptide linkers are stable in the systemic circulation and only unleash the drug in the target cells (see *supra*). Examples are Valine-citrulline (VC or Val-Cit), Valine-alanine (VA), phenylalanine-lysine (PL), and glycine-glycine-phenylalanine-glycine (GGFG).

β-glucuronide based linkers are recognized and hydrolyzed by β-glucuronidase or β-galactosidase for the drug release, while the β-glucuronidase and β-galactosidase are enriched in lysosomes and tumor necrotic regions. β-glucuronidase is inactive at physiologic pH (blood circulation) and active at lysosomal pH. Such selective site of action allows for the cleavage of the glycosidic linkage of the β-glucuronidase-sensitive β-glucuronide linker, thereby enabling the selective release of cytotoxic payloads (see *supra*).

Phosphate based linkers are a class of enzyme-cleavable linkers expressed exclusively to target enzymes in the lysosomal compartment. These linkers target pyrophosphatase and acid phosphatase enzymes, which hydrolyze pyrophosphates and terminal monophosphates into their respective alcohols.

Moieties that can be introduced as part of the linkers to facilitate the drug-linker connection or antibody-linker connection include maleimidocaproyl, maleimide-caproyl (MC) moiety, maleimide-methylene-cyclohexyl carbonyl moiety spacer, maleimide propoyl (MP), para-aminobenzylcarbamate (PABC) spacer, para-aminobenzyloxycarbamoyl (PABC) spacer, aminomethoxy methylenecarbonyl (-NH-CH2-217 O-CH2-CO-) spacer, 4-(4'-acetylphenoxy) butanoic acid moiety, acylhydrazide (-CO-NH-NH2) and thiol (-SH) moieties, Lys-PABC, PEG chain and PEG8.

Examples of the linkers include, but not limited to succinimidyl-4-(N-maleimidomethyl) cyclohexane-1-carboxylate (SMCC), sulfo-SMCC, MC-VC-PABC, CL2A, MC- GGFG, MC, or MP-PEG8-VA-PABC.

The drug/payload in the drug-linker compound can be a cytotoxin, a therapeutic peptide or polypeptide.

The peptide or polypeptide may be any peptide or polypeptide which has therapeutic properties, for example antinociceptive, antidiabetes, antitumor or antiviral activity. Additionally, or alternatively, the drug preferably comprises an amine group, a thiol group, or a carboxylic acid group, as these types of groups provide ideal sites for conjugation of the drug with the linker of the present disclosure. Examples of biologics drug are pseudomonas aeruginosa exotoxin PE38, diphtheria toxin, staphylococcus aureus enterotoxin A/E-120, shigatoxin, ricin, and urease.

The cytotoxin are activated after release from ADC inside the cytoplasm of tumor cells and are able to destroy the tumor cells. There are two main classes of cytotoxins that can be used in the ADC designs: microtubule-disrupting agents (such as auristatins and maytansinoids) and DNA-damaging agents (such as calicheamicins, duocarmycin, and doxorubicin). Examples included, but not limited to includes auristatins, maytansinoids, benzodiazepines, tubulysins, duocarmycin, calicheamicins, exatecans, irinotecans (SN38), doxorubicin, anthracycline, pyrrolobenzodiazepenes

(PBD), TLR agonist, and STING agonists.

Auristatins are synthetic antineoplastic agents derived from the natural product dolastatin 10. They block the tubulin polymerization process resulting in cell-cycle arrest and apoptosis. Examples include monomethyl auristatin E (MMAE) and monomethyl auristatin F (MMAF).

Maytansinoids are isolated from the maytansine, a benzoansamacrolide. These drugs inhibit tubulin polymerization. Examples include DM1 and DM4.

Calicheamicins are a class of enediyne antitumor antibiotics derived from the bacterium *Micromonospora echinospora*. Calicheamicin recognizes the minor groove of DNA and halts DNA replication resulting in mitotic arrest and cell death. One example is N-acetyl-calicheamicin, a derivative of calicheamicin.

Duocarmycin is a natural product derivative extracted from the bacteria *Streptomyces* strains. Duocarmycins are another class of DNA minor groove-binding alkylating agents. This class of drugs shows its action by binding to the minor groove of DNA and subsequently cause irreparable alkylation of DNA that disrupts the nucleic acid architecture and structural integrity.

Doxorubicin shows its action by intercalation of DNA that inhibits DNA synthesis. One example is IMMU-110.

Exatecans are synthetic derivatives of the natural cytotoxin, camptothecin, isolated from the Chinese tree *Camptotheca acuminata*. Like camptothecin, exatecan binds to the topoisomerase 1-DNA complex, preventing DNA religation which results in the accumulation of DNA strand breaks and ultimately leads to cell death. Examples of exatecans are DX-8951f and DXd. 7-ethyl-10-hydroxycamptothecin (SN-38) is also a derivative of camptothecin.

Further examples of the cytotoxin includes, without limitation, tublysin A, camptothecin, DGN462, Amberstatin269, anthramycin, SG3199/SCX, IRDye®700DX, TLR7/8 agonist, diABZI STING agonist-2.

The cytotoxin may be a chemotherapeutic agent which may be categorized by their mechanism of action into, for example, the following groups:

- anti-metabolites/anti-cancer agents such as pyrimidine analogs floxuridine, capecitabine, and cytarabine;
- purine analogs, folate antagonists, and related inhibitors;
- antiproliferative/antimitotic agents including natural products such as vinca alkaloid (vinblastine, vincristine) and microtubule such as taxane (paclitaxel, docetaxel), vinblastin, nocodazole, epothilones, vinorelbine (NAVELBINE®), and epipodophyllotoxins (etoposide, teniposide);
- DNA damaging agents such as actinomycin, amsacrine, busulfan, carboplatin, chlorambucil, cisplatin, cyclophosphamide (CYTOXAN®), dactinomycin, daunorubicin, doxorubicin, epirubicin, iphosphamide, melphalan, merchlorethamine, mitomycin, mitoxantrone, nitrosourea, procarbazine, taxol, taxotere, teniposide, etoposide, and triethylenethiophosphoramide;
- antibiotics such as dactinomycin, daunorubicin, doxorubicin, idarubicin, anthracyclines, mitoxantrone, bleomycins, plicamycin (mithramycin), and mitomycin;
- enzymes such as L-asparaginase which systemically metabolizes L-asparagine and deprives cells which do not have the capacity to synthesize their own asparagine;
- antiplatelet agents;
- antiproliferative/antimitotic alkylating agents such as nitrogen mustards cyclophosphamide and analogs (melphalan, chlorambucil, hexamethylmelamine, and thiotepa), alkyl nitrosoureas (carmustine) and analogs, streptozocin, and triazenes (dacarbazine);
- antiproliferative/antimitotic antimetabolites such as folic acid analogs (methotrexate);
- platinum coordination complexes (cisplatin, oxiloplatinim, and carboplatin), procarbazine, hydroxyurea, mitotane, and aminoglutethimide;
- hormones, hormone analogs (estrogen, tamoxifen, goserelin, bicalutamide, and nilutamide), and aromatase inhibitors (letrozole and anastrozole);
- anticoagulants such as heparin, synthetic heparin salts, and other inhibitors of thrombin;
- fibrinolytic agents such as tissue plasminogen activator, streptokinase, urokinase, aspirin, dipyridamole, ticlopidine, and clopidogrel;
- antimigratory agents;
- antisecretory agents (breveldin);
- immunosuppressives tacrolimus, sirolimus, azathioprine, and mycophenolate;
- compounds (TNP-470, genistein) and growth factor inhibitors (vascular endothelial growth factor inhibitors and fibroblast growth factor inhibitors);
- angiotensin receptor blockers, nitric oxide donors;
- anti-sense oligonucleotides;
- antibodies such as trastuzumab and rituximab;
- cell cycle inhibitors and differentiation inducers such as tretinoin;
- inhibitors, topoisomerase inhibitors (doxorubicin, daunorubicin, dactinomycin, eniposide, epirubicin, etoposide,

idarubicin, irinotecan, mitoxantrone, topotecan, and irinotecan), and corticosteroids (cortisone, dexamethasone, hydrocortisone, methylprednisolone, prednisone, and prednisolone);
- growth factor signal transduction kinase inhibitors;
- dysfunction inducers;
- toxins such as Cholera toxin, ricin, Pseudomonas exotoxin, Bordetella pertussis adenylate cyclase toxin, diphtheria toxin, and caspase activators;
- and chromatin.

Further examples of chemotherapeutic agents include:

- alkylating agents such as thiotepa and cyclophosphamide (CYTOXAN®);
- alkyl sulfonates such as busulfan, improsulfan, and piposulfan;
- aziridines such as benzodopa, carboquone, meturedopa, and uredopa;
- emylerumines and memylamelamines including alfretamine, triemylenemelamine, triethylenephosphoramide, triethylenethiophosphoramide, and trimemylolomelamine;
- acetogenins, especially bullatacin and bullatacinone;
- a camptothecin, including synthetic analog topotecan;
- bryostatin;
- callystatin;
- CC-1065, including its adozelesin, carzelesin, and bizelesin synthetic analogs;
- cryptophycins, particularly cryptophycin 1 and cryptophycin 8;
- dolastatin;
- duocarmycin, including the synthetic analogs KW-2189 and CBI-TMI;
- eleutherobin;
- pancratistatin;
- a sarcodictyin;
- spongistatin;
- nitrogen mustards such as chlorambucil, chlornaphazine, cyclophosphamide, estramustine, ifosfamide, mechlorethamine, mechlorethamine oxide hydrochloride, melphalan, novembichin, phenesterine, prednimustine, trofosfamide, and uracil mustard;
- nitrosoureas such as carmustine, chlorozotocin, foremustine, lomustine, nimustine, and ranimustine;
- antibiotics such as the enediyne antibiotics (*e.g., cali*cheamicin, especially calicheamicin gammaII and calicheamicin phiI1), dynemicin including dynemicin A, bisphosphonates such as clodronate, an esperamicin, neocarzinostatin chromophore and related chromoprotein enediyne antibiotic chromomophores, aclacinomycins, actinomycin, authramycin, azaserine, bleomycins, cactinomycin, carabicin, carrninomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin, and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, and zorubicin;
- anti-metabolites such as methotrexate and 5-fluorouracil (5-FU);
- folic acid analogs such as demopterin, methotrexate, pteropterin, and trimetrexate;
- purine analogs such as fludarabine, 6-mercaptopurine, thiamiprine, and thioguanine;
- pyrimidine analogs such as ancitabine, azacitidine, 6-azauridine, carmofur, cytarabine, dideoxyuridine, doxifluridine, enocitabine, and floxuridine;
- androgens such as calusterone, dromostanolone propionate, epitiostanol, mepitiostane, and testolactone;
- anti-adrenals such as aminoglutethimide, mitotane, and trilostane;
- folic acid replinishers such as frolinic acid;
- trichothecenes, especially T-2 toxin, verracurin A, roridin A, and anguidine;
- taxoids such as paclitaxel (TAXOL®) and docetaxel (TAXOTERE®);
- platinum analogs such as cisplatin and carboplatin;
- aceglatone; aldophosphamide glycoside; aminolevulinic acid; eniluracil; amsacrine; hestrabucil; bisantrene; edatraxate; defofamine; demecolcine; diaziquone; elformthine; elliptinium acetate; an epothilone; etoglucid; gallium nitrate; hydroxyurea; lentinan; leucovorin; lonidamine; maytansinoids such as maytansine and ansamitocins; mitoguazone; mitoxantrone; mopidamol; nitracrine; pentostatin; phenamet; pirarubicin; losoxantrone; fluoropyrimidine; folinic acid; podophyllinic acid; 2-ethylhydrazide; procarbazine; polysaccharide-K (PSK); razoxane; rhizoxin; sizofiran; spirogermanium; tenuazonic acid; triaziquone; 2,2',2''-tricUorotriemylamine; urethane; vindesine; dacarbazine; mannomustine; mitobronitol; mitolactol; pipobroman; gacytosine; arabinoside ("Ara-C"); cyclophosphamide;

thiopeta; chlorambucil; gemcitabine (GEMZAR®); 6-thioguanine; mercaptopurine; methotrexate; vinblastine; platinum; etoposide (VP-16); ifosfamide; mitroxantrone; vancristine; vinorelbine (NAVELBINE®); novantrone; teniposide; edatrexate; daunomycin; aminopterin; xeoloda; ibandronate; CPT-11; topoisomerase inhibitor RFS 2000; difluoromethylornithine (DFMO); retinoids such as retinoic acid; capecitabine; FOLFIRI (fluorouracil, leucovorin, and irinotecan);

- and pharmaceutically acceptable salts, acids, or derivatives of any of the above.

In certain embodiments of the present disclosure, the drug linker compound is a linker-cytotoxin structure of ozogamicin (derivatives of calicheamicin, see U.S. Pat. No. 5773001), vedotin (MC-VC-PABC-MMAE, see U.S. Pat. No. 7659241), mafodotin (MC-MMAF, see U.S. Pat. No. 7498298), emtansine (SMCC-DM1, U.S. Pat. No. 5208020), deruxtecan (MC-GGFG-DXd, see U.S. Pat. No. 10195288), govitecan (CL2A-SN38, see U.S. Pat. No. 8420086), or tesirine (MP-PEG8-VA-PABC-SG3199/SCX, see U.S. Pat. No. 9889207), which are hereby incorporated by reference in their entirety.

Further examples of the drug linker compound includes pasudotox (PE38), soravtansine (DM4), ravtansine (DM4), and mertansine (DM1).

In certain embodiments of the present disclosure, the drug linker compound is vedotin having a Formula I, or the derivatives thereof:

Formula I

(Mc-vc-PABC-MMAE).

In certain embodiments of the present disclosure, the drug linker compound is deruxtecan having a Formula II, or the derivatives thereof:

Formula II (Mc-GGFG-DXd).

In certain embodiments of the present disclosure, the drug linker compound is ozogamicin having a Formula III, or

the derivatives thereof:

Formula III

In certain embodiments of the present disclosure, the drug linker compound is mafodotin having a Formula IV, or the derivatives thereof:

Formula IV (MC-MMAF).

In certain embodiments of the present disclosure, the drug linker compound is emtansine having a Formula V, or the derivatives thereof:

Formula V (SMCC-DM1).

In certain embodiments of the present disclosure, the drug linker compound is govitecan having a Formula VI, or the derivatives thereof:

Formula VI

(CL2A-SN38).

In certain embodiments of the present disclosure, the drug linker compound is tesirine having a Formula VII, or the derivatives thereof:

Formula VII

(MP-PEG8-VA-PABC-SG3199/SCX).

In certain embodiments, both the vedotin and the deruxtecan can be connected to the antibodies of the present disclosure via the free thiols of the cysteines of the antibodies.

***Treatment and Uses***

As described herein, the antibodies, variants or derivatives, or the ADCs of the present disclosure may be used in certain treatment and diagnostic methods.

The present disclosure is further directed to antibody-based therapies which involve administering the antibodies of the disclosure to a patient such as an animal, a mammal, and a human for treating one or more of the disorders or conditions described herein. Therapeutic compounds of the disclosure include, but are not limited to, antibodies of the disclosure (including variants and derivatives thereof as described herein) and nucleic acids or polynucleotides encoding

antibodies of the disclosure (including variants and derivatives thereof as described herein).

In some embodiments, provided are methods for treating a cancer in a patient in need thereof. The method, in one embodiment, entails administering to the patient an effective amount of an antibody or antigen-binding fragment thereof, or the ADCs of the present disclosure.

In some embodiments, provided are uses of the antibodies or antigen-binding fragments thereof or the ADCs of the present disclosure in the manufacture of a medicament for treating a cancer in a patient in need thereof.

In some embodiments, provided are the antibodies or antigen-binding fragments thereof or the ADCs of the present disclosure for use in the treatment of a cancer in a patient in need thereof.

Unless stated otherwise, the terms "cancer" and "tumor" are used interchangeably herein. These terms in particular relate but are not limited to cancer and tumors selected from the group comprising basal cell carcinoma; bladder cancer; bone cancer such as osteosarcoma; central nervous system tumors such as cerebellar astrocytoma, cerebral astrocytoma/malignant glioma, craniopharyngioma, ependymoblastoma, ependymoma, medulloblastoma, medulloepithelioma, pineal parenchymal tumors of intermediate differentiation, primitive neuroectodermal tumors, pineoblastoma and spinal cord tumors; Burkitt's lymphoma; breast cancer; cervical cancer; chronic myelogenous leukemia; colon cancer; rectal cancer; colorectal cancer; esophageal cancer; Ewing family of tumors; extrahepatic bile duct cancer; gallbladder cancer; gastrointestinal stromal tumor (GIST); glioma; head and neck cancer; islet cell tumors; Kaposi sarcoma; leukemia; liver cancer; lymphoma; Hodgkin's lymphoma; non-Hodgkin's lymphoma; T-cell lymphoma; mesothelioma; multiple myeloma/plasma cell neoplasm; myeloid leukemia; multiple myeloma; nasopharyngeal cancer; neuroblastoma; small cell lung cancer; non-small cell lung cancer; oropharyngeal cancer; osteosarcoma; ovarian cancer; pancreatic cancer; parathyroid cancer; penile cancer; pharyngeal cancer; phaeochromocytoma; pituitary tumor; prostate cancer; renal cell cancer; respiratory tract carcinoma; retinoblastoma; skin cancer (melanoma); small intestine cancer; soft tissue sarcoma; squamous cell carcinoma; squamous neck cancer; stomach (gastric) cancer; testicular cancer; throat cancer; thyroid cancer; transitional cell cancer of the renal pelvis and ureter; urethral cancer; uterine cancer; vaginal cancer; vulvar cancer and Wilms tumor.

Additional diseases or conditions associated with increased cell survival, that may be treated, prevented, diagnosed and/or prognosed with the antibodies or variants, or derivatives thereof of the disclosure include, but are not limited to, progression, and/or metastases of malignancies and related disorders such as leukemia (including acute leukemias (*e.g., acut*e lymphocytic leukemia, acute myelocytic leukemia (including myeloblastic, promyelocytic, myelomonocytic, monocytic, and erythroleukemia)) and chronic leukemias (*e.g., chro*nic myelocytic (granulocytic) leukemia and chronic lymphocytic leukemia)), polycythemia vera, lymphomas (*e.g., Hodg*kin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyo sarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma and retinoblastoma.

A specific dosage and treatment regimen for any particular patient will depend upon a variety of factors, including the particular antibodies, variant or derivative thereof or the ADCs used, the patient's age, body weight, general health, sex, and diet, and the time of administration, rate of excretion, drug combination, and the severity of the particular disease being treated. Judgment of such factors by medical caregivers is within the ordinary skill in the art. The amount will also depend on the individual patient to be treated, the route of administration, the type of formulation, the characteristics of the compound used, the severity of the disease, and the desired effect. The amount used can be determined by pharmacological and pharmacokinetic principles well known in the art.

Methods of administration of the antibodies, variants or the ADCs include but are not limited to intradermal, intramuscular, intraperitoneal, intravenous, subcutaneous, intranasal, epidural, and oral routes. The antigen-binding polypeptides or compositions may be administered by any convenient route, for example by infusion or bolus injection, by absorption through epithelial or mucocutaneous linings (*e.g., oral* mucosa, rectal and intestinal mucosa, etc.) and may be administered together with other biologically active agents. Thus, pharmaceutical compositions containing the antigen-binding polypeptides of the disclosure may be administered orally, rectally, parenterally, intracistemally, intravaginally, intraperitoneally, topically (as by powders, ointments, drops or transdermal patch), bucally, or as an oral or nasal spray.

The term "parenteral" as used herein refers to modes of administration which include intravenous, intramuscular, intraperitoneal, intrasternal, subcutaneous and intra-articular injection and infusion.

Administration can be systemic or local. In addition, it may be desirable to introduce the antibodies of the

disclosure into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed*, e.g., by u*se of an inhaler or nebulizer, and formulation with an aerosolizing agent.

It may be desirable to administer the antigen-binding polypeptides or compositions of the disclosure locally to the area in need of treatment; this may be achieved by, for example, and not by way of limitation, local infusion during surgery, topical application*, e.g., in c*onjunction, with a wound dressing after surgery, by injection, by means of a catheter, by means of a suppository, or by means of an implant, said implant being of a porous, non-porous, or gelatinous material, including membranes, such as sialastic membranes, or fibers. Preferably, when administering a protein, including an antibody, of the disclosure, care must be taken to use materials to which the protein does not absorb.

Methods of detecting expression of a human CDH17 protein in a sample are also provided, in some embodiments, comprising contacting the sample with the antibody or fragment thereof, and detecting the binding which indicates expression of CDH17 in the sample.

In certain embodiments, provided are uses of the antibodies or antigen-binding fragments thereof of the present disclosure in the manufacture of a kit for detecting expression of a human CDH17 protein in a sample.

### *Polynucleotides Encoding the Antibodies and Methods of Preparing the Antibodies*

The present disclosure also provides isolated polynucleotides or nucleic acid molecules encoding the antibodies, variants or derivatives thereof of the disclosure. The polynucleotides of the present disclosure may encode the entire heavy and light chain variable regions of the antigen-binding polypeptides, variants or derivatives thereof on the same polynucleotide molecule or on separate polynucleotide molecules. Additionally, the polynucleotides of the present disclosure may encode portions of the heavy and light chain variable regions of the antigen-binding polypeptides, variants or derivatives thereof on the same polynucleotide molecule or on separate polynucleotide molecules.

Methods of making antibodies are well known in the art and described herein. In certain embodiments, both the variable and constant regions of the antigen-binding polypeptides of the present disclosure are fully human. Fully human antibodies can be made using techniques described in the art and as described herein. For example, fully human antibodies against a specific antigen can be prepared by administering the antigen to a transgenic animal which has been modified to produce such antibodies in response to antigenic challenge, but whose endogenous loci have been disabled. Exemplary techniques that can be used to make such antibodies are described in U.S. patents: 6,150,584; 6,458,592; 6,420,140 which are incorporated by reference in their entireties.

### *Compositions*

The present disclosure also provides pharmaceutical compositions. Such compositions comprise an effective amount of an antibody, and an acceptable carrier. In some embodiments, the composition further includes a second anticancer agent (*e.g., an i*mmune checkpoint inhibitor).

In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. Further, a "pharmaceutically acceptable carrier" will generally be a non-toxic solid, semisolid or liquid filler, diluent, encapsulating material or formulation auxiliary of any type.

The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents such as acetates, citrates or phosphates. Antibacterial agents such as benzyl alcohol or methyl parabens; antioxidants such as ascorbic acid or sodium bisulfite; chelating agents such as ethylenediaminetetraacetic acid; and agents for the adjustment of tonicity such as sodium chloride or dextrose are also envisioned. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in Remington's Pharmaceutical Sciences by E. W. Martin, incorporated herein by reference. Such compositions will contain a therapeutically effective amount of the antigen-binding polypeptide, preferably in purified form, together with a suitable amount

of carrier so as to provide the form for proper administration to the patient. The formulation should suit the mode of administration. The parental preparation can be enclosed in ampoules, disposable syringes or multiple dose vials made of glass or plastic.

In an embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds of the disclosure can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

## EXAMPLES

### Example 1. Generation of Mouse Anti-Human CDH17 Antibodies

This example describes the generation of mouse anti-human CDH17 monoclonal antibodies using the hybridoma technology.

*Immunogen:* Full length extracellular domain (ECD) and truncated human CDH17 proteins were used as immunogens during the mice immunization process. Briefly, full length ECD of human CDH17 protein (UniProt reference sequence: Q12864, Q23-M787) were fused with his tag at the C-terminal and referred as hCDH17-his (Acro Bio, Cat. No. CA7H52H3; or customized by Biointron). The truncated human CDH17 protein was generated by fusing the EC6 (S567-L667) and EC7 regions (A668-M787) of human CDH17 with N-terminal of the Fragment C (Fc) region of human Immunoglobulin G (IgG) (hereafter referred as hCDH17 EC6-7-hFc, customized by Biointron).

*Scheme of mouse immunization:* To generate mouse monoclonal antibodies to human CDH17, BALB/c, AJ and SJL mice were immunized with hCDH17-his or hCDH17 EC6-7-hFc protein intraperitoneally or subcutaneously at a biweekly interval. Serum titers of immunized mice were monitored by ELISA against human hCDH17-his protein or hCDH17 EC6-7-hFc and FACS against human CDH17 overexpressed on HEK293 cell line (HEK293-hCDH17, customized by Genomeditech) while HEK293 parental cell line served as the negative control. After 3-6 rounds of immunization, mice with sufficient titers were boosted with hCDH17-his protein and selected for fusions.

*Cell fusion and hybridoma screening:* Splenocytes from the selected mice were fused with mouse myeloma cell line Sp2/0 by electrofusion. These hybridoma cells were then plated in 96 well flat-bottom microplates and allowed to secrete mouse antibodies in the supernatant. During primary screening, protein binding to hCDH17-his by ELISA and cell binding to HEK293-hCDH17 by FACS were used to screen positive clones in a high-throughput way. A following confirmative screening was pursued to identify the clones binding to cynomolgus CDH17 ECD protein (Isoform 1, NCBI reference sequence: XP_005563762.1, M1-M787) fused with Fc tag (cynoCDH17-hFc, Sino Bio, Cat. No. 90147-C02H) by ELISA.

*Subcloning screening and sequencing:* Positive primary clones meeting with the above criteria from each fusion were subcloned by limiting dilution to ensure that hybridoma subclones were derived from a single parental cell. Subcloning were screened with the same criteria as primary clone screening as described above. The subclones with efficient binding potency to human CDH17 were selected for subsequent sequencing.

The resulting variable regions of the mouse antibodies were fused with the constant region of human IgG1 to generate chimeric CDH17 mAbs. The DNA sequences of chimeric antibodies were cloned into the pcDNA3.4 plasmid and expressed in CHO-K1 cells followed by purification of antibodies from the culture supernatant by Protein A affinity chromatography column or beads. The purified chimeric antibodies were subjected to serial *in vitro* screening processes to determine affinity, binding ability, epitope, specificity, and species cross-reactivity.

A series of CDH17 chimeric mAbs, including 16C18, 20C3E8, 29D2D7, 29H8D3, 61C7F12, 67A11B11, 69E3H11, 95F2C2F12, 103G6G1, 120B10C5, 134C7B1, 143H10E4, 152A1D12 and 155B11C6, was selected for further analysis based on the performance in screening assays. The amino acid sequences of the variable regions of the selected chimeric CDH17 antibodies are provided in **Table 1** as below, with the CDR sequences summarized in **Table 2A** and **2B**.

***Table 1. Sequences of the Variable Region of CDH17 Chimeric mAbs (Underline indicates CDRs)***

| Name | Sequence | SEQ ID NO: |
|---|---|---|
| 16C18 VH | QVQLQQSGAELAKPGTSVKMSCKASGYNFTNYWMNWVKQRPGQGLEWIGTFNPSYGYTEYNQKFKDKAILTADKSSSTAYMQLNSLTSEDSAVYYCARRSSHFDYWGQGTTLTVSS | 7 |
| 16C18 VL | DIVMSQSPSSLAVSAGEKVTMSCKSSQSLLNSRTRTNYLAWYQQKPGQSPKLLIYWASTRESGVPDRFTGSGSGTDFTLTISSVQAEDLAVYYCKQSYNLPTFGGGTKLEIK | 8 |
| 20C3E8 VH | QVQLQQSGAELARPGASVKLSCKASGYTFTSYGISWVKQRTGQGLEWIGEIYPRSGNTYYNEKFKGKATLTADKSSSTAYMELRSLTSEDSAVYFCSRLDYGTSPFDYWGQGTTLTVSS | 15 |
| 20C3E8 VL | NIVMTQSPKSMSMSVGERVTLSCKASENVGTFVSWYQHKPEQSPKLLIYGASNRYTGVPDRFTGSGSATDFTLTISSVQAEDLADYHCGQSYSYPYTFGGGTKLEIK | 16 |
| 29D2D7 VH | QVQLQQSGAELVRPGASVTLSCKASGYTFTDYDMHWVKQTPVHGLEWIGGFDPESAGTVYNLRFKDKAILTADKSSSTAYMELRSLTSEDSAVYYCARKGYYSVSSPFTYWGQGTLVSVSA | 23 |
| 29D2D7 VL | DIGMSQSPSSLAVSVGEKVTMSCKSSQSLLYSSNQKNYLAWYQQKPGQSPKLLIYWASTRESGVPDRFTGSGSGTDFTLTINSVKAEDLAVYYCQQCYSYPRTFGGGTKLEIK | 24 |
| 29H8D3 VH | QVQLQQSDAELVKPGASVKISCKVSGYTFTDHTIHWMKQRPEQGLEWIGYIYPRDGYTKYNEKFKDKATLTADKSSSTAYIQLNTLTSEDSAVYFCARFYYGYWYFDVWGTGTTVTVSS | 31 |
| 29H8D3 VL | DIVMTQAAPSVPVTPGESVSISCRSSKSLLRSNGNTYLYWFLQRPGQSPQVLIYRMSNLASGVPDRFSGSGSGTAFTLRISRVEAEDVGVYYCMQHLEYPFTFGAGTKLELK | 32 |
| 61C7F12 VH | EVQLVESGGGLVKPGGSLKLSCAASGFTFSSYAMSWVRQTPEKRLEWVATINIGGSYTYYPDSVKGRFTISRDNAKNTLYLQMSSLRSEDTAMYYCARQEGRYWYFDVWGAGTTVTVSS | 39 |
| 61C7F12 VL | QIVLTQSPAIMSASLGERVTMTCTASSSVSSSYLHWYQQKPGSSPKLWIYSTSNLASGVPARFSGSGSGTSYSLTISSMEAEDAATYYCHQFHRSPYTFGGGTKLEIK | 40 |
| 67A11B11 VH | EIQLQQSGPELVKPGASVKVSCKASGYAFTTYNMYWVKQSHGQSLEWIGYIDPYNGVTIYNQKFKGTATLTVDKSSSTAYMHLNSLSSEDSAVYYCTITAATLDAMDYWGQGTSVTVSS | 47 |
| 67A11B11 VL | DIVMTQSHKFMSTSVGDRVSITCKASQNVATAVAWYQQKPGQSPKVLIYWTSTRHNGVPDRFTGSGSGTDFTLTISNVQSEDLADYFCQQYNSHPLTFGAGTRLELK | 48 |
| 69E3H11 VH | QVQLQQPGAELVKPGASVKLSCKASGYTFTTYWMHWVKQRPGQGLEWIGEIDPSDSYANYNQKFKGKATLTVDKSSSTAYMQLSSLTSEDSAVYYCALYYGYGDWGQGTTLTVSS | 55 |

(continued)

| Name | Sequence | SEQ ID NO: |
|---|---|---|
| 69E3H11 VL | EIVLTQSPTTMAASPGEKIIITCSVSSSISSIHLHWYQQKPGFSPKLLIYRTSNLASGVPTRFSGSGSGTSYSLTIGTMEAEDVATYYCQQGSNLPLTFGAGTRLELK | 56 |
| 95F2C2F12 VH | QVQLQQSGAELMKPGASVKLSCKATGYTFTGYWIEWIKQRPGHGLEWIGEILPGSGSTNYNEKFKGKATFTADTSSNTAYMELSSLTTEDSAIYYCARAGYNNWGQGTTLTVSS | 63 |
| 95F2C2F12 VL | DIQMTQSPSSLSASLGERVSLTCRASQDIGRSVNWLQQEPDGTIKRLIYATSSLESGVPKRFSGSRSGSDYFLTISSLESEDFVDYYCLQYASSPLTFGAGTKLELK | 64 |
| 103G6G1 VH | EVKLVESEGGLVQPGSSMKLSCTASGFTFSDFYMAWVRQVPEKGLEWVANINYDSTITYYLDSLKSRFIISRDNAENILYLQMSSLKSEDTATYYCARLLYYYGSLYFDVWGTGTTVTVSS | 71 |
| 103G6G1 VL | DIQMTQSPASLSASVGETVTITCRASENIYSYLAWYQQKQGKSPQLLVYDAKTLAEGVPSRFSGSGSGTQFSLKINSLQPEDFGSYYCQHHYGIPITFGAGTNLELK | 72 |
| 120B10C5 VH | QVQLQQSGAELAKPGASVKLSCRTSGYTFTNYWMHWVKQRPGQGLEWIGYINPSSGYTKYNQKFKDKATLTADKSSSTAYMQLSSLTYEDSAVHYCARSLNYYDSGYWYFDVWGTGTAVTVSS | 79 |
| 120B10C5 VL | DIVLTQSPASLAVSLGQRATISCRASQSVSTSSYSYMHWYQQKPGQPPKLLIKYASNLESRVPARFSGSGSGTDFTLNIHPVEEEDSATYYCQHSWEIPWTFGGGTKLEIK | 80 |
| 134C7B1 VH | AVQLQQSGPELLKPGASVKISCKASGYTFTDYTMHWVKQSHGKSLEWIGGINPNYGGTSYNETFKDKATLTVDKSSNTAYMELRSLTSEDSAVYYCARIPYGSYPMDYWGQGTSVTVSS | 87 |
| 134C7B1 VL | DIVMSQSPSSLTVSVGEKVTMSCKSSQSLLYSSNQKIYLAWYQQKPGQSPKLLISWASTRESGVPDRFTGSGSGTDFTLTISSVKAEDLADYYCQQYYRYRTFGGGTKLEIK | 88 |
| 143H10E4 VH | QVQLQQSDAELVKPGASVKISCKASGSTFTDHTIHWVKQRPEQGLEWIGYIYPVYGYTQNNEKFKGKATLTADKSSSTAYMQLNSLTSEDSAVYFCARSRDSNPYYFDYWGQGTTLTVSS | 95 |
| 143H10E4 VL | EIVLTQSPALMAASPGEKVTITCTVSSSISSSNLHWYQQKSGTSPKPWIYGTSNLASGVPVRFSGSGSGTSYSLTISSMEAEDAATYYCQQWSSYPFTFGSGTKLKMK | 96 |
| 152A1D12 VH | EVQLQQSGPELVKPGASVKISCKASGYTFTDYNMHWVKQSHGKSLEWIGYIYRNNGYNGYNQKFKNKATLTVDSSSSTAYMELRSLTSEDSAVYYCARNYWRGAMDYWGQGTSVTVSS | 103 |
| 152A1D12 VL | QIVLTQSPAIMSASPGEKVTMTCSASSSISYMHWYQQKPGTSPKRWIYDTSKLASGVPARFSGSGSGTSYSLTISSMEAEDAGTYYCHQRSSYPWTFGGGTKLEIK | 104 |

(continued)

| Name | Sequence | SEQ ID NO: |
|---|---|---|
| 155B11C6 VH | QVQLQQSGAELARPGASVKMSCKASGYTFTSYTMHWVKQRPGQGLEWIGYIDPKSDYNYYNQKFKDKATLTADKSSSTAYMQLSGLTSEDSTVYYCARGFFKGYFDVWGAGTTVTVSS | 111 |
| 155B11C6 VL | EIVLTQSPPLMAASPGEKVTITCSVSSSISSSNLHWYQQKSETSPKPWIYGTSNLASGVPIRFSGSGSGTSYSLTISSMEAEDAATYYCQQWTSYPFTFGAGTKLELK | 112 |

*Table 2A. Sequences of CDRs in Heavy Chain of CDH17 Chimeric mAbs (Kabat numbering)*

| Antibody | VH CDR1 | SEQ ID NO: | VH CDR2 | SEQ ID NO: | VH CDR3 | SEQ ID NO: |
|---|---|---|---|---|---|---|
| 16C18 | NYWMN | 1 | TFNPSYGYTEYNQKFKD | 2 | RSSHFDY | 3 |
| 20C3E8 | SYGIS | 9 | EIYPRSGNTYYNEKFKG | 10 | LDYGTSPFDY | 11 |
| 29D2D7 | DYDMH | 17 | GFDPESAGTVYNLRFKD | 18 | KGYYSVSSPFTY | 19 |
| 29H8D3 | DHTIH | 25 | YIYPRDGYTKYNEKFKD | 26 | FYYGYWYFDV | 27 |
| 61C7F12 | SYAMS | 33 | TINIGGSYTYYPDSVKG | 34 | QEGRYWYFDV | 35 |
| 67A11B11 | TYNMY | 41 | YIDPYNGVTIYNQKFKG | 42 | TAATLDAMDY | 43 |
| 69E3H11 | TYWMH | 49 | EIDPSDSYANYNQKFKG | 50 | YYGYGD | 51 |
| 95F2C2F12 | GYWIE | 57 | EILPGSGSTNYNEKFKG | 58 | AGYNN | 59 |
| 103G6G1 | DFYMA | 65 | NINYDSTITYYLDSLKS | 66 | LLYYYGSLYFDV | 67 |
| 120B10C5 | NYWMH | 73 | YINPSSGYTKYNQKFKD | 74 | SLNYYDSGYWYFDV | 75 |
| 134C7B1 | DYTMH | 81 | GINPNYGGTSYNETFKD | 82 | IPYGSYPMDY | 83 |
| 143H10E4 | DHTIH | 89 | YIYPVYGYTQNNEKFKG | 90 | SRDSNPYYFDY | 91 |
| 152A1D12 | DYNMH | 97 | YIYRNNGYNGYNQKFKN | 98 | NYWRGAMDY | 99 |
| 155B11C6 | SYTMH | 105 | YIDPKSDYNYYNQKFKD | 106 | GFFKGYFDV | 107 |

*Table 2B. Sequences of CDRs in Light Chain of CDH17 Chimeric mAbs (Kabat numbering)*

| Antibody | VL CDR1 | SEQ ID NO: | VL CDR2 | SEQ ID NO: | VL CDR3 | SEQ ID NO: |
|---|---|---|---|---|---|---|
| 16C18 | KSSQSLLNSRTRTNYLA | 4 | WASTRES | 5 | KQSYNLPT | 6 |
| 20C3E8 | KASENVGTFVS | 12 | GASNRYT | 13 | GQSYSYPYT | 14 |
| 29D2D7 | KSSQSLLYSSNQKNYLA | 20 | WASTRES | 21 | QQCYSYPRT | 22 |
| 29H8D3 | RSSKSLLRSNGNTYLY | 28 | RMSNLAS | 29 | MQHLEYPFT | 30 |
| 61C7F12 | TASSSVSSSYLH | 36 | STSNLAS | 37 | HQFHRSPYT | 38 |
| 67A11B11 | KASQNVATAVA | 44 | WTSTRHN | 45 | QQYNSHPLT | 46 |
| 69E3H11 | SVSSSISSIHLH | 52 | RTSNLAS | 53 | QQGSNLPLT | 54 |
| 95F2C2F12 | RASQDIGRSVN | 60 | ATSSLES | 61 | LQYASSPLT | 62 |
| 103G6G1 | RASENIYSYLA | 68 | DAKTLAE | 69 | QHHYGIPIT | 70 |
| 120B10C5 | RASQSVSTSSYSYMH | 76 | YASNLES | 77 | QHSWEIPWT | 78 |
| 134C7B1 | KSSQSLLYSSNQKIYLA | 84 | WASTRES | 85 | QQYYRYRT | 86 |
| 143H10E4 | TVSSSISSSNLH | 92 | GTSNLAS | 93 | QQWSSYPFT | 94 |

(continued)

| Antibody | VL CDR1 | SEQ ID NO: | VL CDR2 | SEQ ID NO: | VL CDR3 | SEQ ID NO: |
|---|---|---|---|---|---|---|
| 152A1D12 | SASSSISYMH | 100 | DTSKLAS | 101 | HQRSSYPWT | 102 |
| 155B11C6 | SVSSSISSSNLH | 108 | GTSNLAS | 109 | QQWTSYPFT | 110 |

**Example 2. Binding Activity of the Chimeric Monoclonal Antibodies Targeting CDH17**

***2.1. ELISA binding to human CDH17***

In order to determine the binding ability of the chimeric mAbs to human CDH17 protein, ELISA-based binding assay was performed as follows. In brief, hCDHl7-his protein was diluted with 1 ×ELISA coating buffer (Solarbio, Cat. No. C1050) at 2 μg/mL and adsorbed to wells of 96-well microplates overnight at 4 °C. After blocking the wells with 1% bovine serum albumin (BSA) to prevent non-specific binding, the CDH17 chimeric mAbs or an isotype control were titrated at a 4-fold dilution rate starting from 100 nM and added to the wells pre-adsorbed with the antigen. The mixture was incubated for 1 hour at room temperature (RT). The bound CDH17 mAbs were recognized by a detective antibody against human IgG Fc which was conjugated with horseradish peroxidase (HRP) (Jackson Immuno, Cat. No. 109-035-008). Tetramethylbenzidine (TMB), a substrate of HRP, was added to the wells to visualize the binding signal. After sufficient color development, stop solution was added to the wells. The absorbance of the signal was detected at 450 nm with Envision multilabel plate readers (PerkinElemer). The graph and statistics analysis were generated with four-parameter nonlinear regression curve fit in Graphpad Prism 9 software.

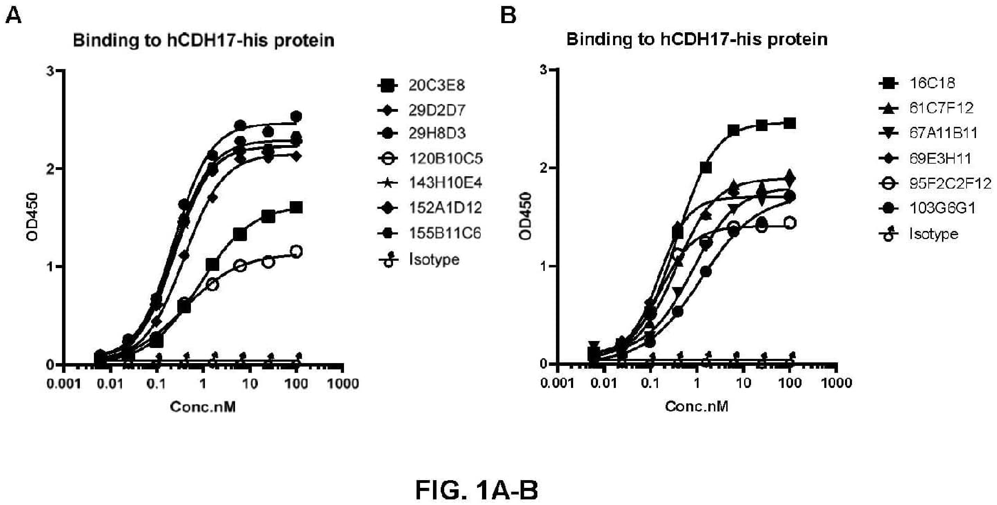

As shown in FIG. 1, all the CDH17 chimeric mAbs efficiently bound to human CDH17 protein. The EC50 and the maximum value of the binding curve of each antibody were summarized in **Table 3.**

***Table 3. Binding Activities of the CDH17 Antibodies to Antigen***

| Antigen / Antibody | Human CDH17 EC50 (nM) | Human CDH17 Cmax (Top OD450nm) |
|---|---|---|
| 16C18 | 0.371 | 2.456 |
| 20C3E8 | 0.844 | 1.606 |
| 29D2D7 | 0.390 | 2.128 |
| 29H8D3 | 0.238 | 2.534 |
| 61C7F12 | 0.354 | 1.940 |
| 67A11B11 | 0.795 | 1.846 |
| 69E3H11 | 0.157 | 1.693 |
| 95F2C2F12 | 0.157 | 1.440 |
| 103G6G1 | 1.239 | 1.714 |
| 120B10C5 | 0.381 | 1.154 |
| 143H10E4 | 0.249 | 2.278 |
| 152A1D12 | 0.243 | 2.278 |
| 155B11C6 | 0.234 | 2.323 |

***2.2. ELISA binding to human CDH16***

Since CDH16 and CDH17 belong to the same 7D-cadherin subfamily, it is important to identify the specificity of the CDH17 chimeric monoclonal antibodies.

To determine the non-specific binding of the chimeric mAbs to human CDH16 protein (hCDH16-his, Novoprotein, Cat. No. CJ16), ELISA binding assay was performed as previously described. The hCDH16-his protein was used as the coating antigen at 1 μg/mL.

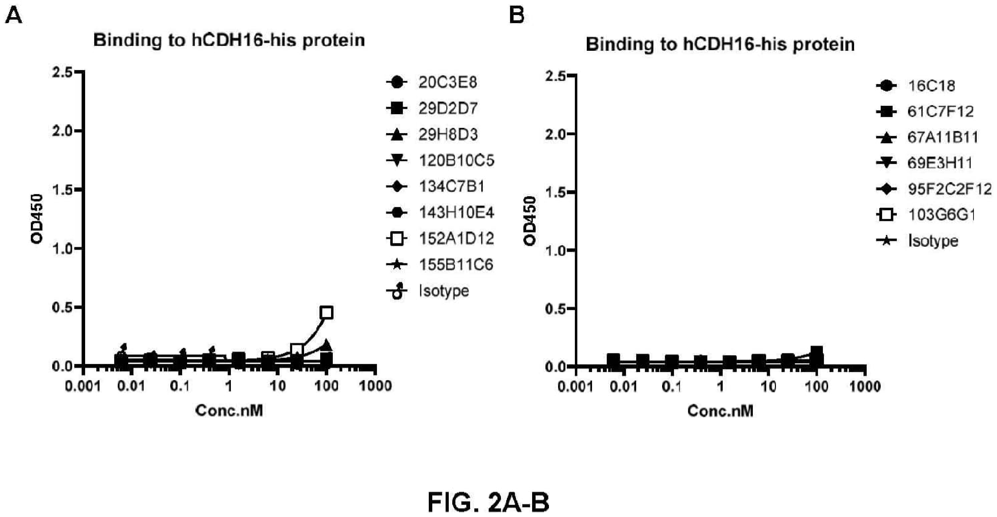

As shown in **FIG. 2,** all the CDH17 chimeric mAbs displayed neglectable binding to human CDH16 protein, indicating their specificity to human CDH17.

*2.3. Affinity measurement*

The binding affinity of the chimeric monoclonal antibodies to human CDH17 protein was determined with Biacore™ 8K. Briefly, the antibodies were captured with Pro-A chips at a concentration of 2 μg/mL. Single dose (*, 100 nM or 400 nM) or two doses (25 nM and 100 nM) of human CDH17-his protein were injected over captured antibody at a flow rate of 30 μL/min. The antigen was allowed to associate for 120 s -150 s and dissociate for 200 s-500 s. Data analysis was carried out using Biacore™ 8K evaluation software.

The results showed that all the chimeric CDH17 monoclonal antibodies exhibited high affinity to human CDH17, with the equilibrium dissociation constant (KD) ranging from $1\times10^{-7}$ to $1\times10^{-9}$ M (**Table 4**).

***Table 4. Affinity Ranking Results of CDH17 Chimeric Antibodies***

| Affinity / Antibody | Antigen: Human CDH17-his | | |
|---|---|---|---|
| | ka(1/Ms) | kd(1/s) | KD(M) |
| 16C18 | 2.12E+04 | 5.08E-04 | 2.39E-08 |
| 20C3E8* | 3.01E+04 | 3.85E-03 | 1.28E-07 |
| 29D2D7 * | 2.44E+05 | 5.93E-04 | 2.43E-09 |
| 29H8D3 | 8.80E+05 | 1.12E-03 | 1.28E-09 |
| 61C7F12* | 1.47E+05 | 3.86E-03 | 2.63E-08 |
| 67A11B11* | 2.62E+04 | 2.09E-04 | 8.01E-09 |
| 69E3H11 | 3.98E+04 | 2.36E-04 | 5.93E-09 |
| 95F2C2F12 | 1.06E+05 | 1.12E-03 | 1.05E-08 |
| 103G6G1 | 1.71E+05 | 8.03E-02 | 4.71E-07 |
| 120B10C5 | 1.03E+05 | 5.45E-04 | 5.30E-09 |
| 134C7B1 | 2.06E+05 | 2.86E-03 | 1.39E-08 |
| 143H10E4 | 4.42E+05 | 8.06E-04 | 1.82E-09 |
| 152A1D12 | 1.93E+05 | 9.46E-04 | 4.89E-09 |
| 155B11C6 | 3.14E+05 | 2.56E-03 | 8.17E-09 |

* Single dose affinity

*2.4. Epitope/domain mapping*

In order to evaluate the exact binding domain of the chimeric mAbs to human CDH17, cell-based and protein-based binding assays were employed as followed. Briefly, HEK293 cells stably expressing a series of ECDs of human CDH17 were constructed. The human CDH17 protein has a distinct extracellular structure consisting of seven cadherin domains (ECs). Consequently, cell lines expressing human CDH17 EC1 (Q23-Q128, HEK293-EC1), EC1 and EC2 (Q23-P244, HEK293-EC1-2), EC3 and EC4 (V245-F449, HEK293-EC3-4), EC5 and EC6 (E450-L667, HEK293-EC5-6), EC6 (S567-L667, HEK293-EC6) and EC7 (A668-M787, HEK293-EC7) conjugating with the transmembrane domain (TM) and intracellular domain (ICD) of human CDH17 were constructed (in-house made or customized by Genomeditech). In addition, the truncated human CDH17 EC3 (V245-C340) protein fused with mouse IgG1 Fc tag (EC3-mFc, customized by Novoprotein) was constructed in order to help determine the exact binding domain of certain EC3-4 binders. For cell binding assay, the indicated CDH17 chimeric mAbs or an isotype control were diluted at a concentration of 100 nM in the staining buffer (DPBS buffer containing 2% FBS). The antibody dilutions were incubated with $5\times10^4$ cells in 96-well microplates for 30 minutes (mins) at 4 °C. Then the cell-antibody mixture was washed with staining buffer twice. The antibodies binding to the antigen on the cell surface were detected with goat anti-human IgG (H+L) cross-adsorbed secondary antibody conjugated with Alexa Fluor™ 488 (ThermoFisher, Cat. No. A11013) at a dilution rate of 1:2000 for 30 mins at 4 °C followed by extensive washing. Cells were analyzed by flow cytometer LSRFortessa™ Cell Analyzer (BD Biosciences). Data were analyzed with Flowjo 10.0 software. The graphs were generated in Graphpad Prism 9 software. For ELISA binding assay, the experiment was performed as previously described in **Example 2.1**.

As summarized in **Table 5**, these CDH17 chimeric antibodies specifically bound to different ECD domains of human CDH17 expressed on HEK293 cells or truncated human CDH17 proteins. More specifically, 29H8D3, 143H10E4, 152A1D12 and 155B11C6 bound to the EC1 domain of human CDH17. 120B10C5 bound to the EC2 domain. 29D2D7 bound to the EC3 domain. 20C3E8 and 103G6G1 bound to the EC4 domain. 16C8 bound to the EC5 domain. 61C7F12 and 67A11B11 specifically bound to the EC6 domain of human CDH17. 69E3H11 and 95F2C2F12 bound to the membrane-proximal EC7 domain of human CDH17.

***Table 5. Domain Mapping of the CDH17 Chimeric Antibodies***

| Ab \ Cell line /protein | HEK293 -EC1 | HEK293- EC1-2 | HEK293 -EC3-4 | HEK293 -EC5-6 | HEK293 -EC6 | HEK29 3-EC7 | EC3- mFc | Domain |
|---|---|---|---|---|---|---|---|---|
| | MFI at 100nM of Ab concentration | | | | | | OD450 | / |
| 16C18 | 15.1 | 20.6 | 287 | 14836 | 332 | 334 | 0.054 | EC5 |
| 20C3E8 | 10.3 | 10.2 | 76032 | 256 | 294 | 310 | 0.047 | EC4 |
| 29D2D7 | 11.1 | 40.4 | 40180 | 244 | 289 | 299 | 0.874 | EC3 |
| 29H8D3 | 27929 | 52279 | 253 | 284 | 314 | 463 | 0.056 | EC1 |
| 61C7F12 | 13.1 | 16.4 | 256 | 70571 | 2283 | 348 | 0.076 | EC6 |
| 67A11B11 | 11.3 | 12.7 | 258 | 79424 | 2891 | 306 | 0.05 | EC6 |
| 69E3H11 | 19.7 | 34.6 | 281 | 288 | 308 | 13496 | 0.057 | EC7 |
| 95F2C2F12 | 11.5 | 13.3 | 258 | 249 | 309 | 13933 | 0.061 | EC7 |
| 103G6G1 | 9.95 | 10.9 | 87322 | 252 | 298 | 300 | 0.047 | EC4 |
| 120B10C5 | 10.7 | 11011 | 262 | 271 | 298 | 312 | 0.055 | EC2 |
| 143H10E4 | 25999 | 48900 | 216 | 233 | 383 | 300 | 0.053 | EC1 |
| 152A1D12 | 26233 | 43998 | 232 | 253 | 294 | 355 | 0.061 | EC1 |
| 155B11C6 | 22411 | 43331 | 229 | 245 | 289 | 293 | 0.043 | EC1 |
| Isotype | 10.1 | 10 | 276 | 273 | 315 | 327 | 0.05 | / |

***2.5 Binding ability to human CDH17-overexpressing HEK293 cells***

In order to evaluate the binding activity of the chimeric mAbs to human CDH17 expressed on cells, cell based binding assay was performed as followed. Briefly, HEK293 cell line stably expressing full length human CDH17 (referred as HEK293-hCDH17) was constructed (customized by Genomeditech). Parental HEK293 cells were used as the negative control. For cell binding assay, the indicated CDH17 chimeric mAbs or an isotype control were diluted at a fourfold dilution rate starting from a concentration of 100 nM in the staining buffer (DPBS buffer containing 2% FBS). The antibody dilutions were incubated with $5x10^4$ cells in 96-well microplates for 30 minutes (mins) at 4 °C. Then the cell-antibody mixture was washed with staining buffer twice. The antibodies binding to the antigen on the cell surface were detected with goat anti-human IgG (H+L) cross-adsorbed secondary antibody conjugated with Alexa Fluor™ 488 (ThermoFisher, Cat. No. A11013) at a dilution rate of 1:2000 for 30 mins at 4 °C followed by extensive washing. Cells were analyzed by flow cytometer LSRFortessa™ Cell Analyzer (BD Biosciences). Data were analyzed with Flowjo 10.0 software. The graphs and statistics analysis were generated with three-parameter or four-parameter nonlinear regression curve fit in Graphpad Prism 9 software.

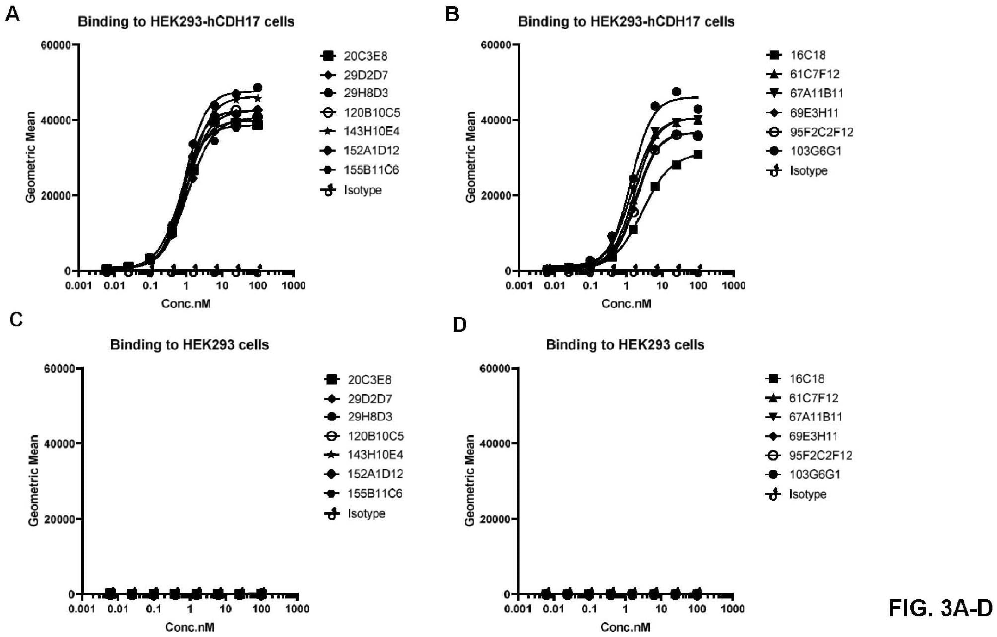

As shown in **FIG. 3A** and **3B**, all the CDH17 chimeric monoclonal antibodies efficiently bound to human CDH17 expressed on HEK293 cells in a dose-dependent manner. On the contrary, no specific binding was observed on the parental HEK293 cells (**FIG. 3C** and **3D**).

The EC50 values of the binding capability of CDH17 chimeric antibodies to human CDH17 expressed on HEK293 cells were summarized in **Table 6**.

***2.6 Binding ability to human CDH17-expressing tumor cells***

To evaluate the binding ability of the chimeric mAbs to human CDH17 expressed on tumor cells, several human tumor cell lines including pancreatic cancer cell line AsPC1, colorectal adenocarcinoma cell line HCT-8 and gastric adenocarcinoma cell line AGS cells, representing high, medium and low levels of CDH17 expression and different tumor types respectively, were employed in the cell-based binding assay following the protocol described in **Example 2.5**.

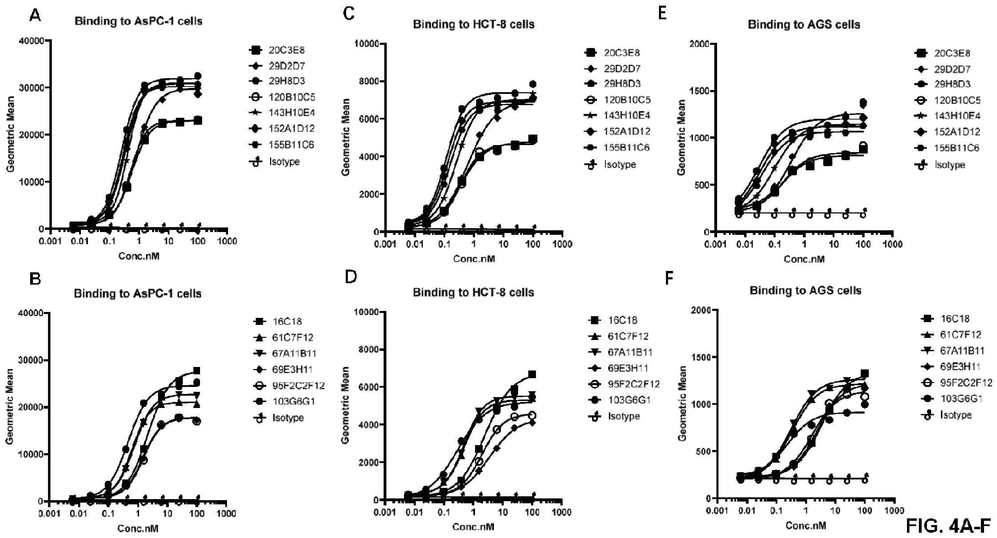

As shown in **FIG. 4A**-**4F**, all the CDH17 chimeric antibodies efficiently bound to human CDH17 expressed on tumor cells in a dose-dependent manner. More importantly, the binding abilities of individual CDH17 chimeric antibodies showed similar trends in these three tumor cell lines. Interestingly, antibodies binding to the membrane-distal regions of human CDH17 displayed better binding efficiency than those binding to membrane-proximal regions of human CDH17. These phenomena emphasize the superiority of binding potency of the CDH17 membrane-distal region binders than those of the membrane-proximal region ones.

The EC50 values of the binding capability of CDH17 chimeric antibodies to human CDH17 endogenously expressed on tumor cells were summarized in **Table 6**.

***2.7 Binding ability to cyno CDH17 overexpressing cells***

To evaluate species cross-reactivity of the chimeric mAbs to cynomolgus CDH17 expressed on cells, cell based

binding assay was employed following the protocol described **Example 2.5**. Briefly, HEK293 cell line stably expressing one splicing isoform of full length cynomolgus CDH17 (Isoform 2, Uniprot reference sequence: A0A2K5X8I8, M1-M840, referred as HEK293-cynoCDH17) was constructed (customized by Genomeditech). This isoform is different from cyno CDH17 isoform 1 (NCBI reference sequence: XP_005563762.1, M1-S832) as well as human CDH17 in their N-terminal regions. The indicated CDH17 chimeric Abs or an isotype control were tested in this assay.

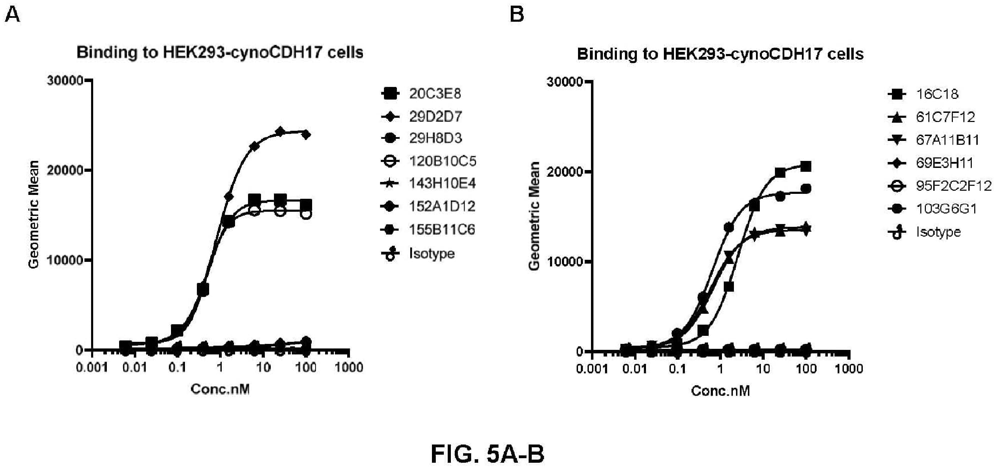

As shown in **FIG. 5A** and **5B**, CDH17 chimeric antibodies including EC2 to EC6 domain binders efficiently bound to cynomolgus CDH17 isoform 2 expressed on HEK293 cells. However, EC1 and EC7 binding mAbs barely bound to cynomolgus CDH17 isoform 2 expressed on cells.

The EC50 values of the binding capability of CDH17 chimeric antibodies to cynomolgus CDH17 expressed on HEK293 cells were summarized in **Table 6**.

***Table 6. Binding Properties of Chimeric Antibodies to hCDH17 and cynoCDH17 on Cells***

| Cell line / Antibody | HEK293-hCDH17 | AsPC1 | HCT-8 | AGS | HEK293-cynoCDH17 |
|---|---|---|---|---|---|
| | EC50(nM) | | | | |
| 16C181 | 2.802 | 2.151 | 2.515 | 3.463 | 2.545 |
| 20C3E8 | 0.906 | 0.563 | 0.341 | 0.177 | 0.513 |
| 29D2D7 | 1.114 | 0.797 | 0.609 | 0.367 | 0.821 |
| 29H8D3 | 0.899 | 0.259 | 0.117 | 0.029 | -- |
| 61C7F12 | 1.685 | 0.626 | 0.484 | 0.334 | 0.675 |
| 67A11B11 | 1.378 | 0.760 | 0.480 | 0.320 | 0.581 |
| 69E3H111 | 1.766 | 1.486 | 3.493 | 2.414 | -- |
| 95F2C2F12 | 1.879 | 1.552 | 1.955 | 1.639 | -- |
| 103G6G1 | 1.341 | 0.466 | 0.290 | 0.238 | 0.648 |
| 120B10C5 | 1.038 | 0.511 | 0.347 | 0.186 | 0.476 |
| 143H10E4 | 1.139 | 0.423 | 0.251 | 0.096 | -- |
| 152A1D12 | 0.864 | 0.292 | 0.129 | 0.031 | -- |
| 155B11C6 | 0.684 | 0.339 | 0.157 | 0.037 | -- |

Note: -- represents no specific binding

## Example 3. Humanization of CDH17 Chimeric Antibodies

### *3.1 Humanization design of CDH17 chimeric antibodies*

Based on the performance of the chimeric antibodies, several antibodies were selected for further humanization. The variable regions of CDH17 chimeric antibodies were selected to perform humanization. Briefly, the amino acid sequences of the VH and VL were aligned with the available database of human Ig gene sequences to identify the overall best-matching human germline Ig gene sequences. Then, the CDRs of heavy chain and light chain of CDH17 chimeric antibodies were grafted onto the candidate human germlines. A 3D model of the grafted antibodies was generated by Molecular Operating Environment (MOE) to determine if there was any critical human amino acid in the framework region essential to be back-mutated to the corresponding mouse amino acids to maintain the CDR conformation and function.

For the heavy chain of 29H8D3, the candidate human germline sequences were the *IGHV1-18*01* gene. For the light chain of 29H8D3, the candidate human germline sequences were the *IGKV2-28*01* gene. A24V, V37M, M48I, R67K, V68A, M70L, T72A, T74K, M81I, R84N, R87T, D89E and Y95F were subjected to back-mutations in the heavy chain framework of human germline sequence *IGHV1-18*01*. Y41F, L51V, D75A and Q105A were subjected to back-mutations in the light chain framework of human germline sequence *IGKV2-28*01*.

Different combinations of backmutation sites were selected to generate variable regions of humanized antibodies. The sequences of the variable regions of the heavy chain and light chain of the humanized antibodies for 29H8D3 were listed in **Table 7A**. The pairing of the VH and VL for individual humanized antibodies was listed in **Table 7B**. The variable regions of humanized antibodies were then fused to the constant region of human IgG1 for humanized antibody

production and functional characterization.

***Table 7A. Sequences of Variable Regions of 29H8D3 Humanized Antibodies (underlining indicates CDR; bold/ italic indicates back mutations)***

| **29H8D3** | | |
|---|---|---|
| | **Sequence** | **SEQ ID NO:** |
| 29H8D3 VH | QVQLQQSDAELVKPGASVKISCKVSGYTFTDHTIHWMKQRPEQGLEWIGYIYPRDGYTKYNEKFKDKATLTADKSSSTAYIQLNTLTSEDSAVYFCARFYYGYWYFDVWGTGTTVTVSS | 31 |
| 29H8D3 HU-VH1-1 | QVQLVQSGAEVKKPGASVKVSCKASGYTFTDHTIHWVRQAPGQGLEWMGYIYPRDGYTKYNEKFKDRVTMTTDTSTSTAYMELRSLRSDDTAVYYCARFYYGYWYFDVWGQGTTVTVSS | 113 |
| 29H8D3 HU-VH1-2 | QVQLVQSGAEVKKPGASVKVSCKASGYTFTDHTIHWVRQAPGQGLEW***I***GYIYPRDGYTKYNEKFKDR***A***TMTTDTSTSTAYMELRSLRSDDTAVYYCARFYYGYWYFDVWGQGTTVTVSS | 114 |
| 29H8D3 HU-VH1-3 | QVQLVQSGAEVKKPGASVKVSCKASGYTFTDHTIHW***M***RQAPGQGLEW***I***GYIYPRDGYTKYNEKFKDR***A***TMTTDTSTSTAYMELRSLRSDDTAVYYCARFYYGYWYFDVWGQGTTVTVSS | 115 |
| 29H8D3 HU-VH1-4 | QVQLVQSGAEVKKPGASVKVSCKASGYTFTDHTIHW***M***RQRPGQGLEW***I***GYIYPRDGYTKYNEKFKDR***A***T***L***T***A***DTSTSTAYMELRSLRSDDTAVYYCARFYYGYWYFDVWGQGTTVTVSS | 116 |
| 29H8D3 HU-VH1-5 | QVQLVQSGAEVKKPGASVKVSCKASGYTFTDHTIHW***M***RQRPGQGLEW***I***GYIYPRDGYTKYNEKFKD***KA***T***L***T***A***D***K***STSTAYMELRSLRSDDTAVYYCARFYYGYWYFDVWGQGTTVTVSS | 117 |
| 29H8D3 HU-VH1-6 | QVQLVQSGAEVKKPGASVKVSCK***V***SGYTFTDHTIHW***M***KQRPGQGLEW***I***GYIYPRDGYTKYNEKFKD***KA***T***L***T***A***D***K***STSTAY***I***EL***N***SLRSDDTAVY***F***CARFYYGYWYFDVWGQGTTVTVSS | 118 |
| 29H8D3 HU-VH2-1 | QVQLVQSGAEVKKPGASVKVSCKASGYTFTDHTIHWVRQAPGQGLEWMGYIYPRDGYTKYNEKFKDRVT***L***T***A***D***K***STSTAYMEL***N***SL***T***S***E***DTAVY***F***CARFYYGYWYFDVWGRGTLVTVSS | 119 |
| IGHV1-18*01 | QVQLVQSGAEVKKPGASVKVSCKASGYTFTSYGISWVRQAPGQGLEWMGWISAYNGNTNYAQKLQGRVTMTTDTSTSTAYMELRSLRSDDTAVYYCAR | 120 |
| 29H8D3 VL | DIVMTQAAPSVPVTPGESVSISCRSSKSLLRSNGNTYLYWFLQRPGQSPQVLIYRMSNLASGVPDRFSGSGSGTAFTLRISRVEAEDVGVYYCMQHLEYPFTFGAGTKLELK | 32 |
| 29H8D3 HU-VL1-1 | DIVMTQSPLSLPVTPGEPASISCRSSKSLLRSNGNTYLYWYLQKPGQSPQLLIYRMSNLASGVPDRFSGSGSGTDFTLKISRVEAEDVGVYYCMQHLEYPFTFGQGTKLEIK | 121 |
| 29H8D3 HU-VL1-2 | DIVMTQSPLSLPVTPGEPASISCRSSKSLLRSNGNTYLYWYLQKPGQSPQ***V***LIYRMSNLASGVPDRFSGSGSGTDFTLKISRVEAEDVGVYYCMQHLEYPFTFGQGTKLEIK | 122 |
| 29H8D3 HU-VL1-3 | DIVMTQSPLSLPVTPGEPASISCRSSKSLLRSNGNTYLYW***F***LQKPGQSPQ***V***LIYRMSNLASGVPDRFSGSGSGTDFTLKISRVEAEDVGVYYCMQHLEYPFTFGQGTKLEIK | 123 |

(continued)

| 29H8D3 | | |
|---|---|---|
| | Sequence | SEQ ID NO: |
| 29H8D3 HU-VL1-4 | DIVMTQSPLSLPVTPGEPASISCRSSKSLLRSNGNTYLYW***F***LQKPGQSPQ***V***LIYRMSNLASGVPDRFSGSGSGT***A***FTLKISRVEAEDVGVYYCMQHLEYPFTFGQGTKLEIK | 201 |
| 29H8D3 HU-VL1-5 | DIVMTQSPLSLPVTPGEPASISCRSSKSLLRSNGNTYLYW***F***LQKPGQSPQ***V***LIYRMSNLASGVPDRFSGSGSGT***A***FTLKISRVEAEDVGVYYCMQHLEYPFTFG***A***GTKLEIK | 202 |
| IGKV2-28*01 | DIVMTQSPLSLPVTPGEPASISCRSSQSLLHSNGYNYLDWYLQKPGQSPQLLIYLGSNRASGVPDRFSGSGSGTDFTLKISRVEAEDVGVYYCMQALQTP | 124 |

***Table 7B. Pairing of VH and VL for 29H8D3 Humanized Antibodies***

| 29H8D3 | HU-VL1-1 | HU-VL1-2 | HU-VL1-3 | HU-VL1-4 | HU-VL1-5 | VL |
|---|---|---|---|---|---|---|
| **HU-VH1-1** | z1 | z2 | z3 | z17 | z21 | |
| **HU-VH1-2** | z4 | z5 | z6 | z18 | z22 | |
| **HU-VH1-3** | z7 | z8 | z9 | z19 | z23 | |
| **HU-VH1-4** | z10 | z11 | z12 | z20 | | |
| **HU-VH1-5** | z13 | z14 | | | | |
| **HU-VH1-6** | z15 | | | | | |
| **HU-VH2-1** | | z16 | | | | |
| **VH** | | | | | | Chimeric |

For the heavy chain of 29D2D7, the candidate human germline sequences were the *IGHV1-18*01* gene. For the light chain of 29D2D7, the candidate human germline sequences were the *IGKV4-1*01* gene. R38K, M48I, R67K, V68A, M70L, T72A, T74K, R87T and D89E were subjected to back-mutations in the heavy chain framework of human germline sequence *IGHV1-18*01*. V3G, T5S and P49S were subjected to back-mutations in the light chain of human germline sequence *IGKV4-1*01*.

Different combinations of backmutation sites were selected to generate variable regions of humanized antibodies. The sequences of the variable regions of the heavy chain and light chain of the humanized antibodies for 29D2D7 were listed in **Table 8A**. The pairing of the VH and VL for individual humanized antibodies was listed in **Table 8B**. The variable regions of humanized antibodies were then fused to the constant region of human IgG1 for antibody production and functional characterization.

***Table 8A. Sequences of Variable Regions of 29D2D7 Humanized Antibodies (underlining indicates CDR; bold/ italic indicates back mutations)***

| 29D2D7 | | |
|---|---|---|
| | Sequence | SEQ ID NO: |
| 29D2D7 VH | QVQLQQSGAELVRPGASVTLSCKASGYTFTDYDMHWVKQTPVHGLEWIGGFDPESAGTVYNLRFKDKAILTADKSSSTAYMELRSLTSEDSAVYYCARKGYYSVSSPFTYWGQGTLVSVSA | 23 |
| 29D2D7 HU-VH1-1 | QVQLVQSGAEVKKPGASVKVSCKASGYTFTDYDMHWVRQAPGQGLEWMGGFDPESAGTVYNLRFKDRVTMTTDTSTSTAYMELRSLRSDDTAVYYCARKGYYSVSSPFTYWGQGTLVTVSS | 125 |

(continued)

| 29D2D7 | | |
|---|---|---|
| | **Sequence** | **SEQ ID NO:** |
| 29D2D7 HU-VH1-2 | QVQLVQSGAEVKKPGASVKVSCKASGYTFTDYDMHWVRQAPGQGLEW***I***G GFDPESAGTVYNLRFKDRVTMTTDTSTSTAYMELRSLRSDDTAVYYCAR KGYYSVSSPFTYWGQGTLVTVSS | 126 |
| 29D2D7 HU-VH1-3 | QVQLVQSGAEVKKPGASVKVSCKASGYTFTDYDMHWVRQAPGQGLEW***I***G GFDPESAGTVYNLRFKDR***A***TMTTDTSTSTAYMELRSLRSDDTAVYYCAR KGYYSVSSPFTYWGQGTLVTVSS | 127 |
| 29D2D7 HU-VH1-4 | QVQLVQSGAEVKKPGASVKVSCKASGYTFTDYDMHWV***K***QAPGQGLEW***I***G GFDPESAGTVYNLRFKDR***A***T***L***TTDTSTSTAYMELRSLRSDDTAVYYCAR KGYYSVSSPFTYWGQGTLVTVSS | 128 |
| 29D2D7 HU-VH1-5 | QVQLVQSGAEVKKPGASVKVSCKASGYTFTDYDMHWV***K***QAPGQGLEW***I***G GFDPESAGTVYNLRFKD***KA***T***L***TTDTSTSTAYMELRSLRSDDTAVYYCAR KGYYSVSSPFTYWGQGTLVTVSS | 129 |
| 29D2D7 HU-VH1-6 | QVQLVQSGAEVKKPGASVKVSCKASGYTFTDYDMHWV***K***QAPGQGLEW***I***G GFDPESAGTVYNLRFKD***KA***T***L***TTD***K***STSTAYMELRSLRSDDTAVYYCAR KGYYSVSSPFTYWGQGTLVTVSS | 130 |
| 29D2D7 HU-VH1-7 | QVQLVQSGAEVKKPGASVKVSCKASGYTFTDYDMHWV***K***QAPGQGLEW***I***G GFDPESAGTVYNLRFKD***KA***T***L***T***A***D***K***STSTAYMELRSLRS***E***DTAVYYCAR KGYYSVSSPFTYWGQGTLVTVSS | 131 |
| 29D2D7 HU-VH2-1 | QVQLVQSGAEVKKPGASVKVSCKASGYTFTDYDMHWVRQAPGKGLEWMG GFDPESAGTVYNLRFKDRVT***L***T***A***D***K***STSTAYMELRSL***T***S***E***DTAVYYCAR KGYYSVSSPFTYWGQGTLVTVSS | 132 |
| IGHV1-18*01 | QVQLVQSGAEVKKPGASVKVSCKASGYTFTSYGISWVRQAPGQGLEWMG WISAYNGNTNYAQKLQGRVTMTTDTSTSTAYMELRSLRSDDTAVYYCAR | 133 |
| 29D2D7 VL | DIGMSQSPSSLAVSVGEKVTMSCKSSQSLLYSSNQKNYLAWYQQKPGQS PKLLIYWASTRESGVPDRFTGSGSGTDFTLTINSVKAEDLAVYYCQQCY SYPRTFGGGTKLEIK | 24 |
| 29D2D7 HU-VL1-1 | DIVMTQSPDSLAVSLGERATINCKSSQSLLYSSNQKNYLAWYQQKPGQP PKLLIYWASTRESGVPDRFSGSGSGTDFTLTISSLQAEDVAVYYCQQCY SYPRTFGGGTKVEIK | 134 |
| 29D2D7 HU-VL1-2 | DIVMTQSPDSLAVSLGERATINCKSSQSLLYSSNQKNYLAWYQQKPGQ***S*** PKLLIYWASTRESGVPDRFSGSGSGTDFTLTISSLQAEDVAVYYCQQCY SYPRTFGGGTKVEIK | 135 |
| 29D2D7 HU-VL1-3 | DI***G***MTQSPDSLAVSLGERATINCKSSQSLLYSSNQKNYLAWYQQKPGQ***S*** PKLLIYWASTRESGVPDRFSGSGSGTDFTLTISSLQAEDVAVYYCQQCY SYPRTFGGGTKVEIK | 136 |
| 29D2D7 HU-VL1-4 | DI***G***M***S***QSPDSLAVSLGERATINCKSSQSLLYSSNQKNYLAWYQQKPGQ***S*** PKLLIYWASTRESGVPDRFSGSGSGTDFTLTISSLQAEDVAVYYCQQCY SYPRTFGGGTKVEIK | 137 |

(continued)

| 29D2D7 | | |
|---|---|---|
| | Sequence | SEQ ID NO: |
| IGKV4-1*01 | DIVMTQSPDSLAVSLGERATINCKSSQSVLYSSNNKNYLAWYQQKPGQP PKLLIYWASTRESGVPDRFSGSGSGTDFTLTISSLQAEDVAVYYCQQYY STP | 138 |

*Table 8B. Pairing of VH and VL for 29D2D7 Humanized Antibodies*

| 29D2D7 | HU-VL1-1 | HU-VL1-2 | HU-VL1-3 | HU-VL1-4 | VL |
|---|---|---|---|---|---|
| **HU-VH1-1** | z1 | z2 | z3 | z20 | |
| **HU-VH1-2** | z4 | z5 | z6 | z21 | |
| **HU-VH1-3** | z7 | z8 | z9 | z22 | |
| **HU-VH1-4** | z10 | z11 | z12 | z23 | |
| **HU-VH1-5** | z13 | z14 | z15 | z24 | |
| **HU-VH1-6** | z16 | z17 | z18 | | |
| **HU-VH1-7** | z19 | | | | |
| **HU-VH2-1** | | | | z25 | |
| **VH** | | | | | Chimeric |

For the heavy chain of 61C7F12, the candidate human germline sequences were the *IGHV3-21*01* gene. For the light chain of 61C7F12, the candidate human germline sequences were the *IGKV1-9*01 or IGKV3-20*01* gene. S49A was subjected to back-mutation in the heavy chain framework of human germline sequence *IGHV3-21*01.* D1Q, Q3V, A44S, L48W, E71S, F72Y and T73S were subjected to back-mutations in the light chain framework of human germline sequence *IGKV1-9*01.* There were no back-mutations involved in the light chain framework of human germline sequence *IGKV3-20*01.*

Different combinations of backmutation sites were selected to generate variable regions of humanized antibodies. The sequences of the variable regions of the heavy chain and light chain of the humanized antibodies for 61C7F12 were listed in Table 9A. The pairing of the VH and VL for individual humanized antibodies was listed in **Table 9B.** The variable regions of humanized antibodies were then fused to the constant region of human IgG1 for antibody production and functional characterization.

***Table 9A. Sequences of Variable Region of 61C7F12 Humanized Antibodies (underlining indicates CDR; bold/ italic indicates back mutations)***

| 61C7F12 | | |
|---|---|---|
| | Sequence | SEQ ID NO: |
| 61C7F12 VH | EVQLVESGGGLVKPGGSLKLSCAASGFTFSSYAMSWVRQTPEKRLEWVA TINIGGSYTYYPDSVKGRFTISRDNAKNTLYLQMSSLRSEDTAMYYCAR QEGRYWYFDVWGAGTTVTVSS | 39 |
| 61C7F12 HU-VH1-0 | EVQLVESGGGLVKPGGSLRLSCAASGFTFSSYAMSWVRQAPGKGLEWVS TINIGGSYTYYPDSVKGRFTISRDNAKNSLYLQMNSLRAEDTAVYYCAR QEGRYWYFDVWGQGTTVTVSS | 139 |
| 61C7F12 HU-VH1-1 | EVQLVESGGGLVKPGGSLRLSCAASGFTFSSYAMSWVRQAPGKGLEWV***A*** TINIGGSYTYYPDSVKGRFTISRDNAKNSLYLQMNSLRAEDTAVYYCAR QEGRYWYFDVWGQGTTVTVSS | 140 |
| IGHV3-21*01 | EVQLVESGGGLVKPGGSLRLSCAASGFTFSSYSMNWVRQAPGKGLEWVS SISSSSSYIYYADSVKGRFTISRDNAKNSLYLQMNSLRAEDTAVYYCAR | 141 |

(continued)

| 61C7F12 | | |
|---|---|---|
| | **Sequence** | **SEQ ID NO:** |
| 61C7F12 VL | QIVLTQSPAIMSASLGERVTMTCTASSSVSSSYLHWYQQKPGSSPKLWIYSTSNLASGVPARFSGSGSGTSYSLTISSMEAEDAATYYCHQFHRSPYTFGGGTKLEIK | 40 |
| 61C7F12 HU-VL1-0 | DIQLTQSPSFLSASVGDRVTITCTASSSVSSSYLHWYQQKPGKAPKLLIYSTSNLASGVPSRFSGSGSGTEFTLTISSLQPEDFATYYCHQFHRSPYTFGGGTKVEIK | 142 |
| 61C7F12 HU-VL1-1 | DIQLTQSPSFLSASVGDRVTITCTASSSVSSSYLHWYQQKPGKAPKL***W***IYSTSNLASGVPSRFSGSGSGTE***Y***TLTISSLQPEDFATYYCHQFHRSPYTFGGGTKVEIK | 143 |
| 61C7F12 HU-VL1-2 | DI***V***LTQSPSFLSASVGDRVTITCTASSSVSSSYLHWYQQKPGK***S***PKL***W***IYSTSNLASGVPSRFSGSGSGTE***YS***LTISSLQPEDFATYYCHQFHRSPYTFGGGTKVEIK | 144 |
| 61C7F12 HU-VL1-3 | ***Q***I***V***LTQSPSFLSASVGDRVTITCTASSSVSSSYLHWYQQKPGK***S***PKL***W***IYSTSNLASGVPSRFSGSGSGT***SYS***LTISSLQPEDFATYYCHQFHRSPYTFGGGTKVEIK | 145 |
| IGKV1-9*01 | DIQLTQSPSFLSASVGDRVTITCRASSQGISS*YLAWYQQKPGKAPKLLIYAASTLQSGVPSRFSGSGSGTEFTLTISSLQPEDFATYYCQQLNSYP | 146 |
| 61C7F12 HU-VL2-0 | EIVLTQSPGTLSLSPGERATLSCTASSSVSSSYLHWYQQKPGQAPRLLIYSTSNLASGIPDRFSGSGSGTDFTLTISRLEPEDFAVYYCHQFHRSPYTFGGGTKVEIK | 147 |
| IGKV3-20*01 | EIVLTQSPGTLSLSPGERATLSCRASQSVSSSYLAWYQQKPGQAPRLLIYGASSRATGIPDRFSGSGSGTDFTLTISRLEPEDFAVYYCQQYGSYT | 148 |

***Table 9B. Pairing of VH and VL for 61C7F12 Humanized Antibodies***

| 61C7F12 | HU-VL1-0 | HU-VL1-1 | HU-VL1-2 | HU-VL1-3 | HU-VL2-0 | VL |
|---|---|---|---|---|---|---|
| **HU-VH1-0** | z1 | z2 | z3 | z4 | z9 | |
| **HU-VH1-1** | z5 | z6 | z7 | z8 | z10 | |
| **VH** | | | | | | Chimeric |

For the heavy chain of 69E3H11, the candidate human germline sequences were the *IGHV1-3*01 or IGHV1-46*01* gene. For the light chain of 69E3H11, the candidate human germline sequences were the *IGKV6-21*01 or IGKV3-20*01* gene. R38K, M48I, R67K, V68A, I70L, R72V, T74K and R98L were subjected to back-mutations in the heavy chain framework of human germline sequence *IGHVI-3 *01.* M70L, V79A and R87T were subjected to back-mutations in the heavy chain framework of human germline sequence *IGHV1-46*01.* K50Y, and F72Y were subjected to back-mutations in the light chain framework of human germline sequence *IGKV6-21*01.* There were no back-mutations involved in the light chain framework of human germline sequence *IGKV3-20*01.*

Different combinations of backmutation sites were selected to generate variable regions of humanized antibodies. The sequences of the variable regions of the heavy chain and light chain of the humanized antibodies for 69E3H11 were listed in **Table 10A.** The pairing of the VH and VL for individual humanized antibodies was listed in **Table 10B.** The variable regions of humanized antibodies were then fused to the constant region of human IgG1 for antibody production and functional characterization.

***Table 10A. Sequences of Variable Region of 69E3H11 Humanized Antibodies (underlining indicates CDR; bold/italic indicates back mutations)***

| **69E3H11** | | |
|---|---|---|
| | **Sequence** | **SEQ ID NO:** |
| 69E3H11-VH | QVQLQQPGAELVKPGASVKLSCKASGYTFTTYWMHWVKQRPGQGLEWIGEIDPSDSYANYNQKFKGKATLTVDKSSSTAYMQLSSLTSEDSAVYYCALYYGYGDWGQGTTLTVSS | 55 |
| 69E3H11 HU-VH1-0 | QVQLVQSGAEVKKPGASVKVSCKASGYTFTTYWMHWVRQAPGQRLEWMGEIDPSDSYANYNQKFKGRVTITRDTSASTAYMELSSLRSEDTAVYYCARYYGYGDWGQGTTVTVSS | 149 |
| 69E3H11 HU-VH1-1 | QVQLVQSGAEVKKPGASVKVSCKASGYTFTTYWMHWVRQAPGQRLEW***I***GEIDPSDSYANYNQKFKGR***A***TITRDTSASTAYMELSSLRSEDTAVYYCA***L***YYGYGDWGQGTTVTVSS | 150 |
| 69E3H11 HU-VH1-2 | QVQLVQSGAEVKKPGASVKVSCKASGYTFTTYWMHWVRQAPGQRLEW***I***GEIDPSDSYANYNQKFKG***KA***T***L***T***V***DTSASTAYMELSSLRSEDTAVYYCA***L***YYGYGDWGQGTTVTVSS | 151 |
| 69E3H11 HU-VH1-3 | QVQLVQSGAEVKKPGASVKVSCKASGYTFTTYWMHWV***K***QAPGQRLEW***I***GEIDPSDSYANYNQKFKG***KA***T***L***T***V***D***K***SASTAYMELSSLRSEDTAVYYCA***L***YYGYGDWGQGTTVTVSS | 152 |
| IGHV1-3*01 | QVQLVQSGAEVKKPGASVKVSCKASGYTFTSYAMHWVRQAPGQRLEWMGWINAGNGNTKYSQKFQGRVTITRDTSASTAYMELSSLRSEDTAVYYCAR | 153 |
| 69E3H11 HU-VH2-1 | QVQLVQSGAEVKKPGASVKVSCKASGYTFTTYWMHWVRQAPGQGLEWMGEIDPSDSYANYNQKFKGRVT***L***TRDTSTST***A***YMELSSL***T***SEDTAVYYCARYYGYGDWGQGTTVTVSS | 154 |
| IGHV1-46*01 | QVQLVQSGAEVKKPGASVKVSCKASGYTFTSYYMHWVRQAPGQGLEWMGIINPSGGSTSYAQKFQGRVTMTRDTSTSTVYMELSSLRTSEDTAVYYCAR | 155 |
| 69E3H11 VL | EIVLTQSPTTMAASPGEKIIITCSVSSSISSIHLHWYQQKPGFSPKLLIYRTSNLASGVPTRFSGSGSGTSYSLTIGTMEAEDVATYYCQQGSNLPLTFGAGTRLELK | 56 |
| 69E3H11 HU-VL1-0 | EIVLTQSPDFQSVTPKEKVTITCSVSSSISSIHLHWYQQKPDQSPKLLIKRTSNLASGVPSRFSGSGSGTDFTLTINSLEAEDAATYYCQQGSNLPLTFGQGTRLEIK | 156 |
| 69E3H11 HU-VL1-1 | EIVLTQSPDFQSVTPKEKVTITCSVSSSISSIHLHWYQQKPDQSPKLLI***Y***RTSNLASGVPSRFSGSGSGTDFTLTINSLEAEDAATYYCQQGSNLPLTFGQGTRLEIK | 157 |
| 69E3H11 HU-VL1-2 | EIVLTQSPDFQSVTPKEKVTITCSVSSSISSIHLHWYQQKPDQSPKLLI***Y***RTSNLASGVPSRFSGSGSGTD***Y***TLTINSLEAEDAATYYCQQGSNLPLTFGQGTRLEIK | 158 |
| IGKV6-21*01 | EIVLTQSPDFQSVTPKEKVTITCRASQSIGS*SLHWYQQKPDQSPKLLIKYASQSFSGVPSRFSGSGSGTDFTLTINSLEAEDAATYYCHQSSSLP | 159 |
| 69E3H11 HU-VL2-0 | EIVLTQSPGTLSLSPGERATLSCSVSSSISSIHLHWYQQKPGQAPRLLIYRTSNLASGIPDRFSGSGSGTDFTLTISRLEPEDFAVYYCQQGSNLPLTFGQGTRLEIK | 160 |

(continued)

| 69E3H11 | | |
|---|---|---|
| | **Sequence** | **SEQ ID NO:** |
| IGKV3-20*01 | EIVLTQSPGTLSLSPGERATLSCRASQSVSSSYLAWYQQKPGQAPRLLI<br>YGASSRATGIPDRFSGSGSGTDFTLTISRLEPEDFAVYYCQQYGSSP | 161 |

***Table 10B. Pairing of VH and VL for 69E3H11 Humanized Antibodies***

| 69E3H11 | HU-VL1-0 | HU-VL1-1 | HU-VL1-2 | HU-VL2-0 | VL |
|---|---|---|---|---|---|
| **HU-VH1-0** | z1 | z2 | z3 | z11 | |
| **HU-VH1-1** | z4 | z5 | z6 | | |
| **HU-VH1-2** | z7 | z8 | z9 | | |
| **HU-VH1-3** | z10 | | | z12 | |
| **HU-VH2-1** | z13 | | | z14 | |
| **VH** | | | | | Chimeric |

For the heavy chain of 143H10E4, the candidate human germline sequences were the *IGHV1-69*02* gene. For the light chain of 143H10E4, the candidate human germline sequences were the *IGKV6-21 *01 or IGKV3-20*01* gene. G27S, S30T, M48I, R67K, V68A, I70L, S84N and Y95F were subjected to back-mutations in the heavy chain framework of human germline sequence *IGHV1-69*02.* L47P, L48W, K50Y, F72Y and T73S were subjected to back-mutations in the light chain framework of human germline sequence *IGKV6-21 *01.* There were no back-mutations involved in the light chain framework of human germline sequence *IGKV3-20*01.*

Different combinations of backmutation sites were selected to generate variable regions of humanized antibodies. The sequences of the variable regions of the heavy chain and light chain of the humanized antibodies for 143H10E4 were listed in **Table 11A.** The pairing of the VH and VL for individual humanized antibodies was listed in **Table 11B.** The variable regions of humanized antibodies were then fused to the constant region of human IgG1 for antibody production and functional characterization.

***Table 11A. Sequences of Variable Regions of 143H10E4 Humanized Antibodies (underlining indicates CDR; bold/italic indicates back mutations)***

| 143H10E4 | | |
|---|---|---|
| | **Sequence** | **SEQ ID NO:** |
| 143H10E4 VH | QVQLQQSDAELVKPGASVKISCKASGSTFTDHTIHWVKQRPEQGLEWIG<br>YIYPVYGYTQNNEKFKGKATLTADKSSSTAYMQLNSLTSEDSAVYFCAR<br>SRDSNPYYFDYWGQGTTLTVSS | 95 |
| 143H10E4 HU-VH1-0 | QVQLVQSGAEVKKPGSSVKVSCKASG***S***TF***T***DHTIHWVRQAPGQGLEWMG<br>YIYPVYGYTQNNEKFKGRVTITADKSTSTAYMELSSLRSEDTAVYYCAR<br>SRDSNPYYFDYWGQGTTVTVSS | 162 |
| 143H10E4 HU-VH1-1 | QVQLVQSGAEVKKPGSSVKVSCKASG***S***TF***T***DHTIHWVRQAPGQGLEW***I***G<br>YIYPVYGYTQNNEKFKGR***A***TITADKSTSTAYMELSSLRSEDTAVYYCAR<br>SRDSNPYYFDYWGQGTTVTVSS | 163 |
| 143H10E4 HU-VH1-2 | QVQLVQSGAEVKKPGSSVKVSCKASG***S***TF***T***DHTIHWVRQAPGQGLEW***I***G<br>YIYPVYGYTQNNEKFKGR***A***TITADKSTSTAYMEL***N***SLRSEDTAVYYCAR<br>SRDSNPYYFDYWGQGTTVTVSS | 164 |
| 143H10E4 HU-VH1-3 | QVQLVQSGAEVKKPGSSVKVSCKASG***S***TF***T***DHTIHWVKQAPGQGLEW***I***G<br>YIYPVYGYTQNNEKFKG***KA***T***L***TADKSTSTAYMEL***N***SLRSEDTAVY***F***CAR<br>SRDSNPYYFDYWGQGTTVTVSS | 165 |

(continued)

| 143H10E4 | | |
|---|---|---|
| | **Sequence** | **SEQ ID NO:** |
| IGHV1-69*02 | QVQLVQSGAEVKKPGSSVKVSCKASGGTFSSYTISWVRQAPGQGLEWMG RIIPILGIANYAQKFQGRVTITADKSTSTAYMELSSLRSEDTAVYYCAR | 166 |
| 143H10E4 VL | EIVLTQSPALMAASPGEKVTITCTVSSSISSSNLHWYQQKSGTSPKPWI YGTSNLASGVPVRFSGSGSGTSYSLTISSMEAEDAATYYCQQWSSYPFT FGSGTKLKMK | 96 |
| 143H10E4 HU-VL1-1 | EIVLTQSPDFQSVTPKEKVTITCTVSSSISSSNLHWYQQKPDQSPKLLI ***Y***GTSNLASGVPSRFSGSGSGTDFTLTINSLEAEDAATYYCQQWSSYPFT FGQGTKLEIK | 167 |
| 143H10E4 HU-VL1-2 | EIVLTQSPDFQSVTPKEKVTITCTVSSSISSSNLHWYQQKPDQSPKL***W***I ***Y***GTSNLASGVPSRFSGSGSGTD***Y***TLTINSLEAEDAATYYCQQWSSYPFT FGQGTKLEIK | 168 |
| 143H10E4 HU-VL1-3 | EIVLTQSPDFQSVTPKEKVTITCTVSSSISSSNLHWYQQKPDQSPK***PW***I ***Y***GTSNLASGVPSRFSGSGSGTD***YS***LTINSLEAEDAATYYCQQWSSYPFT FGQGTKLEIK | 169 |
| IGKV6-21*01 | EIVLTQSPDFQSVTPKEKVTITCRASQSIGSS*LHWYQQKPDQSPKLLI KYASQSFSGVPSRFSGSGSGTDFTLTINSLEAEDAATYYCHQSSSLP | 170 |
| 143H10E4 HU-VL2-0 | EIVLTQSPATLSLSPGERATLSCTVSSSISSSNLHWYQQKPGLAPRLLI YGTSNLASGIPDRFSGSGSGTDFTLTISRLEPEDFAVYYCQQWSSYPFT FGQGTKLEIK | 171 |
| IGKV3D-20*01 | EIVLTQSPATLSLSPGERATLSCGASQSVSSSYLAWYQQKPGLAPRLLI YDASSRATGIPDRFSGSGSGTDFTLTISRLEPEDFAVYYCQQYGSSP | 172 |

***Table 11B. Pairing of VH and VL for 143H10E4 Humanized Antibodies***

| 143H10E4 | HU-VL1-1 | HU-VL1-2 | HU-VL1-3 | HU-VL2-0 | VL |
|---|---|---|---|---|---|
| **HU-VH1-0** | z1 | z2 | z3 | z11 | |
| **HU-VH1-1** | z4 | z5 | z6 | | |
| **HU-VH1-2** | z7 | z8 | z9 | | |
| **HU-VH1-3** | z10 | | | z12 | |
| **VH** | | | | | Chimeric |

For the heavy chain of 152A1D12, the candidate human germline sequences were the *IGHV1-3*01* gene. For the light chain of 152A1D12, the candidate human germline sequences were the *IGKV1-13*02* or *IGKV3-11*01* gene. R38K, M48I, R67K, V68A, I70L and R72V were subjected to back-mutations in the heavy chain framework of human germline sequence *IGHV1-3*01.* L46R, L47W and F71Y were subjected to back-mutations in the hybrid light chain framework of the human germline sequence generated from a combination of human germline sequences *IGKV1-13*02* and *IGKV3-11*01.* L47W and I58V were subjected to back-mutations in the light chain framework of human germline sequence *IGKV3-11 *01.*

Different combinations of backmutation sites were selected to generate variable regions of humanized antibodies. The sequences of the variable regions of the heavy chain and light chain of the humanized antibodies for 152A1D12 were listed in **Table 12A.** The pairing of the VH and VL for individual humanized antibodies was listed in **Table 12B.** The variable regions of humanized antibodies were then fused to the constant region of human IgG1 for antibody production and functional characterization.

***Table 12A. Sequences of Variable Regions of 152A1D12 Humanized Antibodies (underlining indicates CDR; bold/italic indicates back mutations)***

| **152A1D12** | | |
|---|---|---|
| | **Sequence** | **SEQ ID NO:** |
| 152A1D12 VH | EVQLQQSGPELVKPGASVKISCKASGYTFTDYNMHWVKQSHGKSLEWIGYIYRNNGYNGYNQKFKNKATLTVDSSSSTAYMELRSLTSEDSAVYYCARNYWRGAMDYWGQGTSVTVSS | 103 |
| 152A1D12 HU-VH1-0 | QVQLVQSGAEVKKPGASVKVSCKASGYTFTDYNMHWVRQAPGQRLEWMGYIYRNNGYNGYNQKFKNRVTITRDTSASTAYMELSSLRSEDTAVYYCARNYWRGAMDYWGQGTTVTVSS | 173 |
| 152A1D12 HU-VH1-1 | QVQLVQSGAEVKKPGASVKVSCKASGYTFTDYNMHWVRQAPGQRLEW***I***GYIYRNNGYNGYNQKFKNR***A***TITRDTSASTAYMELSSLRSEDTAVYYCARNYWRGAMDYWGQGTTVTVSS | 174 |
| 152A1D12 HU-VH1-2 | QVQLVQSGAEVKKPGASVKVSCKASGYTFTDYNMHWV***K***QAPGQRLEW***I***GYIYRNNGYNGYNQKFKNR***A***T***L***TRDTSASTAYMELSSLRSEDTAVYYCARNYWRGAMDYWGQGTTVTVSS | 175 |
| 152A1D12 HU-VH1-3 | QVQLVQSGAEVKKPGASVKVSCKASGYTFTDYNMHWV***K***QAPGQRLEWIGYIYRNNGYNGYNQKFKNKATLTVDTSASTAYMELSSLRSEDTAVYYCARNYWRGAMDYWGQGTTVTVSS | 176 |
| IGHV1-3*01 | QVQLVQSGAEVKKPGASVKVSCKASGYTFTSYAMHWVRQAPGQRLEWMGWINAGNGNTKYSQKFQGRVTITRDTSASTAYMELSSLRSEDTAVYYCAR | 177 |
| 152A1D12 VL | QIVLTQSPAIMSASPGEKVTMTCSASSSISYMHWYQQKPGTSPKRWIYDTSKLASGVPARFSGSGSGTSYSLTISSMEAEDAGTYYCHQRSSYPWTFGGGTKLEIK | 104 |
| 152A1D12 HU-VL1-0 | EIVLTQSPATLSLSPGERATLSCSASSSISYMHWYQQKPGKAPKLLIYDTSKLASGVPSRFSGSGSGTDFTLTISSLQPEDFATYYCHQRSSYPWTFGGGTKVEIK | 178 |
| 152A1D12 HU-VL1-1 | EIVLTQSPATLSLSPGERATLSCSASSSISYMHWYQQKPGKAPK***R***LIYDTSKLASGVPSRFSGSGSGTDFTLTISSLQPEDFATYYCHQRSSYPWTFGGGTKVEIK | 179 |
| 152A1D12 HU-VL1-2 | EIVLTQSPATLSLSPGERATLSCSASSSISYMHWYQQKPGKAPK***RW***IYDTSKLASGVPSRFSGSGSGTDFTLTISSLQPEDFATYYCHQRSSYPWTFGGGTKVEIK | 180 |
| 152A1D12 HU-VL1-3 | EIVLTQSPATLSLSPGERATLSCSASSSISYMHWYQQKPGKAPK***RW***IYDTSKLASGVPSRFSGSGSGTD***Y***TLTISSLQPEDFATYYCHQRSSYPWTFGGGTKVEIK | 181 |
| IGKV1-13*02 | AIQLTQSPSSLSASVGDRVTITCRASQGISSALAWYQQKPGKAPKLLIYDASSLESGVPSRFSGSGSGTDFTLTISSLQPEDFATYYCQQFNSYP | 182 |
| 152A1D12 HU-VL2-0 | EIVLTQSPATLSLSPGERATLSCSASSSISYMHWYQQKPGQAPRLLIYDTSKLASGIPARFSGSGSGTDFTLTISSLEPEDFAVYYCHQRSSYPWTFGGGTKVEIK | 183 |
| 152A1D12 HU-VL2-1 | EIVLTQSPATLSLSPGERATLSCSASSSISYMHWYQQKPGQAPRL***W***IYDTSKLASG***V***PARFSGSGSGTDFTLTISSLEPEDFAVYYCHQRSSYPWTFGGGTKVEIK | 184 |

(continued)

| 152A1D12 | | |
|---|---|---|
| | **Sequence** | **SEQ ID NO:** |
| IGKV3-11*01 | EIVLTQSPATLSLSPGERATLSCRASQSVSSYLAWYQQKPGQAPRLLIY<br>DASNRATGIPARFSGSGSGTDFTLTISSLEPEDFAVYYCQQRSNWP | 185 |

*Table 12B. Pairing of VH and VL for 152A1D12 Humanized Antibodies*

| 152A1D12 | HU-VL1-0 | HU-VL1-1 | HU-VL1-2 | HU-VL1-3 | HU-VL2-0 | HU-VL2-1 | VL |
|---|---|---|---|---|---|---|---|
| **HU-VH1-0** | z1 | z2 | z3 | z4 | z16 | | |
| **HU-VH1-1** | z5 | z6 | z7 | z8 | | | |
| **HU-VH1-2** | z9 | z10 | z11 | z12 | | | |
| **HU-VH1-3** | z13 | z14 | z15 | | z17 | z18 | |
| **VH** | | | | | | | Chimeric |

For the heavy chain of 155B11C6, the candidate human germline sequences were the *IGHV1-2*02 or IGHV1-46*01* gene. For the light chain of 155B11C6, the candidate human germline sequences were the *IGKV3-20*01 or IGKV6-21 *01* gene. R38K, M48I, R67K, V68A, M70L, R72A and T74K were subjected to back-mutations in the heavy chain framework of human germline sequence *IGHV1-2*02.* M70L, R72A, T74K, S85G and R87T were subjected to back-mutations in the heavy chain framework of human germline sequence *IGHV1-46*01.* A44S, L47P, L48W, I59V, D71S and F72Y were subjected to back-mutations in the light chain framework of human germline sequence *IGKV3-20*01.* L47P, L48W, K50Y and F72Y were subjected to back-mutations in the light chain framework of human germline sequence *IGKV6-21 *01.*

Different combinations of backmutation sites were selected to generate variable regions of humanized antibodies. The sequences of the variable regions of the heavy chain and light chain of the humanized antibodies for 155B11C6 were listed in **Table 13A.** The pairing of the VH and VL for individual humanized antibodies was listed in **Table 13B.** The variable regions of humanized antibodies were then fused to the constant region of human IgG1 for antibody production and functional characterization.

*Table 13A. Sequences of Variable Regions of 155B11C6 Humanized Antibodies (underlining indicates CDR; bold/italic indicates back mutations)*

| 155B11C6 | | |
|---|---|---|
| | **Sequence** | **SEQ ID NO:** |
| 155B11C6 VH | QVQLQQSGAELARPGASVKMSCKASGYTFTSYTMHWVKQRPGQGLEWIG<br>YIDPKSDYNYYNQKFKDKATLTADKSSSTAYMQLSGLTSEDSTVYYCAR<br>GFFKGYFDVWGAGTTVTVSS | 111 |
| 155B11C6 HU-VH1-0 | QVQLVQSGAEVKKPGASVKVSCKASGYTFTSYTMHWVRQAPGQGLEWMG<br>YIDPKSDYNYYNQKFKDRVTMTRDTSISTAYMELSRLRSDDTAVYYCAR<br>GFFKGYFDVWGQGTTVTVSS | 186 |
| 155B11C6 HU-VH1-2 | QVQLVQSGAEVKKPGASVKVSCKASGYTFTSYTMHWVRQAPGQGLEW***I***G<br>YIDPKSDYNYYNQKFKDR***A***T***L***TRDTSISTAYMELSRLRSDDTAVYYCAR<br>GFFKGYFDVWGQGTTVTVSS | 187 |
| 155B11C6 HU-VH1-4 | QVQLVQSGAEVKKPGASVKVSCKASGYTFTSYTMHWV***K***QAPGQGLEW***I***G<br>YIDPKSDYNYYNQKFKD***KA***T***L***T***A***DTSISTAYMELSRLRSDDTAVYYCAR<br>GFFKGYFDVWGQGTTVTVSS | 188 |
| 155B11C6 HU-VH1-5 | QVQLVQSGAEVKKPGASVKVSCKASGYTFTSYTMHWV***K***QAPGQGLEW***I***G<br>YIDPKSDYNYYNQKFKD***KA***T***L***T***A***D***K***SISTAYMELSRLRSDDTAVYYCAR<br>GFFKGYFDVWGQGTTVTVSS | 189 |

(continued)

| 155B11C6 | | |
|---|---|---|
| | **Sequence** | **SEQ ID NO:** |
| IGHV1-2*02 | QVQLVQSGAEVKKPGASVKVSCKASGYTFTGYYMHWVRQAPGQGLEWMG WINPNSGGTNYAQKFQGRVTMTRDTSISTAYMELSRLRSDDTAVYYCAR | 190 |
| 155B11C6 HU-VH2-1 | QVQLVQSGAEVKKPGASVKVSCKASGYTFTSYTMHWVRQAPGQGLEWMG YIDPKSDYNYYNQKFKDRVT***LTA***D***K***STSTAYMELS***G***L***T***SEDTAVYYCAR GFFKGYFDVWGQGTTVTVSS | 191 |
| IGHV1-46*01 | QVQLVQSGAEVKKPGASVKVSCKASGYTFTSYYMHWVRQAPGQGLEWMG IINPSGGSTSYAQKFQGRVTMTRDTSTSTVYMELSSLRSEDTAVYYCAR | 192 |
| 155B11C6 VL | EIVLTQSPPLMAASPGEKVTITCSVSSSISSSNLHWYQQKSETSPKPWI YGTSNLASGVPIRFSGSGSGTSYSLTISSMEAEDAATYYCQQWTSYPFT FGAGTKLELK | 112 |
| 155B11C6 HU-VL1-0 | EIVLTQSPGTLSLSPGERATLSCSVSSSISSSNLHWYQQKPGQAPRLLI YGTSNLASGIPDRFSGSGSGTDFTLTISRLEPEDFAVYYCQQWTSYPFT FGQGTKLEIK | 193 |
| 155B11C6 HU-VL1-1 | EIVLTQSPGTLSLSPGERATLSCSVSSSISSSNLHWYQQKPGQAPRL***W***I YGTSNLASG***V***PDRFSGSGSGTDFTLTISRLEPEDFAVYYCQQWTSYPFT FGQGTKLEIK | 194 |
| 155B11C6 HU-VL1-2 | EIVLTQSPGTLSLSPGERATLSCSVSSSISSSNLHWYQQKPGQAPR***PW***I YGTSNLASG***V***PDRFSGSGSGTD***Y***TLTISRLEPEDFAVYYCQQWTSYPFT FGQGTKLEIK | 195 |
| 155B11C6 HU-VL1-3 | EIVLTQSPGTLSLSPGERATLSCSVSSSISSSNLHWYQQKPGQ***S***PR***PW***I YGTSNLASG***V***PDRFSGSGSGT***SY***TLTISRLEPEDFAVYYCQQWTSYPFT FGQGTKLEIK | 196 |
| IGKV3-20*01 | EIVLTQSPGTLSLSPGERATLSCRASQSVSSSYLAWYQQKPGQAPRLLI YGASSRATGIPDRFSGSGSGTDFTLTISRLEPEDFAVYYCQQYGSYT | 197 |
| 155B11C6 HU-VL2-0 | EIVLTQSPDFQSVTPKEKVTITCSVSSSISSSNLHWYQQKPDQSPKLLI KGTSNLASGVPSRFSGSGSGTDFTLTINSLEAEDAATYYCQQWTSYPFT FGQGTKLEIK | 198 |
| 155B11C6 HU-VL2-1 | EIVLTQSPDFQSVTPKEKVTITCSVSSSISSSNLHWYQQKPDQSPK***PW***I ***Y***GTSNLASGVPSRFSGSGSGTD***Y***TLTINSLEAEDAATYYCQQWTSYPFT FGQGTKLEIK | 199 |
| IGKV6-21*01 | EIVLTQSPDFQSVTPKEKVTITCRASQSIGSS*LHWYQQKPDQSPKLLI KYASQSFSGVPSRFSGSGSGTDFTLTINSLEAEDAATYYCHQSSSLP | 200 |

***Table 13B. Pairing of VH and VL for 155B11C6 Humanized Antibodies***

| 155B11C6 | HU-VL1-0 | HU-VL1-1 | HU-VL1-2 | HU-VL1-3 | HU-VL2-0 | HU-VL2-1 | VL |
|---|---|---|---|---|---|---|---|
| **HU-VH1-0** | z1 | z2 | z3 | z4 | | | |
| **HU-VH1-2** | z5 | z6 | z7 | | | | |
| **HU-VH1-4** | z8 | z9 | | | | | |
| **HU-VH1-5** | z10 | | | | | | |
| **HU-VH2-1** | | | | | z11 | z12 | |

(continued)

| 155B11C6 | HU-VL1-0 | HU-VL1-1 | HU-VL1-2 | HU-VL1-3 | HU-VL2-0 | HU-VL2-1 | VL |
|---|---|---|---|---|---|---|---|
| VH | | | | | | | Chimeric |

### *3.2 Binding properties of the CDH17 humanized antibodies to human CDH17-expressing cells*

Cell binding activity of the CDH17 humanized antibodies was confirmed in cell-based binding assay with various CDH17-expressing cells including HEK293-hCDH17, pancreatic cancer cell line AsPC-1, gastric cancer cell line AGS and colorectal cancer cell line HCT-8 cells. The assay was performed following the protocol described above.

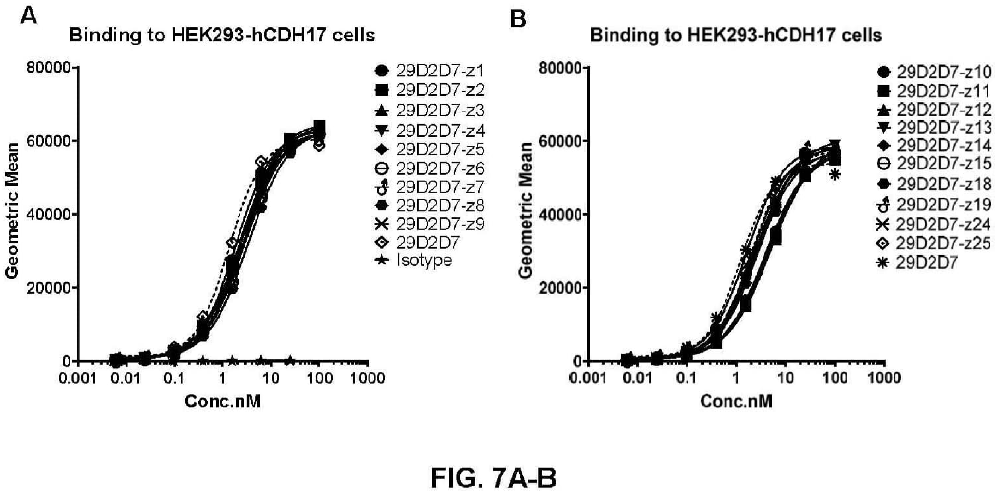

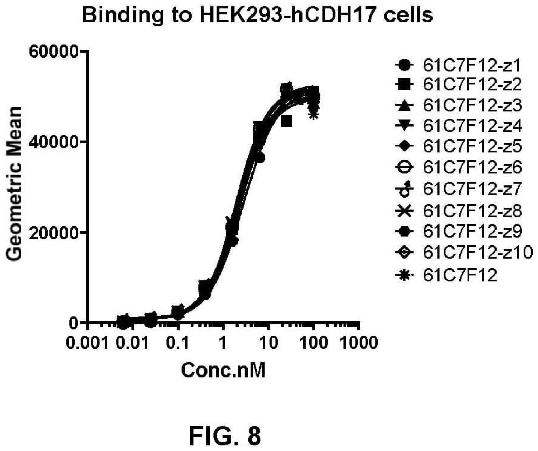

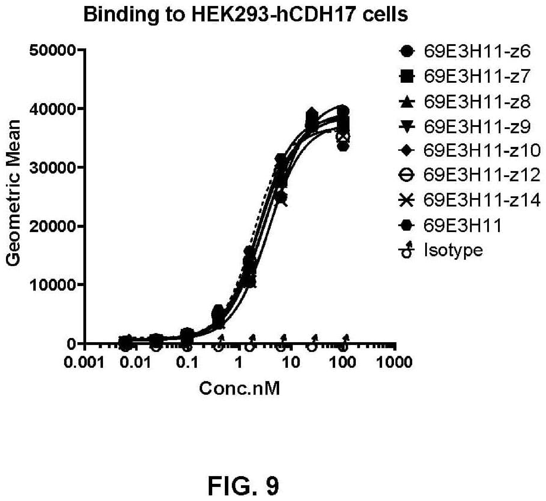

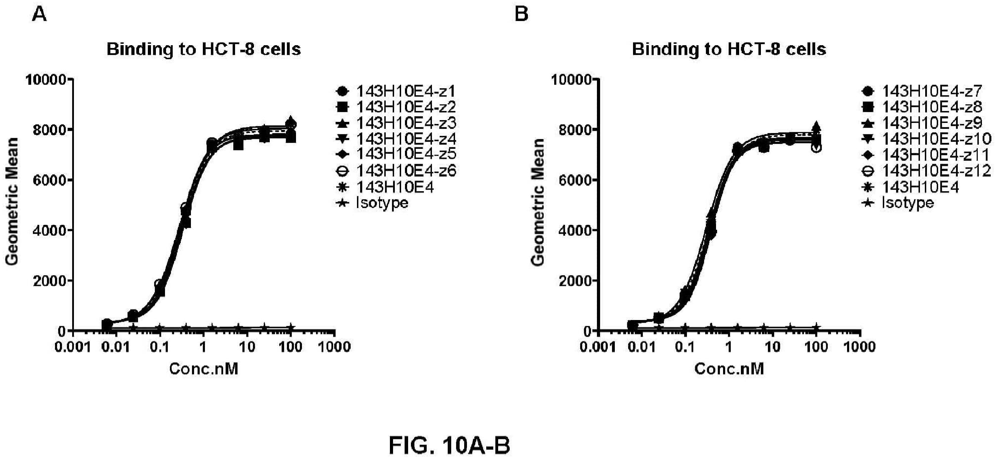

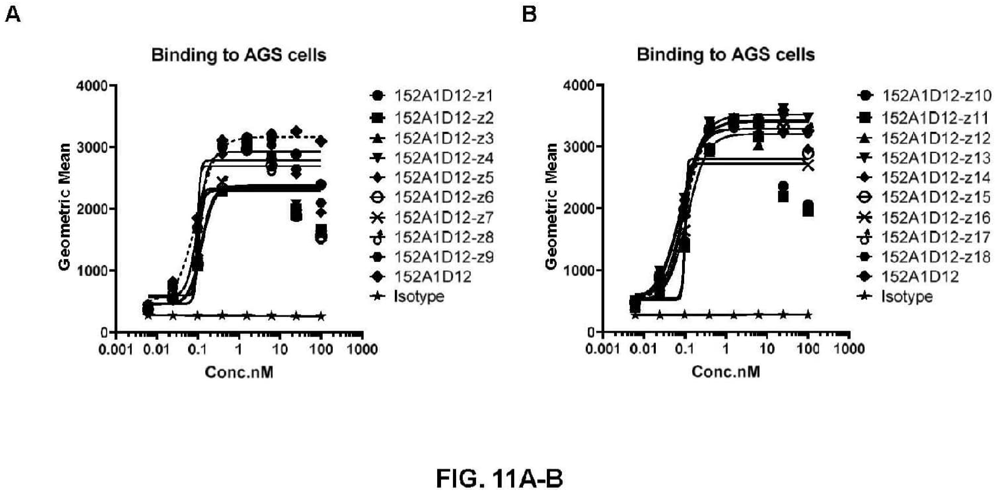

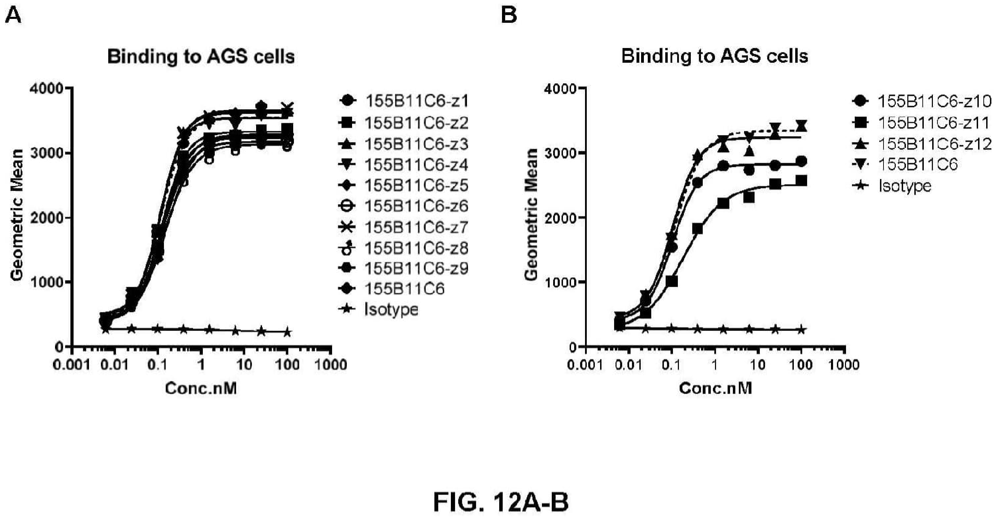

As shown in **FIG. 6- FIG. 12,** the CDH17 humanized antibodies including 29H8D3-z3, 29H8D3-z6, 29H8D3-z9, 29H8D3-z12, 29D2D7-z19, all the humanized 61C7F12 mAbs, 69E3H11-z10, 69E3H11-z12, 143H10E4-z3, 143H10E4-z6, 143H10E4-z9, 152A1D12-z13, 152A1D12-z17, 152A1D12-z18, 155B11C6-z3, 155B11C6-z4 and 155B11C6-z7 showed comparable cell binding efficacy to their respective chimeric antibodies.

To confirm the antigen binding potency, species cross-reactivity and specificity of CDH17 humanized antibody, one of the humanized antibodies 29H8D3-z12 was evaluated for its binding potency to recombinant CDH17 proteins of different species and several cadherin superfamily proteins including human CDH16, CDH9, CDH10, CDH3 and CDH6 by ELISA.

In order to determine the binding ability of the humanized mAb to various antigens, ELISA-based binding assay was performed as follows. In brief, HCDH17-his protein (customized by Biointron), rhesus CDH17-his (UniProt reference sequence: A0A1D5R2B4, Q23-T784, Kactus, Cat. No. CDH-RM117), cyno CDH17-hFc (Isoform 1, Sino bio, Cat. No. 90147-C02H), rat CDH17-his (Sino bio, Cat. No. 80283-R08H), mouse CDH17-his (Kactus, Cat. No. CDH-MM117), human CDH16-his (hCDH16-his, Novoprotein, Cat. No. CJ16), human CDH9-his (Acro bio, Cat. No. CA9-H52H6), human CDH10-his protein (Acro bio, Cat. No. CA0-H52H5), human CDH3-his protein (Kactus, Cat. No. CDH-HM103) and human CDH6-his protein (Katcus, Cat. No. CDH-HM106) were diluted with 1×ELISA coating buffer (Solarbio, Cat. No. C1050) at 1 μg/mL and adsorbed to wells of 96-well microplates overnight at 4 °C. After blocking the wells with 2 % bovine serum albumin (BSA) to prevent non-specific binding, the humanized CDH17 mAb or an isotype control were titrated at a 4-fold dilution rate starting from 50 nM and added to the wells pre-adsorbed with the antigen. The mixture was incubated for 1 hour at room temperature (RT). The bound CDH17 mAb were recognized by a detective antibody goat anti-human IgG $F(ab')_2$ conjugated with horseradish peroxidase (HRP) (Jackson Immuno, Cat. No.109-036-097). Tetramethylbenzidine (TMB), a substrate of HRP, was added to the wells to visualize the binding signal. After sufficient color development, stop solution was added to the wells. The absorbance of the signal was detected at 450 nm with Envision multilabel plate readers (PerkinElemer). The graph and statistics analysis were generated with four-parameter nonlinear regression curve fit in Graphpad Prism 9 software.

As shown in **FIG. 6C-6G**, 29H8D3-z12 efficiently bound to human, rhesus and cyno CDH17 isoform 1 proteins but not rat and mouse CDH17 proteins. The binding EC50 of the 29H8D3-z12 antibody (EC50: 0.187 nM) to human CDH17 was comparable to its parental antibody 29H8D3 (EC50: 0.238 nM shown in **Table 3**). More importantly, 29H8D3-z12 displayed a comparable binding EC50 to human and rhesus CDH17 proteins (EC50: 0.187 nM vs. 0.163 nM), indicating a favorable cross-reactivity of 29H8D3-z12 to non-human primate (NHP) CDH17 protein which would facilitate preclinical NHP toxicity evaluation. Moreover, 29H8D3-z12 exhibited high selectivity of binding to human CDH17 as no binding to human CDH16, CDH9, CDH10, CDH3 and CDH6 proteins was observed (**FIG. 6H-6L**).

In order to confirm the binding potency of 29H8D3-z12 to NHP CDH17 antigen, the cell-based binding by FACS was performed with rhesus CDH17 (UniProt reference sequence: A0A1D5R2B4, M1-S832) overexpressed on HEK293 cells (HEK293-rhesus CDH17, in-house constructed) and HEK293-cyno CDH17 (Isoform 2, customized by Genome-ditech in **Example 2.7**). For cell binding assay, the CDH17 mAb or an isotype control were diluted at a three-fold dilution rate starting from a concentration of 50 nM in the staining buffer (DPBS buffer containing 2% FBS). The antibody dilutions were incubated with $5 \times 10^4$ cells in 96-well microplates for 30 minutes (mins) at 4 °C. Then the cell-antibody mixture was washed with staining buffer twice. The antibodies binding to the antigen on the cell surface were detected with goat anti-human IgG Fc secondary antibody conjugated with Alexa Fluor® 647 (Jackson Immuno, Cat. No. 109-606-098) at a dilution rate of 1:1000 for 30 mins at 4 °C followed by extensive washing. Cells were analyzed by flow cytometer LSRFortessa™ Cell Analyzer (BD Biosciences). Data were analyzed with Flowjo 10.0 software. The graphs and statistics analysis were generated with three-parameter or four-parameter nonlinear regression curve fit in Graphpad Prism 9 software.

As shown in **FIG. 6M** and **6N**, 29H8D3-z12 bound efficiently to rhesus CDH17 (EC50: 0.225 nM). However, in consistence with previous result shown in **FIG. 5A**, 29H8D3-z12 showed no binding to cyno CDH17 isoform 2 protein expressed on HEK293 cells, although binding to recombinant cyno CDH17 isoform 1 protein was observed in **FIG. 6E**. This phenomenon was probably due to the low similarity of the predicted EC1 domain of cyno CDH17 isoform 2 to that of cyno

CDH17 isoform 1 which showed higher homology with human CDH17 protein. Collectively, 29H8D3-z12 exhibited high specificity and favorable cross-reactivity with efficient binding to human and rhesus CDH17 protein expressed on cells.

### *3.3 Affinity ranking of humanized antibodies by Biacore™*

To explore whether the humanized antibodies could maintain their binding kinetics, affinity ranking was performed with Biacore ™. The antibodies (2 μg/ml) were captured with Protein A chips. Single dose (*, 100 nM or 400 nM) or two doses (25 nM and 100 nM) of human CDH17-his protein were injected over captured antibodies at a flow rate of 30 μL/min. The antigen was allowed to associate for 120 s-150 s and dissociate for 200 s- 500 s. The experiment was carried out on a Biacore ™ 8K. Data analysis was carried out using Biacore ™ 8K evaluation software.

The results shown in **Table 14** demonstrated that CDH17 humanized antibodies including 29H8D3-z3, 29H8D3-z6, 29H8D3-z9, 29H8D3-z12, 29H8D3-z17 to 29H8D3-z23, 29D2D7-z19, 61C7F12-z1, 61C7F12-z2, 61C7F12-z3, 61C7F12-z8, 61C7F12-z9, 61C7F12-z10, 69E3H11-z12, 143H10E4-z3, 143H10E4-z6, 143H10E4-z9, 152A1D12-z13, 152A1D12-z17, 152A1D12-z18, 155B11C6-z3, 155B11C6-z4 and 155B11C6-z7 showed comparable affinity to their respective chimeric antibodies.

***Table 14. Affinity Ranking Results of CDH17 Humanized Antibodies***

| Affinity / Antibody | Antigen: Human CDH17 His | | |
|---|---|---|---|
| | **ka(1/Ms)** | **kd(1/s)** | **KD(M)** |
| 29H8D3 | 8.80E+05 | 1.12E-03 | 1.28E-09 |
| 29H8D3-z3 | 1.15E+06 | 5.83E-04 | 5.07E-10 |
| 29H8D3-z6 | 1.14E+06 | 6.63E-04 | 5.81E-10 |
| 29H8D3-z9 | 1.11E+06 | 6.75E-04 | 6.06E-10 |
| 29H8D2-z12 | 8.93E+05 | 7.31E-04 | 8.19E-10 |
| 29H8D2-z17 | 3.94E+05 | 3.62E-04 | 9.19E-10 |
| 29H8D2-z18 | 3.60E+05 | 4.15E-04 | 1.15E-09 |
| 29H8D2-z19 | 3.48E+05 | 4.44E-04 | 1.28E-09 |
| 29H8D2-z20 | 3.12E+05 | 4.47E-04 | 1.43E-09 |
| 29H8D2-z21 | 3.55E+05 | 3.93E-04 | 1.11E-09 |
| 29H8D2-z22 | 3.41E+05 | 4.19E-04 | 1.23E-09 |
| 29H8D2-z23 | 3.40E+05 | 4.30E-04 | 1.26E-09 |
| 29D2D7* | 2.44E+05 | 5.93E-04 | 2.43E-09 |
| 29D2D7-z19 | 1.33E+05 | 7.53E-04 | 5.65E-09 |
| 61C7F12 | 1.47E+05 | 3.86E-03 | 2.63E-08 |
| 61C7F12-z1 | 1.61E+05 | 7.03E-03 | 4.36E-08 |
| 61C7F12-z2 | 1.46E+05 | 1.16E-02 | 7.92E-08 |
| 61C7F12-z3 | 1.44E+05 | 1.18E-02 | 8.17E-08 |
| 61C7F12-z4 | 1.53E+05 | 1.63E-02 | 1.07E-07 |
| 61C7F12-z5 | 5.70E+04 | 2.69E-02 | 4.71E-07 |
| 61C7F12-z6 | 6.15E+04 | 2.21E-02 | 3.59E-07 |
| 61C7F12-z7 | 6.05E+04 | 1.94E-02 | 3.21E-07 |

| 61C7F12-z8 | 1.33E+05 | 1.13E-02 | 8.51E-08 |
|---|---|---|---|
| 61C7F12-z9 | 1.71E+05 | 7.34E-03 | 4.28E-08 |
| 61C7F12-z10 | 1.67E+05 | 1.17E-02 | 7.00E-08 |
| 69E3H11* | 3.98E+04 | 2.36E-04 | 5.93E-09 |
| 69E3H11-z12 | 3.53E+05 | 3.28E-04 | 9.31E-10 |
| 143H10E4 | 1.92E+05 | 6.09E-04 | 3.18E-09 |
| 143H10E4-z3 | 1.93E+05 | 6.06E-04 | 3.14E-09 |
| 143H10E4-z6 | 1.81E+05 | 5.39E-04 | 2.98E-09 |
| 143H10E4-z9 | 1.84E+05 | 5.31E-04 | 2.88E-09 |
| 152A1D12 | 1.72E+05 | 1.11E-03 | 6.44E-09 |
| 152A1D12-z13 | 1.99E+05 | 1.26E-03 | 6.34E-09 |
| 152A1D12-z17 | 2.09E+05 | 1.18E-03 | 5.62E-09 |
| 152A1D12-z18 | 2.12E+05 | 1.22E-03 | 5.73E-09 |
| 155B11C6 | 2.74E+05 | 2.74E-03 | 9.98E-09 |
| 155B11C6-z3 | 2.72E+05 | 2.30E-03 | 8.47E-09 |
| 155B11C6-z4 | 2.47E+05 | 2.44E-03 | 9.87E-09 |
| 155B11C6-z7 | 2.37E+05 | 1.65E-03 | 6.95E-09 |

* Single dose affinity

**Example 4. Optimization of the Humanized CDH17 Antibodies**

After antibody humanization, CDR regions are often needed to be further optimized to remove potential post-translational modification (PTM) sites in order to increase developability including long-term stability, manufacturability, and homogeneity of the humanized antibodies. PTM such as deamidation, isomerization, glycosylation and oxidation can compromise the potency, efficacy, and safety of therapeutic antibodies. Alternatively, in certain circumstance, affinity maturation of the humanized antibodies is pursued to improve the binding affinity of the humanized antibodies which may bring enhanced activities.

***4.1 PTM removal and affinity maturation design of the humanized CDH17 antibodies***

In this case, computational tools have been used to predict PTM liable sites to facilitate engineering antibodies with better physical and chemical properties.

A close examination of the CDR regions of the 29D2D7-z19 humanized mAb identified an unpaired cysteine (C) located in the CDR3 of the VL region which might induce heterogeneity of the antibody during the production process. Therefore, amino acid replacement was conducted in this location and the sequences of the mutated variable region of the 29D2D7-z19 humanized mAbs are listed in **Table 15A** and the mutated CDRs are summarized in **Table 15B**.

PTM site prediction of the CDR regions of the 29H8D3-z12 humanized mAb identified a potential isomerization site Aspartic acid (D) located in the CDR2 of the VH region, two potential deamidation sites Asparagine (N) located in the CDR2 of the VL region and a potential oxidation site Methionine (M) located in the CDR3 of the VL region which might be affected in an oxidation stress condition. Therefore, amino acid replacement was conducted in this location and the selected sequences of the mutated variable region of the 29H8D3-z12 humanized mAbs are listed in **Table 15A** and the mutated CDRs are summarized in **Table 15B.**

***Table 15A. Sequences of Variable Regions of CDH17 Humanized Antibodies (underlining indicates CDR; bold/italic indicates PTM mutations)***

| Name | Sequence | SEQ ID NO: |
|---|---|---|
| 29D2D7-z19 VH | QVQLVQSGAEVKKPGASVKVSCKASGYTFTDYDMHWVKQAPGQGLEWIGGFDPESAGTVYNLRFKDKATLTADKSTSTAYMELRSLRSEDTAVYYCARKGYYSVSSPFTYWGQGTLVTVSS | 131 |
| 29D2D7-z19 VL | DIVMTQSPDSLAVSLGERATINCKSSQSLLYSSNQKNYLAWYQQKPGQPPKLLIYWASTRESGVPDRFSGSGSGTDFTLTISSLQAEDVAVYYCQQCYSYPRTFGGGTKVEIK | 134 |

(continued)

| Name | Sequence | SEQ ID NO: |
|---|---|---|
| 29D2D7-z19p1 VL | DIVMTQSPDSLAVSLGERATINCKSSQSLLYSSNQKNYLAWYQQKPGQPPKLLIYWASTRESGVPDRFSGSGSGTDFTLTISSLQAEDVAVYYCQQ***H***YSYPRTFGGGTKVEIK | 203 |
| 29D2D7-z19p2 VL | DIVMTQSPDSLAVSLGERATINCKSSQSLLYSSNQKNYLAWYQQKPGQPPKLLIYWASTRESGVPDRFSGSGSGTDFTLTISSLQAEDVAVYYCQQ***L***YSYPRTFGGGTKVEIK | 204 |
| 29D2D7-z19p3 VL | DIVMTQSPDSLAVSLGERATINCKSSQSLLYSSNQKNYLAWYQQKPGQPPKLLIYWASTRESGVPDRFSGSGSGTDFTLTISSLQAEDVAVYYCQQ***Y***YSYPRTFGGGTKVEIK | 205 |
| 29D2D7-z19p4 VL | DIVMTQSPDSLAVSLGERATINCKSSQSLLYSSNQKNYLAWYQQKPGQPPKLLIYWASTRESGVPDRFSGSGSGTDFTLTISSLQAEDVAVYYCQQ***S***YSYPRTFGGGTKVEIK | 206 |
| 29D2D7-z19p5 VL | DIVMTQSPDSLAVSLGERATINCKSSQSLLYSSNQKNYLAWYQQKPGQPPKLLIYWASTRESGVPDRFSGSGSGTDFTLTISSLQAEDVAVYYCQQ***T***YSYPRTFGGGTKVEIK | 207 |
| 29H8D3-z12 VH | QVQLVQSGAEVKKPGASVKVSCKASGYTFTDHTIHWMRQRPGQGLEWIGYIYPRDGYTKYNEKFKDRATLTADTSTSTAYMELRSLRSDDTAVYYCARFYYGYWYFDVWGQGTTVTVSS | 116 |
| 29H8D3-z12 VL | DIVMTQSPLSLPVTPGEPASISCRSSKSLLRSNGNTYLYWFLQKPGQSPQVLIYRMSNLASGVPDRFSGSGSGTDFTLKISRVEAEDVGVYYCMQHLEYPFTFGQGTKLEIK | 123 |
| 29H8D3-z12p9 VL | DIVMTQSPLSLPVTPGEPASISCRSSKSLLRSNGNTYLYWFLQKPGQSPQVLIYRMSNLASGVPDRFSGSGSGTDFTLKISRVEAEDVGVYYC***H***QHLEYPFTFGQGTKLEIK | 208 |
| 29H8D3-z12p10 VL | DIVMTQSPLSLPVTPGEPASISCRSSKSLLRSNGNTYLYWFLQKPGQSPQVLIYRMSNLASGVPDRFSGSGSGTDFTLKISRVEAEDVGVYYC***K***QHLEYPFTFGQGTKLEIK | 209 |
| 29H8D3-z12p13 VL | DIVMTQSPLSLPVTPGEPASISCRSSKSLLRSNGNTYLYWFLQKPGQSPQVLIYRMSNLASGVPDRFSGSGSGTDFTLKISRVEAEDVGVYYC***S***QHLEYPFTFGQGTKLEIK | 210 |
| 29H8D3-z12p14 VL | DIVMTQSPLSLPVTPGEPASISCRSSKSLLRSNGNTYLYWFLQKPGQSPQVLIYRMSNLASGVPDRFSGSGSGTDFTLKISRVEAEDVGVYYC***T***QHLEYPFTFGQGTKLEIK | 211 |
| 29H8D3-z12p15 VL | DIVMTQSPLSLPVTPGEPASISCRSSKSLLRSNGNTYLYWFLQKPGQSPQVLIYRMSNLASGVPDRFSGSGSGTDFTLKISRVEAEDVGVYYC***N***QHLEYPFTFGQGTKLEIK | 212 |
| 29H8D3-z12p16 VL | DIVMTQSPLSLPVTPGEPASISCRSSKSLLRSNGNTYLYWFLQKPGQSPQVLIYRMSNLASGVPDRFSGSGSGTDFTLKISRVEAEDVGVYYC***Q***QHLEYPFTFGQGTKLEIK | 213 |

(continued)

| Name | Sequence | SEQ ID NO: |
|---|---|---|
| 29H8D3-z12p17 VL | DIVMTQSPLSLPVTPGEPASISCRSSKSLLRSNGNTYLYWFLQKPGQSPQVLIYRMSNLASGVPDRFSGSGSGTDFTLKISRVEAEDVGVYYC***G***QHLEYPFTFGQGTKLEIK | 214 |
| 29H8D3-z12p21 VL | DIVMTQSPLSLPVTPGEPASISCRSSKSLLRSNGNTYLYWFLQKPGQSPQVLIYRMSNLASGVPDRFSGSGSGTDFTLKISRVEAEDVGVYYC***I***QHLEYPFTFGQGTKLEIK | 215 |
| 29H8D3-z12p22 VL | DIVMTQSPLSLPVTPGEPASISCRSSKSLLRSNGNTYLYWFLQKPGQSPQVLIYRMSNLASGVPDRFSGSGSGTDFTLKISRVEAEDVGVYYC***L***QHLEYPFTFGQGTKLEIK | 216 |
| 29H8D3-z12p1 VH | QVQLVQSGAEVKKPGASVKVSCKASGYTFTDHTIHWMRQRPGQGLEWIGYIYPR***E***GYTKYNEKFKDRATLTADTSTSTAYMELRSLRSDDTAVYYCARFYYGYWYFDVWGQGTTVTVSS | 248 |
| 29H8D3-z12p1 VL | DIVMTQSPLSLPVTPGEPASISCRSSKSLLRSNGNTYLYWFLQKPGQSPQVLIYRMSNLASGVPDRFSGSGSGTDFTLKISRVEAEDVGVYYCMQHLEYPFTFGQGTKLEIK | 123 |
| 29H8D3-z12p3 VH | QVQLVQSGAEVKKPGASVKVSCKASGYTFTDHTIHWMRQRPGQGLEWIGYIYPRDGYTKYNEKFKDRATLTADTSTSTAYMELRSLRSDDTAVYYCARFYYGYWYFDVWGQGTTVTVSS | 116 |
| 29H8D3-z12p3 VL | DIVMTQSPLSLPVTPGEPASISCRSSKSLLRSN***A***NTYLYWFLQKPGQSPQVLIYRMSNLASGVPDRFSGSGSGTDFTLKISRVEAEDVGVYYCMQHLEYPFTFGQGTKLEIK | 249 |
| 29H8D3-z12p4 VH | QVQLVQSGAEVKKPGASVKVSCKASGYTFTDHTIHWMRQRPGQGLEWIGYIYPR***E***GYTKYNEKFKDRATLTADTSTSTAYMELRSLRSDDTAVYYCARFYYGYWYFDVWGQGTTVTVSS | 248 |
| 29H8D3-z12p4 VL | DIVMTQSPLSLPVTPGEPASISCRSSKSLLRSN***A***NTYLYWFLQKPGQSPQVLIYRMSNLASGVPDRFSGSGSGTDFTLKISRVEAEDVGVYYCMQHLEYPFTFGQGTKLEIK | 249 |
| 29H8D3-z12p7 VH | QVQLVQSGAEVKKPGASVKVSCKASGYTFTDHTIHWMRQRPGQGLEWIGYIYPR***E***GYTKYNEKFKDRATLTADTSTSTAYMELRSLRSDDTAVYYCARFYYGYWYFDVWGQGTTVTVSS | 248 |
| 29H8D3-z12p7 VL | DIVMTQSPLSLPVTPGEPASISCRSSKSLLRSN***AQ***TYLYWFLQKPGQSPQVLIYRMSNLASGVPDRFSGSGSGTDFTLKISRVEAEDVGVYYCMQHLEYPFTFGQGTKLEIK | 250 |

***Table 15B. Optimized Heavy Chain CDR and Light Chain CDR of 29D2D7 and 29H8D3***

| CDR | Sequence | SEQ ID NO: |
|---|---|---|
| 29D2D7 VL CDR3 | QQCYSYPRT<br>QQ***H***YSYPRT<br>QQ***L***YSYPRT<br>QQ***Y***YSYPRT<br>QQ***S***YSYPRT<br>QQ***T***YSYPRT | 22<br>217<br>218<br>219<br>220<br>221 |

(continued)

| CDR | Sequence | SEQ ID NO: |
|---|---|---|
| 29H8D3 VL CDR3 | MQHLEYPFT<br>***H***QHLEYPFT<br>***K***QHLEYPFT<br>***S***QHLEYPFT<br>***T***QHLEYPFT<br>***N***QHLEYPFT<br>***Q***QHLEYPFT<br>***G***QHLEYPFT<br>***I***QHLEYPFT<br>***L***QHLEYPFT | 30<br>222<br>223<br>224<br>225<br>226<br>227<br>228<br>229<br>230 |
| 29H8D3 VH CDR2 | YIYPRDGYTKYNEKFKD<br>YIYPR***E***GYTKYNEKFKD | 26<br>245 |
| 29H8D3 VL CDR1 | RSSKSLLRSNGNTYLY<br>RSSKSLLRSN***A***NTYLY<br>RSSKSLLRSN***AQ***TYLY | 28<br>246<br>247 |

Alternatively, affinity maturation has been conducted to improve the affinity and binding activity of humanized CDH17 antibodies.

A series of 69E3H11-z12 variants with mutations in the CDR regions were displayed on phages. The phage clones with high binding potency to human CHD17 protein and CHD17 expressed on tumor cells were sequenced. The resulting Ab sequences were used to generate mAb for further characterization. The sequences of the variable region of the affinity maturated 69E3H11-z12 antibodies are summarized in **Table 15C.**

***Table 15C. Sequences of Variable Regions of CDH17 Humanized Antibodies (underlining indicates CDR; bold/italic indicates mutations)***

| Name | Sequence | SEQ ID NO: |
|---|---|---|
| 69E3H11-z12 VH | QVQLVQSGAEVKKPGASVKVSCKASGYTFTTYWMHWVKQAPGQRLEWIGEIDPSDSYANYNQKFKGKATLTVDKSASTAYMELSSLRSEDTAVYYCALYYGYGDWGQGTTVTVSS | 152 |
| 69E3H11-z12 VL | EIVLTQSPGTLSLSPGERATLSCSVSSSISSIHLHWYQQKPGQAPRLLIYRTSNLASGIPDRFSGSGSGTDFTLTISRLEPEDFAVYYCQQGSNLPLTFGQGTRLEIK | 160 |
| 69E3H11-z12a19 VH | QVQLVQSGAEVKKPGASVKVSCKASGYTFTTYWMHWVKQAPGQRLEWIGEIDPSDSYANYNQKFKGKATLTVDKSASTAYMELSSLRSEDTAVYYCALYYGYGDWGQGTTVTVSS | 152 |
| 69E3H11-z12a19 VL | EIVLTQSPGTLSLSPGERATLSCSVSSSIS***K***IHLHWYQQKPGQAPRLLIYRTSNLASGIPDRFSGSGSGTDFTLTISRLEPEDFAVYYCQQGSNLPLTFGQGTRLEIK | 232 |
| 69E3H11-z12a20 VH | QVQLVQSGAEVKKPGASVKVSCKASGYTFTTYWMHWVKQAPGQRLEWIGEIDPSDSYANYNQKFKGKATLTVDKSASTAYMELSSLRSEDTAVYYCALYYGYGDWGQGTTVTVSS | 152 |
| 69E3H11-z12a20 VL | EIVLTQSPGTLSLSPGERATLSCSVSSSIS***R***IHLHWYQQKPGQAPRLLIYRTSNLASGIPDRFSGSGSGTDFTLTISRLEPEDFAVYYCQQGSNLPLTFGQGTRLEIK | 233 |

(continued)

| Name | Sequence | SEQ ID NO: |
|---|---|---|
| 69E3H11-z12a21 VH | QVQLVQSGAEVKKPGASVKVSCKASGYTFTTYWMHWVKQAPGQRLEWIGEIDPSDSYANYNQKFKGKATLTVDKSASTAYMELSSLRSEDTAVYYCALYYGYGDWGQGTTVTVSS | 152 |
| 69E3H11-z12a21 VL | EIVLTQSPGTLSLSPGERATLSCSVSASISRIHLHWYQQKPGQAPRLLIYRTSNLASGIPDRFSGSGSGTDFTLTISRLEPEDFAVYYCQQGSNLPLTFGQGTRLEIK | 234 |
| 69E3H11-z12a26 VH | QVQLVQSGAEVKKPGASVKVSCKASGYTFTTYWMHWVKQAPGQRLEWIGEIDPSDSYANYNQKFKGKATLTVDKSASTAYMELSSLRSEDTAVYYCALYYGYGDWGQGTTVTVSS | 152 |
| 69E3H11-z12a26 VL | EIVLTQSPGTLSLSPGERATLSCSVSSSISSIHLHWYQQKPGQAPRLLIYRTRNLASGIPDRFSGSGSGTDFTLTISRLEPEDFAVYYCQQGSNLPLTFGQGTRLEIK | 235 |
| 69E3H11-z12a27 VH | QVQLVQSGAEVKKPGASVKVSCKASGYTFTTYWMHWVKQAPGQRLEWIGEIDPSDSYANYNQKFKGKATLTVDKSASTAYMELSSLRSEDTAVYYCALYYGYGDWGQGTTVTVSS | 152 |
| 69E3H11-z12a27 VL | EIVLTQSPGTLSLSPGERATLSCSVSSSISSIHLHWYQQKPGQAPRLLIYRTRNRASGIPDRFSGSGSGTDFTLTISRLEPEDFAVYYCQQGSNLPLTFGQGTRLEIK | 236 |
| 69E3H11-z12a28 VH | QVQLVQSGAEVKKPGASVKVSCKASGYTFTTYWMHWVKQAPGQRLEWIGEIDPSDSYANYNQKFKGKATLTVDKSASTAYMELSSLRSEDTAVYYCALYYGYGDWGQGTTVTVSS | 152 |
| 69E3H11-z12a28 VL | EIVLTQSPGTLSLSPGERATLSCSVSSSISSIHLHWYQQKPGQAPRLLIYRTKNLASGIPDRFSGSGSGTDFTLTISRLEPEDFAVYYCQQGSNLPLTFGQGTRLEIK | 237 |
| 69E3H11-z12a42 VH | QVQLVQSGAEVKKPGASVKVSCKASGYTFTTYWMHWVKQAPGQRLEWIGEIDPSDSYANYNQKFKGKATLTVDKSASTAYMELSSLRSEDTAVYYCALYYGYGDWGQGTTVTVSS | 152 |
| 69E3H11-z12a42 VL | EIVLTQSPGTLSLSPGERATLSCSVSASISRIHLHWYQQKPGQAPRLLIYRTRNRASGIPDRFSGSGSGTDFTLTISRLEPEDFAVYYCQQGSNLPLTFGQGTRLEIK | 238 |

***Table 15D. Optimized Light Chain CDR1 and CDR2 of 69E3H11***

| Antibody | Sequence | SEQ ID NO: |
|---|---|---|
| 69E3H11 VL CDR1 | SVSSSISSIHLH<br>SVSSSISKIHLH<br>SVSSSISRIHLH<br>SVSASISRIHLH | 52<br>239<br>240<br>241 |
| 69E3H11 VL CDR2 | RTSNLAS<br>RTRNLAS<br>RTRNRAS<br>RTKNLAS | 53<br>242<br>243<br>244 |

***4.2 Binding to human CDH17-expressing cells***

Cell binding activity of the PTM site removed and affinity maturated CDH17 humanized antibodies was confirmed in cell-based binding assay with colorectal cancer cell line HCT-8 and LoVo cells as well as pancreatic cancer cell line AsPC1 cells. The assay was performed following the protocol described above.

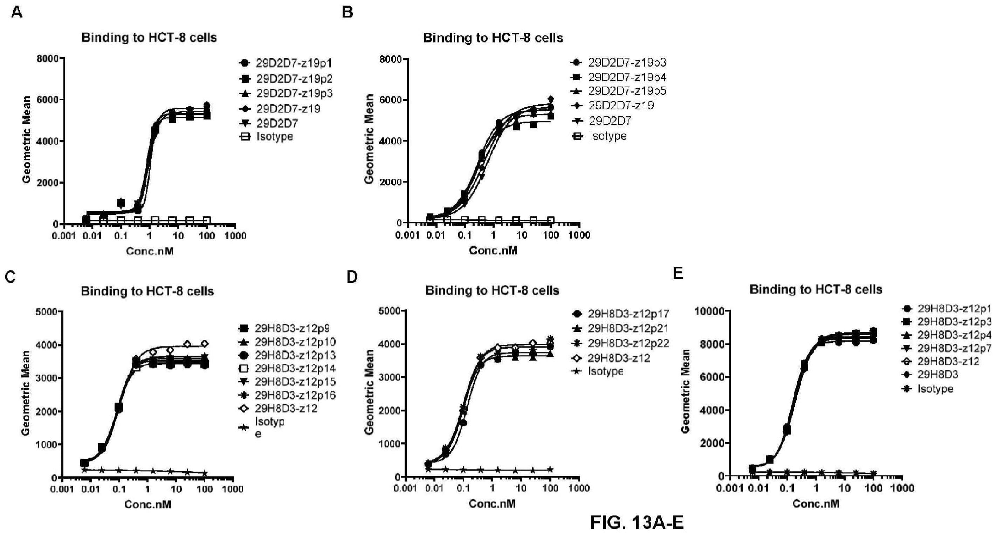

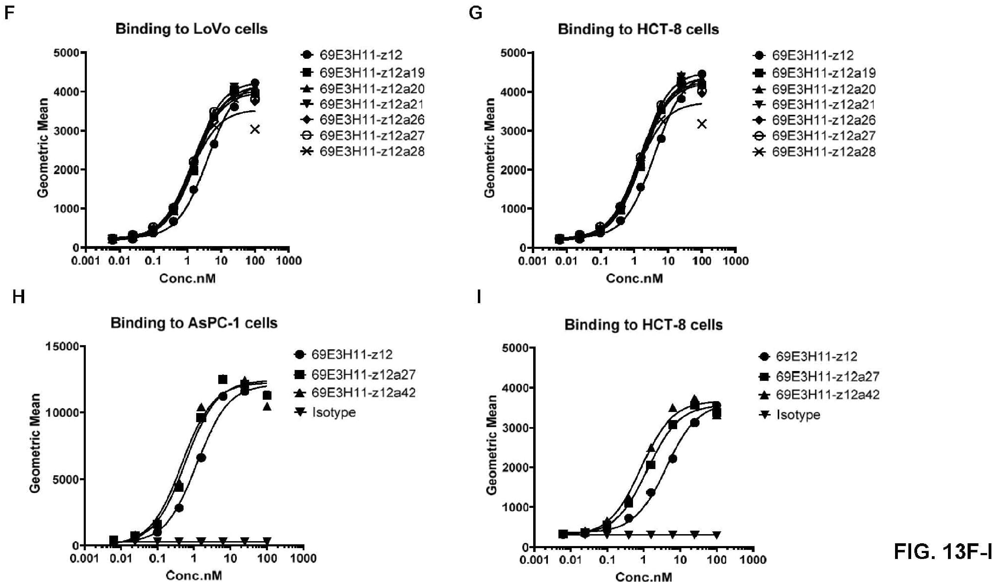

As shown in **FIG. 13A** and **13B**, all the PTM removed variants of 29D2D7-z19 except 29D2D7-z19p4 showed comparable binding potencies to human CDH17 expressed on cells to that of the parental antibody 29D2D7-z19. As shown in **FIG. 13C, 13D** and **13E**, PTM removed 29H8D3-z12p22, 29H8D3-z12p3, 29H8D3-z12p4 and 29H8D3-z12p7 showed comparable human CDH17 binding to the parental antibody 29H9D3-z12 on HCT-8 cells.

As shown in **FIG. 13F-13I**, all the affinity maturated variants of 69E3H11-z12 showed improved binding potency to human CDH17 expressed on LoVo, HCT-8 and AsPC1 cells when compared to their parental antibody 69E3H11-z12.

***4.3 Affinity ranking by Biacore™***

To explore whether the PTM site-removed and the affinity matured humanized antibodies could maintain or improve their binding kinetics, two doses affinity ranking was performed with Biacore ™. The antibodies (2 μg/ml) were captured with Protein A chips. Human CDH17-his protein at 25 nM and 100 nM were injected over captured antibodies for 120 s or 150 s at a flow rate of 30 μL/min. The antigen was allowed to dissociate for 200 s. The experiment was carried out on a Biacore ™ 8K. Data analysis was carried out using Biacore ™ 8K evaluation software.

The results shown in **Table 16A** demonstrated that humanized antibodies including 29D2D7-z19p3, 29D2D7-z19p5 and all the PTM removed 29H8D3-z12 showed comparable affinity to their parental humanized antibodies. All the affinity maturated antibodies showed improved binding affinity when compared to their parental antibody 69E3H11-z12.

***Table 16A. Affinity Ranking Results of CDH17 Humanized Antibodies by Biacore™***

| Affinity / Antibody | Antigen: Human CDH17 His | | |
|---|---|---|---|
| | **ka(1/Ms)** | **kd(1/s)** | **KD(M)** |
| 29D2D7-z19 | 1.33E+05 | 7.53E-04 | 5.65E-09 |
| 29D2D7-z19p3 | 1.14E+05 | 7.83E-04 | 6.87E-09 |
| 29D2D7-z19p5 | 1.12E+05 | 8.46E-04 | 7.56E-09 |
| 29H8D3-z12 | 3.51E+05 | 5.43E-04 | 1.55E-09 |
| 29H8D3-z12p1 | 3.16E+05 | 2.74E-04 | 8.67E-10 |
| 29H8D3-z12p3 | 3.63E+05 | 8.13E-04 | 2.24E-09 |
| 29H8D3-z12p4 | 3.23E+05 | 4.01E-04 | 1.24E-09 |
| 29H8D3-z12p7 | 3.58E+05 | 4.05E-04 | 1.13E-09 |
| 69E3H11-z12 | 7.29E+04 | 1.90E-04 | 2.61E-09 |
| 69E3H11-z12a19 | 9.18E+04 | 9.09E-05 | 9.90E-10 |
| 69E3H11-z12a20 | 9.19E+04 | 9.40E-05 | 1.02E-09 |
| 69E3H11-z12a21 | 9.75E+04 | 9.58E-05 | 9.83E-10 |
| 69E3H11-z12a26 | 8.80E+04 | 9.85E-05 | 1.12E-09 |
| 69E3H11-z12a27 | 8.17E+04 | 1.03E-04 | 1.26E-09 |
| 69E3H11-z12a28 | 8.79E+04 | 1.03E-04 | 1.17E-09 |

In order to confirm the binding affinity of various antibodies, full dose affinity measurement was performed by Bio-Layer Interferometry (BLI, ForteBio OCTET®). Briefly, the antibodies were captured with Protein A sensor at a concentration of 100 nM. Serially diluted human CDH17-his antigen solutions starting from 100 nM were associated with different antibodies for 240 s followed by dissociation for 600 s. Data was analysis by OCTET Analysis Studio 12.2.

As shown in **Table 16B,** the affinity maturated antibodies 69E3H11-z12a27 and 69E3H11-z12a42 showed improved binding affinity when compared to their parental antibody 69E3H11-z12.

***Table 16B. Full Dose Affinity Measurement Results of the Humanized Antibody***

| Affinity / Antibody | Antigen: Human CDH17 His | | |
|---|---|---|---|
| | **ka(1/Ms)** | **kd(1/s)** | **KD(M)** |
| 69E3H11-z12 | 9.15E+04 | 4.31E-04 | 4.71E-09 |
| 69E3H11-z12a27 | 1.27E+05 | 2.20E-04 | 1.73E-09 |
| 69E3H11-z12a42 | 1.74E+05 | 2.91E-04 | 1.67E-09 |

**Example 5. Internalization of the CDH17 Monoclonal Antibodies**

This example characterized the antibody internalization rate of the CDH17 antibodies when binding to CDH17 expressed on human tumor cells.

To this end, an internalization assay was developed as followed. In this case, CDH17 high pancreatic adenocarcinoma cell line AsPC-1 and CDH17 medium human colorectal cancer cell line LoVo and HCT-8 cells were used in this assay. pHAb Thiol Reactive Dyes (Promega, Cat. No. G9835) are pH-sensitive dyes those have very low fluorescence at pH>7, and a dramatic increase in fluorescence as the pH of the solution becomes acidic. pHAb Thiol Reactive Dye has a maleimide group that reacts with thiols. This maleimide group is conjugated to the antibody after the cysteine disulfide bonds in the hinge region of the antibody are reduced to thiols using a reducing agent, such as dithiothreitol (DTT) or tris (2-carboxyethyl) phosphine (TCEP). Briefly, first, a purified anti-human IgG Fc antibody (Biolegend, Cat. No.410701) were labeled with pHAb thiol Reactive Dyes under the manufacturer's instructions. Then, the resulting secondary antibody conjugated with PH-sensitive dye (50 nM) was incubated with various CDH17 antibody dilutions (20 nM) at a volume ratio of 1:1 in culture medium at RT for 30 mins to generate a 2 fold-working solution. Next, 50 μl of 2 fold-working solution was added into 96-well assay plates pre-seeded with 50 μl of $2\times10^4$ CDH17 expressing tumor cells in each well. A dramatic increase in fluorescence will occur when the antigen-antibody complex is internalized into the endosome or lysosome where the pH is around 6.3 or 4.7, respectively. The fluorescence signal was captured and analyzed by a high throughput microplate imager Operetta® CLS™ high content analysis system (PerkinElmer).

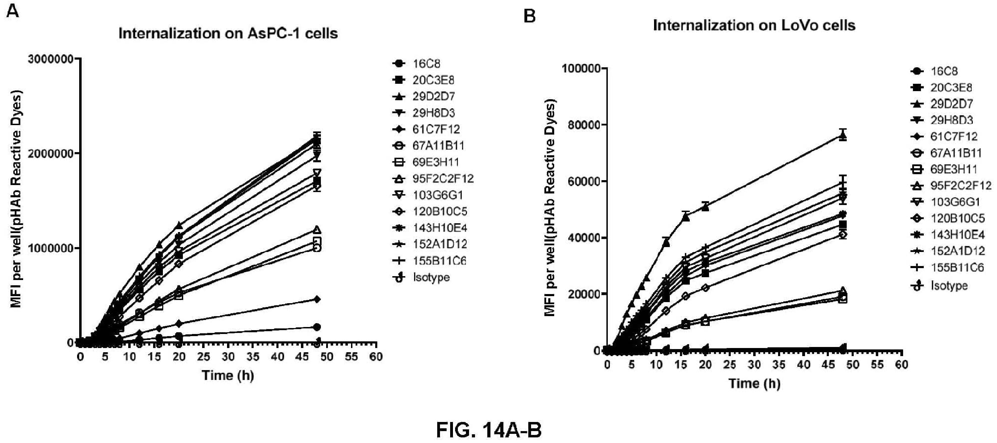

As shown in **FIG. 14A** and **14B**, the membrane-distal EC1 binders 29H8D3, 143H10E4, 152A1D12 and 155B11C6, EC2 binder 120B10C5, EC3 binder 29D2D7 and EC4 binders 20C3E8 and 103G6G1 showed efficient internalization rate on both CDH17 high AsPC-1 cells and CDH17 medium LoVo cells. On the contrary, the membrane-proximal EC6 binders 67A11B11 and EC7 binders 69E3H11 and 95F2C2F12 showed medium level of internalization in these two cell lines. EC5 binder 16C8 and EC6 binder 61C7F12 showed extremely weak or even undetectable internalization on these two CDH17-expressing tumor cells lines. In general, CDH17 membrane-distal binders displayed better performance than the membrane-proximal ones in terms of antibody internalization rate.

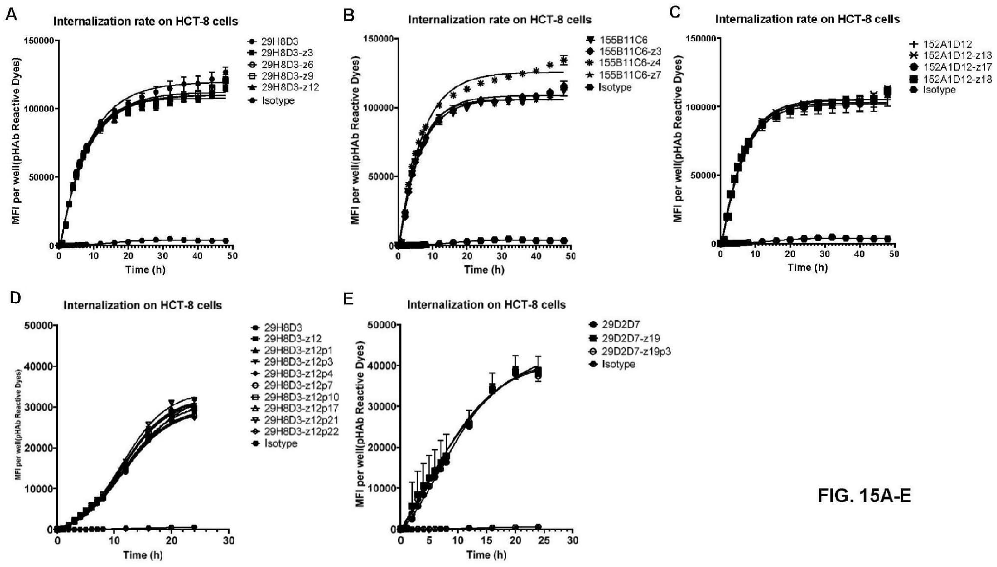

As shown in **FIG. 15A-15E**, all the CDH17 humanized and PTM removed antibodies induced efficient internalization of the antibody-antigen complex on HCT-8 cells which were comparable to their respective parental chimeric antibodies.

**Example 6. Mc-vc-PABC-MMAE-labeled Anti-human IgG Secondary Antibody Based Tumor Cell Killing of CDH17 Antibodies**

CDH17 can be used as a TAA target for ADC drugs attributed to its high expression level in GI cancers such as colorectal and pancreatic cancer. In the following examples, the potency of the CDH17 antibodies in ADC-mediated cytotoxicity were evaluated *in vitro* and *in vivo*.

In this example, the indirect cytotoxicity of CDH17 monoclonal antibodies to tumor cells mediated by a linker-payload conjugated anti-human IgG secondary antibody were characterized with established *in vitro* assay.

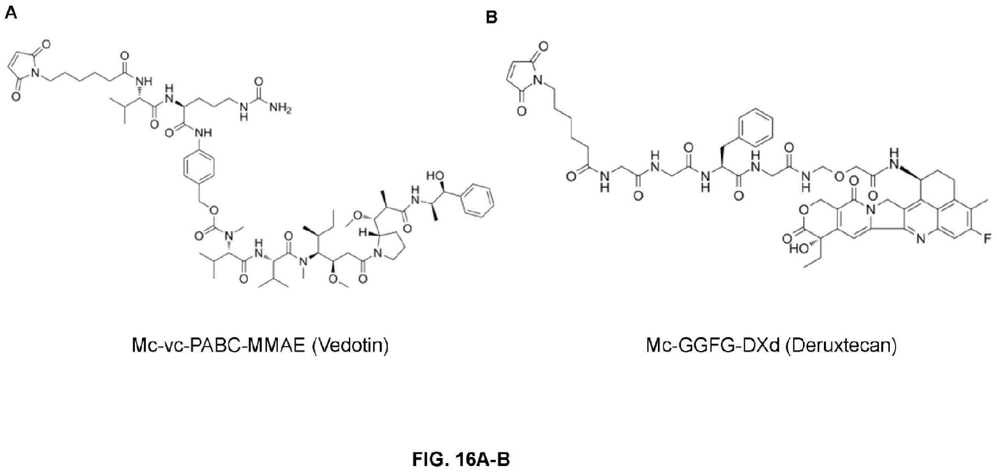

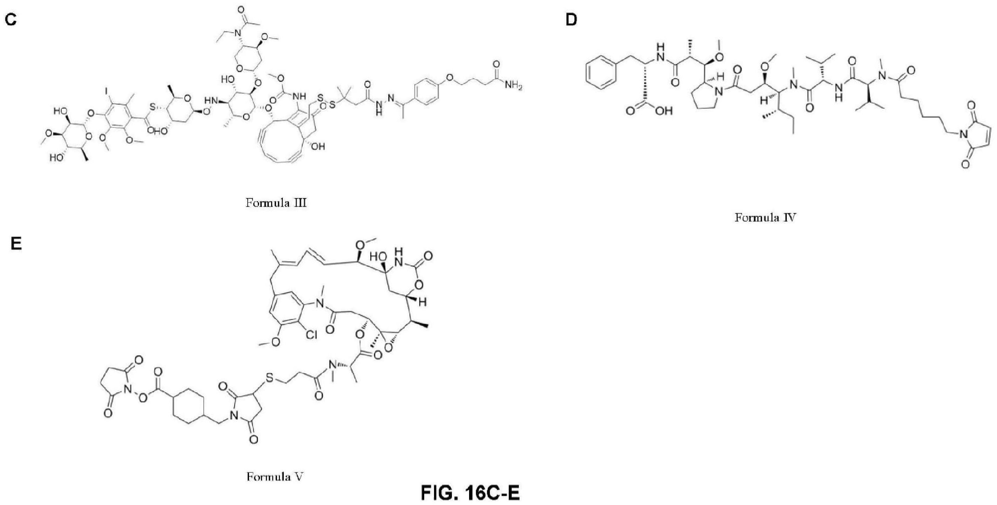

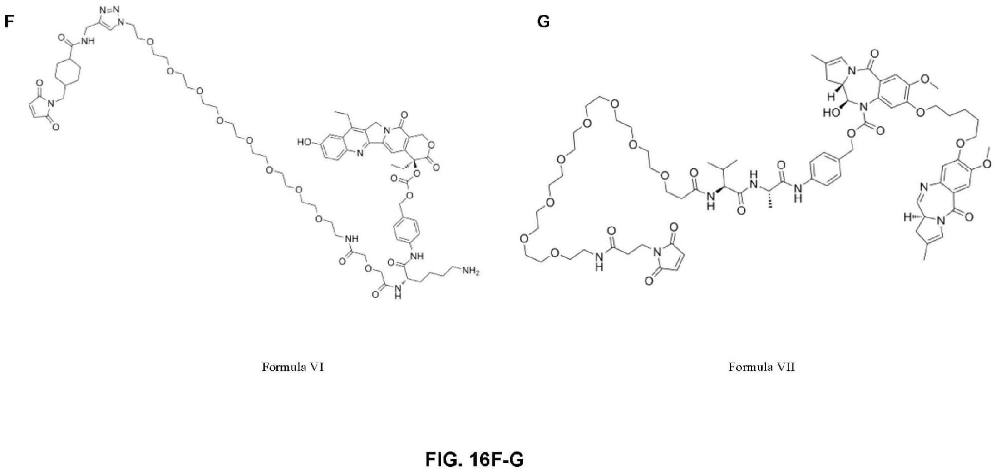

A well-established linker-payload, MC-Val-Cit-PABC-monomethyl auristatin E (hereafter referred to as mc-vc-PABC-MMAE or vc-MMAE, **FIG. 16A**) was explored as a tool linker-payload in this assay. Vc-MMAE is comprised of a thio reactive maleimidocaproyl (MC) group, a protease-sensitive Val-Cit dipeptide, a PABC linker and a MMAE payload which is the mitotic inhibitor by inhibiting microtubulin polymerization. Briefly, anti-human IgG secondary antibody (Abcam, Cat. No. ab98616) was first labeled with vc-MMAE and be used as the secondary antibody to eliminate tumor cells. The vc-MMAE labeled anti-human IgG secondary antibody (25 nM) was then incubated with CDH17 antibody dilutions (10 nM) in the culture medium at RT for 30 mins. Next, the mixture was diluted at a three-fold dilution rate and added into 96-well assay plates pre-seeded with $4\times10^3$ AGS cells in each well at a volume ratio of 1:1 followed by incubation in a $CO_2$ incubator at 37°C for 96 hours (hs). The viability of the tumor cells was detected with CellTiter-Glo® luminescent cell viability assay (100μL, Promega, Cat. No. G7573) by incubating the detection reagent with the cell culture at RT for 10 minutes followed by luminescence detection with Envision multilabel plate readers (PerkinElmer).

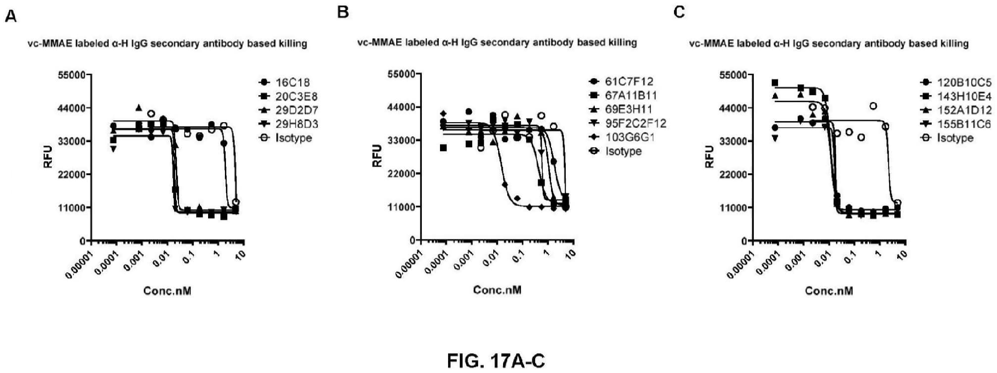

As shown in **FIG. 17A-17C**, the membrane-distal EC1 binders 29H8D3, 143H10E4, 152A1D12 and 155B11C6, EC2 binder 120B10C5, EC3 binder 29D2D7 and EC4 binders 20C3E8 and 103G6G1 showed comparable killing on AGS tumor cells, while membrane-proximal EC5 binder 16C8, EC6 binders 61C7F12 and 67A11B11 and EC7 binders 69E3H11 and 95F2C2F12 showed about 100-fold reduced tumor cell killing ability when compared to those membrane-distal region binders. This phenomenon is consistent with the better efficiency of internalization of the membrane-distal region binding CDH17 antibodies compared with the proximal binders.

**Example 7. *In vitro* Killing of vc-MMAE-labeled CDH17 ADCs**

This example characterized the cytotoxicity efficacy of vc-MMAE labeled CDH17 ADCs on CDH17 expressing

tumor cells.

In this case, tumor cell lines with different expression level of CDH17 were included in this assay. Briefly, CDH17 ADCs were prepared by conjugation of vc-MMAE linker-payload to the thiol of reduced CDH17 monoclonal antibodies through Michael reaction. After conjugation reaction and purification step, the quality control of the CDH17 ADCs was conducted to evaluate the purity, drug antibody ratio (DAR), endotoxins and free linker-payload. No obvious aggregation was observed since purity of all the ADCs determined by SEC-HPLC were over 96%. The DAR of each ADC was determined by HIC-HPLC and the calculated DAR of all the ADCs was around 3.7~ 4.0. Free-linker-payload measured by RP-HPLC was undetectable in all the CDH17 ADCs.

Then, *in vitro* ADC cytotoxicity assay was developed as following. MMAE-sensitive AsPC-1, LoVo and AGS tumor cell lines with high, medium and low level of CDH17 expression separately were used. In short, a number of $8\times10^3$ tumor cells were seeded into 96-well flat plate and cultured in a CO2 incubator at 37°C overnight. On the next day, serially diluted CDH17 ADCs starting from 30nM at a two to five-fold dilution ratio were added into the 96-well plate and incubated with the tumor cells in a $CO_2$ incubator at 37°C for 96 hs. The viability of the tumor cells was detected with CellTiter-Glo reaction regent (100μL) by incubating the detection reagent with the cell culture at RT for 10 minutes followed by luminescence detection with Envision multilabel plate readers (PerkinElemer).

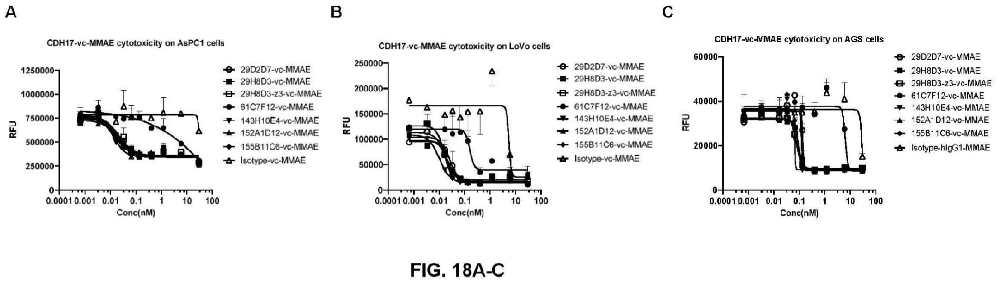

As shown in **FIG. 18A-18C**, vc-MMAE labeled CDH17 antibodies including 29D2D7, 29H8D3, 29H8D3-z3,143H10E4, 152A1D12 and 155B11C6 showed efficient cytotoxicity on all three tumor cell lines including CDH17 high AsPC-1, CDH17 medium LoVo and CDH17 low AGS cells. In contrast, 61C7F12-vc-MMAE consistently exhibited a severely reduced cytotoxicity to AsPC-1, LoVo and AGS cells, which was probably due to the inefficient internalization ability of the antibody part.

The IC50 values of the cytotoxicity of CDH17 ADCs on various tumor cells were summarized in **Table 17**.

***Table 17. Cytotoxicity Properties of vc-MMAE-labeled CDH17 ADCs to Different Cell Lines***

| IC50 (nM) / ADC | AsPC-1 | LoVo | AGS |
|---|---|---|---|
| 29D2D7-vc-MMAE | 0.019 | 0.026 | 0.250 |
| 29H8D3-vc-MMAE | 0.019 | 0.020 | 0.088 |
| 29H8D3-z3-vc-MMAE | 0.021 | 0.016 | 0.081 |
| 61C7F12-vc-MMAE | 249.6 | 0.159 | 63.10 |
| 143H10E4-vc-MMAE | 0.014 | 0.010 | 0.079 |
| 152A1D12-vc-MMAE | 0.200 | 0.019 | 0.135 |
| 155B11C6-vc-MMAE | 0.016 | 0.020 | 0.109 |

**Example 8. *In vitro* Activities of GGFG-DXd-labeled CDH17 ADCs**

This example characterized the binding, internalization and killing efficacy of GGFG-DXd labeled CDH17 ADCs on human CDH17 expressing tumor cells.

In this example, another well-validated linker-payload MC-GGFG-DXd (Deruxtecan, hereafter referred to as DXd, **FIG.16B**) was employed to evaluate the cytotoxicity efficiency of CDH17 ADCs. MC-GGFG-DXd is an ADC linker-payload conjugate composed of a maleimide-GGFG peptide linker and a DX-8951(a DNA topoisomerase I inhibitor) derivative (DXd), used for synthesizing DS-8201, a clinical proven HER2 ADCs. CDH17 ADCs were generated by conjugating MC-GGFG-DXd with CDH17 antibodies followed by quality control on purity, DAR, endotoxins and free linker-payload. No obvious aggregation was observed since purity of all the ADCs determined by SEC-HPLC were over 96%. The DAR of each ADC was determined by RP-HPLC and the calculated DAR of all the ADCs was around 7.50-7.90. Free linker-payload measured by RP-HPLC was undetectable in all the CDH17 ADCs.

***8.1 Binding of GGFG-DXd-labeled CDH17 ADC to human CDH17-expressing cells***

To confirm that the CDH17 ADCs retained that of the parental antibodies, the binding potency of GGFG-DXd labeled CDH17 ADCs and the corresponding parental antibodies was evaluated in cell-based binding assay with pancreatic cancer cell line AsPC-1, gastric cancer cell lines AGS and KATO III, and CRC cell line HT-29 cells. Among these cell lines, AsPC-1 have high level of CDH17 expression and AGS and HCT-8 have relatively medium to low level of CDH17 expression. On the contrary, KATO III and HT-29 cell lines only have marginal expression of CDH17. The assay was performed following the protocol described above.

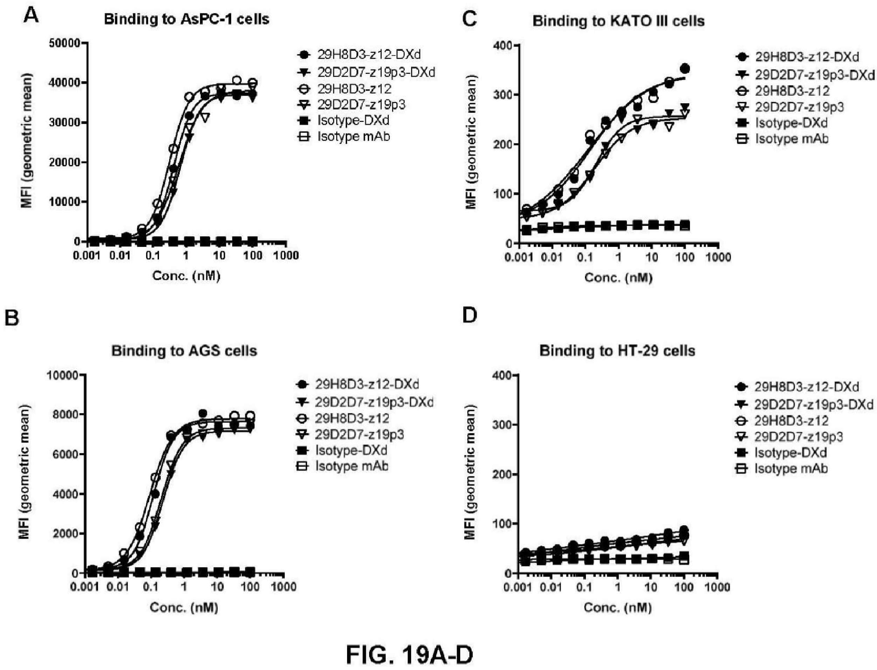

As shown in **FIG. 19A-19D**, 29H8D3-z12-DXd and 29D2D7-z19p3-DXd had similar binding potency to their corresponding parental antibodies on the indicated cells, even on KATO III and HT-29 cell lines with extremely low level of CDH17 expression. These data indicate that conjugation of these CDH17 mAbs with linker-payload did not impair their binding capabilities to the antigen.

***8.2 Internalization of GGFG-DXd-labeled CDH17 ADC to human CDH17-expressing cells***

Next, in order to examine whether CDH17 ADC could mediate efficient internalization of CDH17, the internalization rates and percentages of CDH17 ADC and the corresponding naked antibody were evaluated as follows.

The assay detecting the internalization rate was conducted following the protocol described in **Example 5**. Alternatively, the percentage of internalization was evaluated by FACS. Briefly, CDH17 ADC or mAb was incubated with $5x10^4$ CDH17-expressing tumor cells in 96-well microplates for 30 minutes at 4 °C. Then, the cell-antibody/ADC mixture was washed three times with staining buffer. The cells were resuspended with complete culture medium and divided into two parts. One part was incubated at 37 °C to allow internalization of the ADC or mAb while the other part was maintained on ice as a negative control. After 24 hours, the cell-antibody/ADC mixture was washed with staining buffer for 3 times. The antibodies or ADCs those retained on the cell surface were detected with a goat anti-human Fcγ secondary antibody conjugated with Alexa Fluor™ 647 (Jackson Immuno, Cat. 109-606-098) at a dilution rate of 1:1000 for 30 mins at 4 °C followed by extensive washing. Cells were analyzed by flow cytometer LSRFortessa™ Cell Analyzer (BD Biosciences). Data were analyzed with Flowjo 10.0 software. The graphs were generated in Graphpad Prism 9 software.

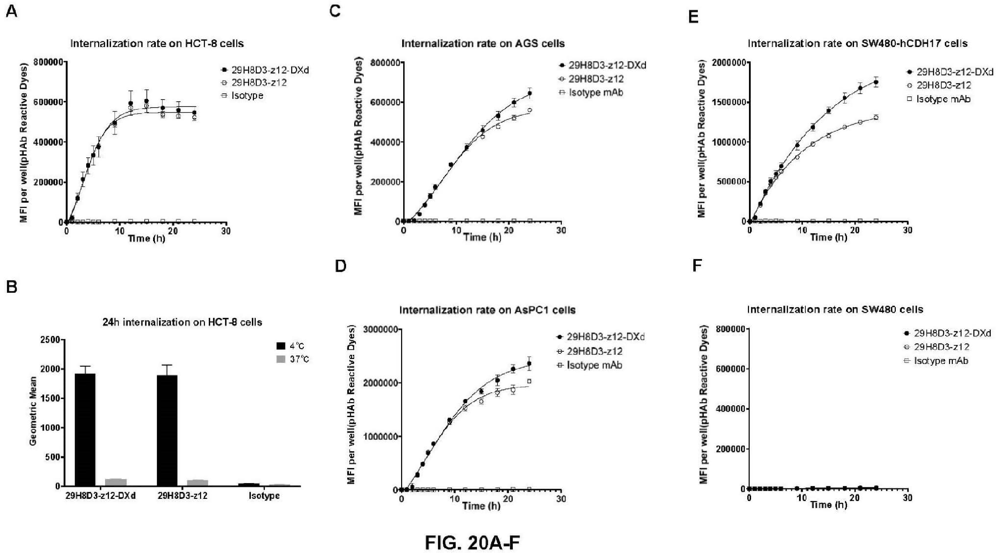

As shown in **FIG. 20A**, both 29H8D3-z12-DXd and 29H8D3-z12 mAb exhibited comparable internalization rate within 24 hours on CDH17 expressing HCT-8 cells. As shown in **FIG. 20C-FIG. 20E**, 29H8D3-z12-DXd exhibited enhanced internalization rates as compared to 29H8D3-z12 mAb within 24 hours on CDH17 expressing AGS, AsPC1 and SW480 cells with over-expression of human CDH17 (SW480-hCDH17). On the contrary, no internalization was detected in CDH17 negative wild type SW480 cells (**FIG. 20F**), indicating target-specific internalization mediated by CDH17 mAb and ADC. In terms of percentage of internalization shown in **FIG. 20B**, approximately 95% of 29H8D3-z12-DXd were internalized into the HCT-8 cells at 24 hours after treatment at 37 °C, which was comparable to the naked mAb 29H8D3-z12. These data collectively demonstrate a fast and efficient internalization of CDH17 ADCs when targeting CDH17 expressing tumor cells and this capability is not affected by the ADC conjugation procedure.

***8.3 In vitro killing efficacy of GGFG-DXd-labeled CDH17 ADCs***

*In vitro* ADC cytotoxicity assay was developed as follows. DXd-sensitive SK-CO-1, HCT-8, AGS, LS1034 and Caco2 cells with high and medium expression level of CDH17 respectively was selected as the target cells. Another three DXd-sensitive cell lines including breast cancer cell line MDA-MB-468, CRC cell lines SW620 and SW480 either lack the native expression of CDH17 or only have neglectable expression level of CDH17. In order to evaluate the on-target effect of CDH17 ADCs, these three tumor cells were enforced to over-express full length human CDH17 and also used as the target cells.

The absolute antigen number was evaluated with QIFIT® cell surface antigen quantification Kit (Quantitative Analysis Kit, Agilent Dako, Code K0078) following the manufacture's instruction**.** Briefly, the CDH17 mAb 69E3H11 with mouse IgG2a constant region or a mouse IgG2a isotype control was diluted at a concentration of 50 nM in the staining buffer (DPBS buffer containing 2% FBS). The antibody dilutions were incubated with $5x10^4$ cells in 96-well microplates for 30 minutes (mins) at 4 °C. The calibration bead conjugated with high-affinity anti-human CD5 mouse IgG2a antibody (Clone CRIS-1) with a range of absolute numbers served as standards of cell surface antigen quantification. Then the cell-antibody mixture and calibration beads were washed with staining buffer twice. The antibodies binding to the antigen on the cell surface or the calibration beads were detected with goat anti-mouse IgG (H+L) secondary antibody conjugated with Alexa Fluor™ 647 (Jackson ImmunoResearch, Cat. No. 115-606-003) at a dilution rate of 1:2000 for 30 mins at 4 °C followed by extensive washing. Cells and calibration beads were analyzed by flow cytometer LSRFortessa™ Cell Analyzer (BD Biosciences). Data were analyzed with Flowjo 10.0 software.

The results showed that SK-CO-1, LS1034, MDA-MB-468-hCDH17, SW480-hCDH17 and SW620-hCDH17 had high absolute number of membrane CDH17 ($>1\times10^5$/cell) and HCT-8, AGS and Caco2 showed medium expression level of CDH17 ($1\times10^4$/cell ~$1\times10^5$/cell) (**Table 18b**).

For *in vitro* killing assay, briefly, a number of $2\times10^3$ to $8\times10^3$ tumor cells were seeded into 96-well flat plate and cultured in a $CO_2$ incubator at 37°C overnight. On the next day, serially diluted CDH17 ADCs starting from 30 nM or 50 nM at a three-fold dilution ratio were added into the 96-well plate and incubated with the tumor cells in a $CO_2$ incubator at 37 °C for 5 to 6 days. The viability of the tumor cells was detected with CellTiter-Glo reaction regent (100μL) by incubating the detection reagent with the cell culture at RT for 10 minutes followed by luminescence detection with Envision multilabel plate readers (PerkinElemer).

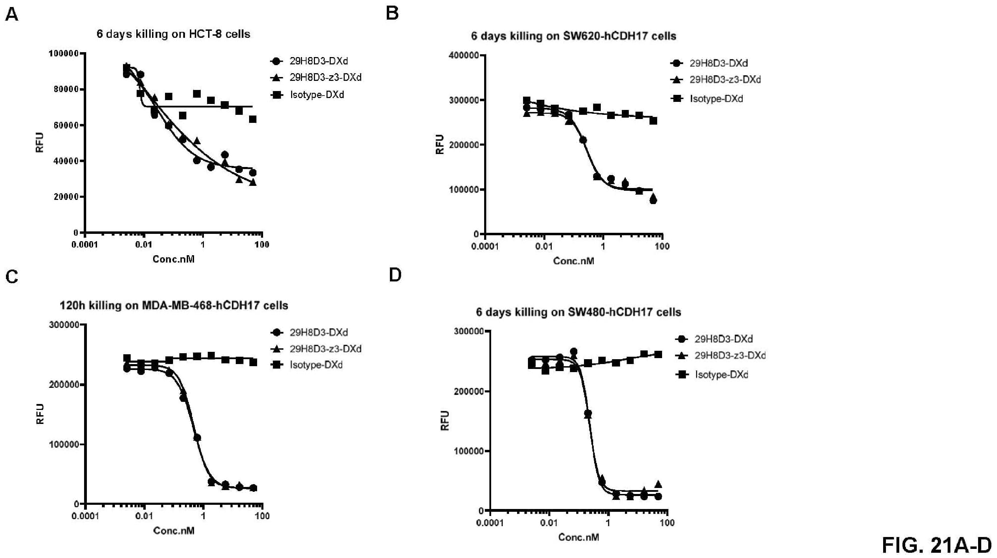

As shown in **FIG. 21A -FIG. 21D**, both 29H8D3-DXd (29H8D3-MC-GGFG-DXd) and 29H8D3-z3-DXd (29H8D3-

z3-MC-GGFG-DXd) exhibited potent and comparable tumor cell killing on HCT-8 and three human CDH17-overexpressing SW620, SW480 and MDA-MB-468 tumor cells, indicating the cytotoxicity efficiency of the CDH17 ADC on CDH17-expressing tumor cells.

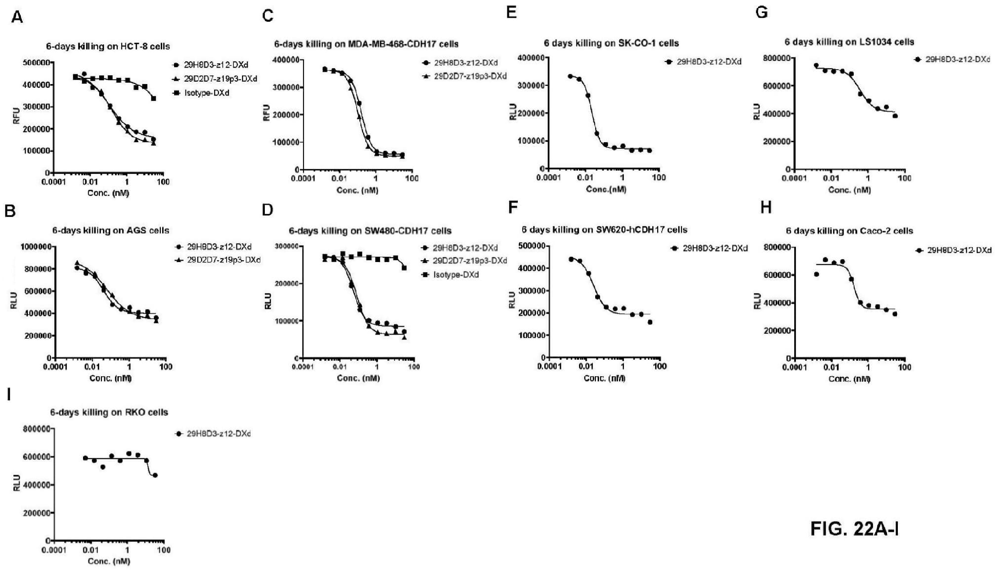

As shown in **FIG. 22A-FIG. 22D**, both 29H8D3-z12-DXd (29H8D3-z12-MC-GGFG-DXd) and 29D2D7-z19p3-DXd (29D2D7-z19-MC-GGFG-DXd) exhibited potent and comparable tumor cell killing on CDH17-expressing high SW480-hCDH17 and MDA-MB-468-hCDH17 and CDH17-expressing medium HCT-8 and AGS cells, indicating the cytotoxicity efficiency of the CDH17 ADC on CDH17-expressing high and medium tumor cells. Moreover, 29H8D3-z12-DXd also displayed dose-dependent cytotoxicity on CDH17-expressing high SK-CO-1, LD1034 and SW620-hCDH17 as well as CDH17-expressing medium Caco2 cells (**FIG. 22E -FIG. 22H**). On the contrary, no obvious cytotoxicity on CDH17 negative RKO cells was observed (**FIG. 22I**), indicating target-specific cytotoxicity mediated by CDH17 ADC.

The IC50 values of the cytotoxicity of CDH17 ADCs on various tumor cells were summarized in **Table 18A** and **Table 18B**.

***Table 18A. Cytotoxicity Properties of DXd-labeled CDH17 ADCs to Different Cell Lines***

| IC50 (nM) / ADC | HCT-8 | SW620 -hCDH17 | MDA-MB-468 -hCDH17 | SW480 -hCDH17 |
|---|---|---|---|---|
| 29H8D3-DXd | 0.041 | 0.290 | 0.469 | 0.240 |
| 29H8D3-z3-DXd | 0.083 | 0.290 | 0.476 | 0.240 |

***Table 18B. Cytotoxicity Properties of DXd-labeled CDH17 ADCs to Different Cell Lines***

| IC50(nM) / ADC | HCT-8 | AGS | MDA-MB-468-hCDH17 | SW480 -hCDH17 | SK-CO-1 | SW620-hCDH17 | LS1034 | Caco2 |
|---|---|---|---|---|---|---|---|---|
| CDH17 absolute number | $5.42\times10^4$ | $4.98\times10^4$ | $5.28\times10^5$ | $1.89\times10^5$ | $1.47\times10^5$ | $1.02\times10^5$ | $1.33\times10^5$ | $9.71\times10^4$ |
| 29H8D3-z12-DXd | 0.107 | 0.041 | 0.158 | 0.053 | 0.022 | 0.025 | 0.37 | 0.17 |
| 29D2D7-z19p3-DXd | 0.155 | 0.063 | 0.104 | 0.082 | / | / | / | / |

### *8.4 In vitro bystander killing efficacy of GGFG-DXd-labeled CDH17 ADCs*

In order to evaluate the bystander killing effect of CDH17-ADCs, an *in vitro* coculture assay was performed as follows. GFP-positive CDH17-expressing SW480-hCDH17 cells (1000 cells/well) were cocultured with human CDH17 negative RKO ($RFP^+$), SW480 or MDA-MB-468 cells (2000 cells/well) in the presence of CDH17 ADCs at a concentration of 1 nM or medium control in a 96-well plate. hCDH17-negative cells seeded with the same cell number (2000 cells/well) in the absence of hCDH17-positive cells was served as the negative control to rule out the direct killing of CDH17 ADCs on human CDH17 negative cells. After 6 days, the absolute numbers of GFP-positive cells and GFP-negative cells were counted with Operetta® CLS™ high content analysis system (PerkinElmer). Cell viability was analysis and graphed by Graphpad Prism 9 software.

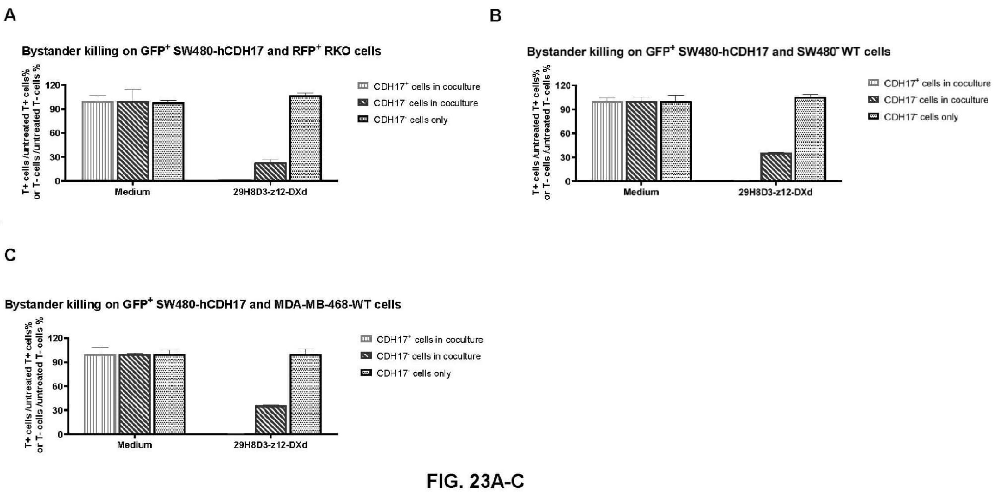

As shown is **FIG. 23A -FIG. 23C**, 29H8D3-z12-DXd showed efficient cytotoxicity to both CDH17 positive SW480-hCDH17 cells and CDH17 negative RKO, SW480 and MDA-MB-468 cells in the coculture systems. In contrast, 29H8D3-z12-DXd did not show any killing effect on CDH17 negative RKO, SW480 and MDA-MB-468 cells in the absence of CDH17 positive SW480-hCDH17 cells in the culture system (**FIG. 23A** - **23C)**. These data collectively demonstrated efficient bystander killing effect of CDH17 ADC *in vitro*.

## Example 9. *In vivo* Anti-tumor Efficacy of vc-MMAE-labeled CDH17 ADCs

This example characterized the *in vivo* anti-tumor activity of vc-MMAE labeled CDH17 ADCs on cell line derived xenograft (CDX) mice models.

To this end, MMAE-sensitive colorectal cell line LoVo cells ($5\times10^6$) were implanted subcutaneously into BALB/c Nude mice. When the tumor volume (TV) reached around 180 $mm^3$, mice were grouped (n=6 mice/group) and simultaneously administered with a single dose of 3 mg/kg indicated CDH17 ADCs intravenously, with the isotype-vc-MMAE and vehicle group as the control. Tumor volume defined as $0.5\times$ length $\times$ $width^2$ was measured three times a week. TGI (%) was calculated as follows: TGI (%) = [1-(mean of tumor volume change of treatment group on evaluation

day)/(mean of tumor volume change of control group on evaluation day)] × 100.

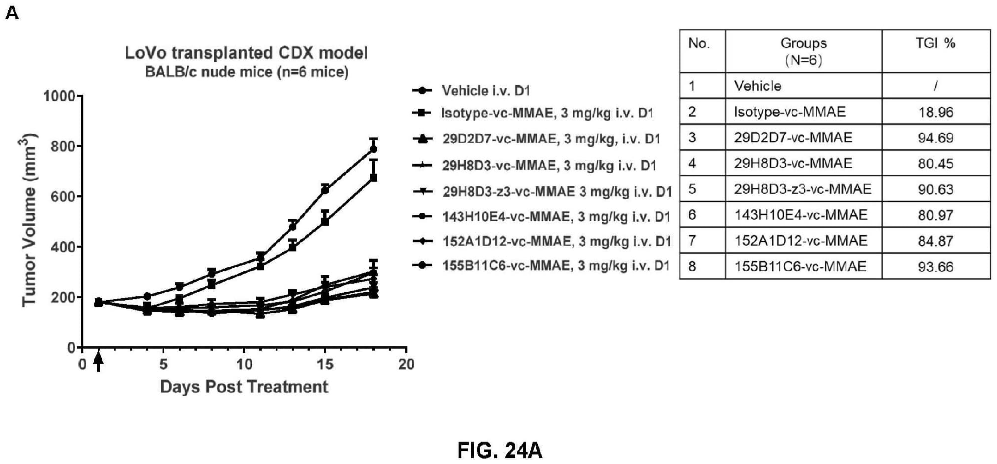

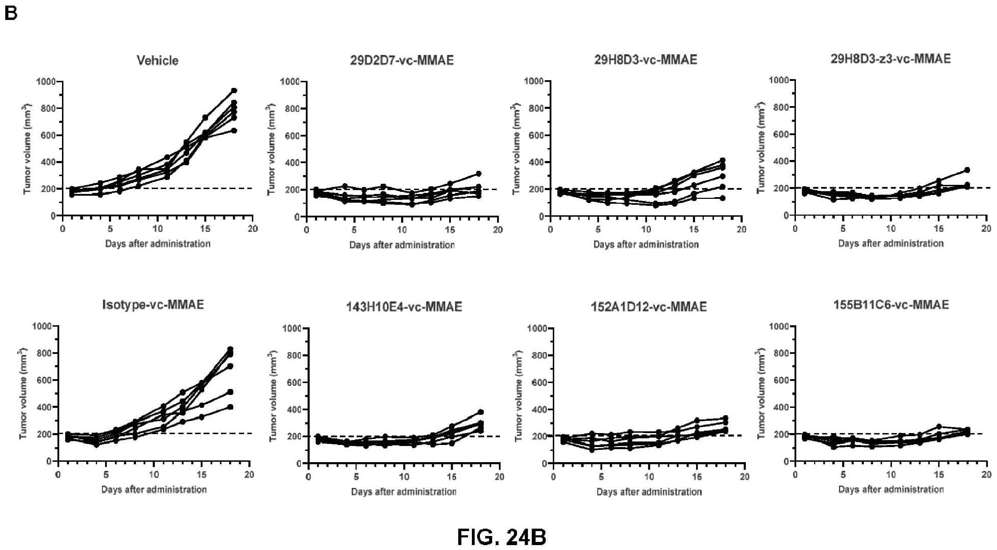

As shown in **FIG. 24A** and **24B**, all the CDH17 ADCs with vc-MMAE linker-payload exhibited robust tumor growth inhibition with the tumor growth index (TGI) ranging from 85-95% at day 18 after treatment. In contrast, non-binding MMAE showed extremely weak and transient tumor growth inhibition (TGI=18.96%) at the end of the study. These data collectively demonstrated the anti-tumor efficiency of vc-MMAE based CDH17 ADCs in *in vivo* CDX mice model.

**Example 10. *In vivo* Anti-tumor Efficacy of GGFG-DXd-labeled CDH17 ADCs in CDX Mice Models**

This example characterized the *in vivo* anti-tumor activity of GGFG-DXd conjugated CDH17 ADCs in CDX mice models.

To this end, colorectal cell line LS1034 cells ($5\times10^6$) with high expression level of CDH17 ($1.33\times10^5$/cell) were resuspended in DPBS and mixed with Matrigel (Corning®, Cat. No. 356234) at 1:1 ratio followed by implantation subcutaneously into CB-17 SCID female mice. When the tumor volume (TV) reached around 150 $mm^3$, mice were grouped (n=4 mice/group) and simultaneously administered with two doses of 3 mg/kg or 10 mg/kg 29H8D3-z12-DXd intravenously at day 1 and day 8, with the vehicle group as the non-treatment control. Tumor volume defined as 0.5× length × $width^2$ was measured three times a week. TGI (%) was calculated as follows: TGI (%) = [1- (mean of tumor volume change of treatment group on evaluation day)/(mean of tumor volume change of control group on evaluation day)] × 100.

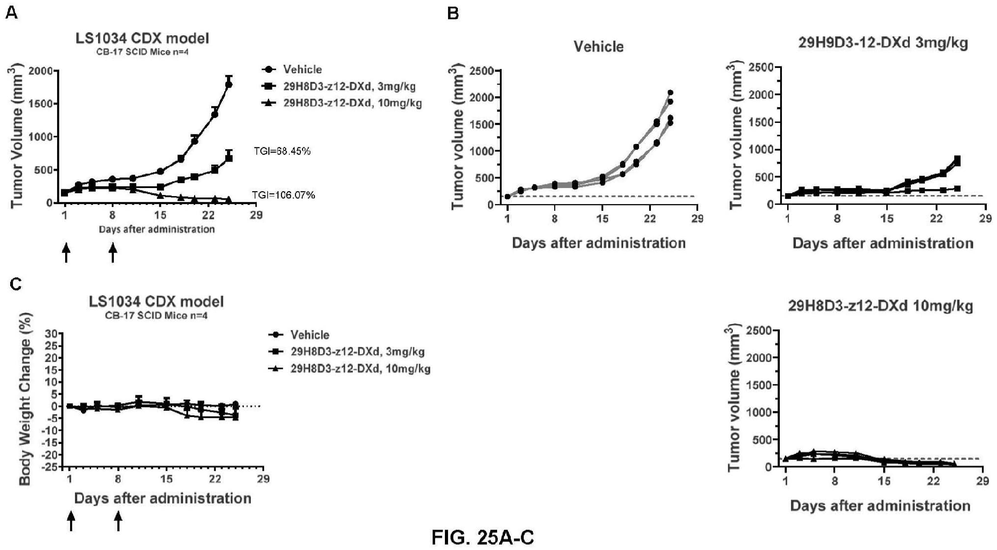

As shown in **FIG. 25A** and **25B**, 29H8D3-z12-DXd exhibited robust and dose-dependent tumor growth inhibition with the tumor growth index (TGI) at 68.45% for 3 mg/kg and 106.07 % for 10 mg/kg at day 25 after treatment compared with vehicle group. Moreover, no body weight change was observed in all the treatment groups until the end of the study (**FIG. 25C**), indicating no systemic toxicity mediated by the CDH17 ADC treatment. These data collectively demonstrated the excellent dose-dependent anti-tumor efficacy of GGFG-DXd conjugated CDH17 ADCs in *in vivo* CDX mice model.

**Example 11. *In vivo* Anti-tumor Efficacy of GGFG-DXd-labeled CDH17 ADCs in Patient-derived Xenograft (PDX) Mice Models**

This example characterized the *in vivo* anti-tumor activity of GGFG-DXd conjugated CDH17 ADCs in patient-derived xenograft (PDX) mice models.

To this end, a panel of CRC PDX mice models with different driver gene mutation including *TP53* and *KRAS* or gene amplification (*ERBB2*) were selected for *in vivo* efficacy evaluation. Of note, one of these models (model 1) showed resistance to SOC treatment (combination of Irinotecan, Calcium Folinate and 5-fluorouracil) in previous *in vivo* efficacy study (data not shown). Another model (model 3) was reported resistant to multiple target therapies and chemotherapies including irinotecan during the clinical treatment. It is of importance to evaluate whether CDH17-GGFG-DXd ADC could still exhibit anti-tumor effect in these tumor model with driver-gene mutations and chemo-resistance.

The CDH17 expression levels of these PDX tumor tissues derived from CRC patients were confirmed by immunohistochemistry (IHC) staining with formalin-fixed, paraffin-embedded (FFPE) sections using a specific CDH17 mAb (Abcam, Cat. No. ab183318) as the primary antibody with rabbit IgG mAb (Abcam, Cat. No. ab172730) served as the isotype control. Histochemical scoring system (H-score) was used for evaluating the intensity and extent of CDH17 expression. The expression grades were classified as 0 (H-score < 1), 1+ (H-score 1-99), 2+ (H-score 100-199) and 3+ (H-score 200-300). PDX tumor samples with high (3+) or medium (2+) expression level of CDH17 were selected and passaged in the host mice. When tumors grew up to about 500~800 $mm^3$, the tumor samples were cut into small pieces (about 3 $mm^3$, 45~60 mg) and inoculated into female NU/NU Nude mice (Vital River) subcutaneously. When the tumor had grown to an appropriate volume, the tumor-bearing mice were randomized into treatment and control groups (n=4 mice per group) with the average tumor volume at around 200 $mm^3$. Simultaneously, a single dose, two doses or three doses of 10 mg/kg CDH17 ADCs as indicated was administered intravenously, with the vehicle treatment as the negative control. Tumor volume defined as 0.5× length × $width^2$ was measured twice a week. TGI (%) was calculated as follows: TGI (%) = [1- (mean of tumor volume change of treatment group on evaluation day) / (mean of tumor volume change of control group on evaluation day)] × 100. Relative change of body weight (%) (RCBW) was calculated as follows: RCBW(%) = $(BW_i - BW_0)/BW_0\times100$, $BW_i$ is the average body weight on the evaluation day and $BW_0$ is the average body weight at the starting dose.

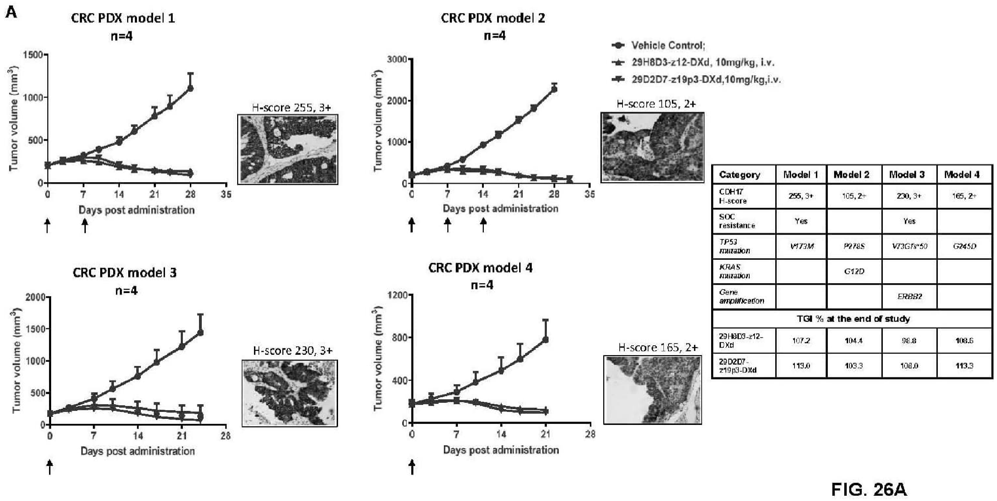

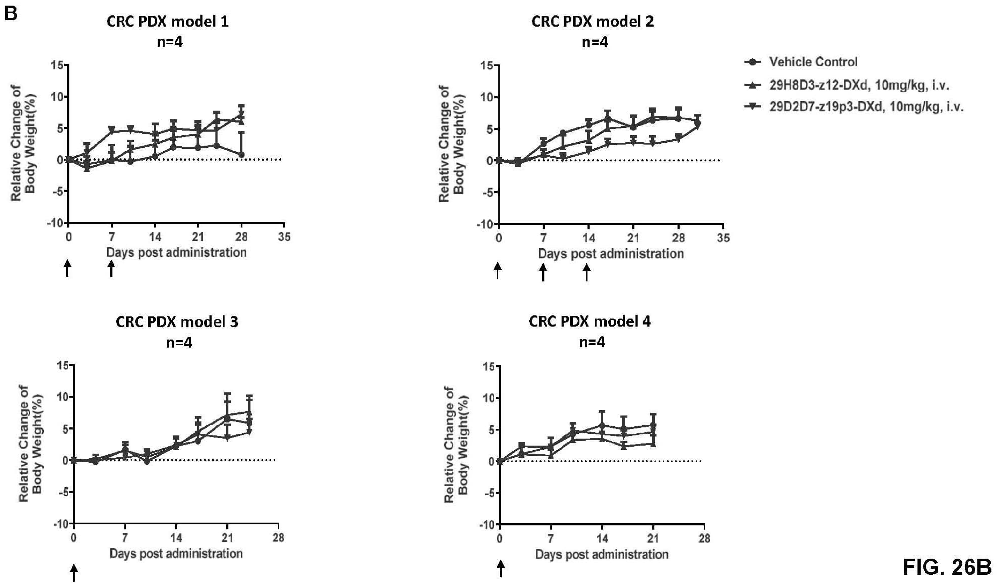

As shown in **FIG. 26A**, the CDH17 ADCs conjugated with GGFG-DXd linker-payload exhibited robust tumor growth inhibition or even regression in all the four CRC PDX models with a varying range of CDH17 expression level and different dose regimens. The tumor growth index (TGI) of the treatment groups ranged from 99% to 113% at the end of the study (**FIG. 26A**). Of note, this significant anti-tumor efficacy of CDH17 ADC was not only observed in SOC resistant PDX models, but also in PDX models with driver-gene mutation or amplification, indicating that the efficacy of CDH17-ADC is independent to the background gene mutation status or previous response to chemotherapeutic drugs even like irinotecan which is also Camptothecin derivatives, similar to DXd. In addition, no body weight loss was observed during the treatment (**FIG. 26B**), indicating no obvious toxicities of these ADCs. These data collectively demonstrated the robust anti-tumor

efficiency of CDH17-GGFG-DXd ADCs in CRC PDX mice model.

Next, in order to understand whether CDH17-GGFG-DXd ADC could efficiently and dose-dependently inhibit tumor growth with a single dose regimen in PDX mice models, three out of four PDX mice models shown in **FIG. 26** were selected for further *in vivo* efficacy study.

The study followed the protocols as described before. When tumors grew up to about 500~800 $mm^3$, the tumor samples were cut into small pieces (about 3 $mm^3$, 45~60 mg) and inoculated into female NU/NU Nude mice (Vital River) subcutaneously. When the tumors had grown to an appropriate volume, the tumor-bearing mice were randomized into treatment and control groups (n=4 mice per group) with the average tumor volume at around 180~200 $mm^3$. Simultaneously, a single dose of 3 mg/kg or 10 mg/kg 29H8D3-z12-DXd was administered intravenously, with the vehicle treatment as the negative control. Tumor volume defined as $0.5\times$ length $\times$ $width^2$ was measured twice a week. TGI (%) was calculated as follows: TGI (%) = [1-(mean of tumor volume change of treatment group on evaluation day)/(mean of tumor volume change of control group on evaluation day)] $\times$ 100.

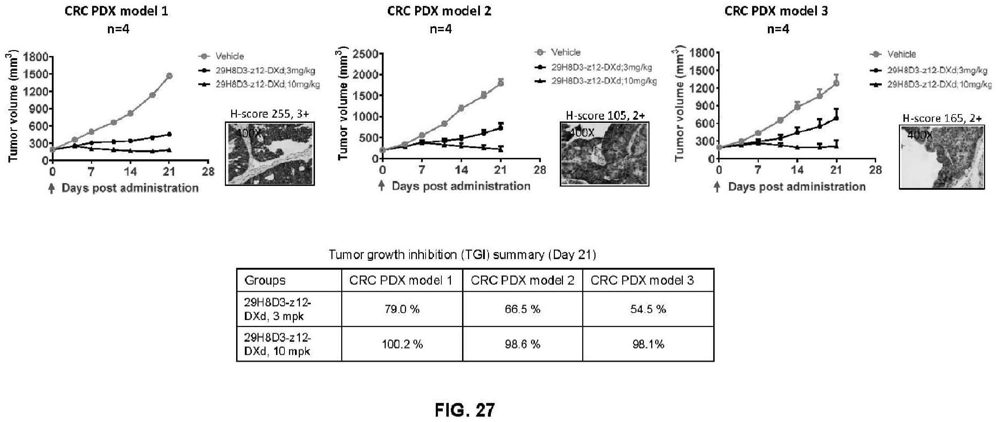

As shown in **FIG. 27**, a single dose of 29H8D3-z12-DXd exhibited robust and dose-dependent tumor growth inhibition in all three CRC PDX mice models with a varying range of CDH17 expression level. A single dose of 3 mg/kg 29H8D3-z12-DXd achieved tumor growth inhibition with the TGI of the treatment groups ranging from 54.5 % to 79.0 % at day 21. A single dose of 10 mg/kg 29H8D3-z12-DXd achieved even enhanced tumor growth inhibition with the TGI of the treatment groups ranging from 98.1 % to 100.2 % at day 21. These data collectively demonstrated efficient and dose-dependent tumor growth inhibition of 29H8D3-z12-DXd ADCs in CRC PDX mice models.

The present disclosure is not to be limited in scope by the specific embodiments described which are intended as single illustrations of individual aspects of the disclosure, and any compositions or methods which are functionally equivalent are within the scope of this disclosure. It will be apparent to those skilled in the art that various modifications and variations can be made in the methods and compositions of the present disclosure without departing from the spirit or scope of the disclosure. Thus, it is intended that the present disclosure cover the modifications and variations of this disclosure provided they come within the scope of the appended claims and their equivalents.

All publications and patent applications mentioned in this specification are herein incorporated by reference to the same extent as if each individual publication or patent application was specifically and individually indicated to be incorporated by reference.

## Claims

1. An antibody or antigen-binding fragment thereof having specificity to a human Cadherin 17 (CDH17) protein, wherein the antibody or antigen-binding fragment thereof comprises a heavy chain variable region (VH) comprising heavy chain complementarity determining regions HCDR1, HCDR2, and HCDR3, and a light chain variable region (VL) comprising light chain complementarity determining regions LCDR1, LCDR2, and LCDR3, wherein the HCDR1, HCDR2, HCDR3, LCDR1, LCDR2, and LCDR3, respectively, comprise the amino acid sequences
SEQ ID NO:49, 50, 51, any one of SEQ ID NO:52 and 239-241, any one of SEQ ID NO:53 and 242-244, and SEQ ID NO:54.

2. The antibody or antigen-binding fragment thereof of claim 1, wherein the HCDR1, HCDR2, HCDR3, LCDR1, LCDR2, or LCDR3 comprises post-translational modification (PTM) site removal, wherein the PTM site comprises one or more of oxidation site, unpaired cysteine residue site, deamidation site, glycation site, disulfide formation site, N-terminal pyroglutamate formation site, and high mannose glycosylation site, optionally wherein

   (a) the HCDR1 comprises the amino acid sequence of SEQ ID NO:49, the HCDR2 comprises the amino acid sequence of SEQ ID NO:50, the HCDR3 comprise the amino acid sequence of SEQ ID NO:51, the LCDR1 comprises an amino acid sequence selected from the group consisting of SEQ ID NO:239-241, the LCDR2 comprises an amino acid sequence selected from the group consisting of SEQ ID NO:53 and 242-244, and the LCDR3 comprises the amino acid sequence of SEQ ID NO:54; or
   (b) the HCDR1 comprises the amino acid sequence of SEQ ID NO:49, the HCDR2 comprises the amino acid sequence of SEQ ID NO:50, the HCDR3 comprise the amino acid sequence of SEQ ID NO:51, the LCDR1 comprises an amino acid sequence selected from the group consisting of SEQ ID NO:52 and 239-241, the LCDR2 comprises an amino acid sequence selected from the group consisting of SEQ ID NO:242-244, and the LCDR3 comprises the amino acid sequence of SEQ ID NO:54.

3. The antibody or antigen-binding fragment thereof of any one of claims 1-2, wherein the antibody is a chimeric antibody or a humanized antibody.

4. The antibody or antigen-binding fragment thereof of claim 1, wherein: the VH comprises an amino acid sequence selected from the group consisting of SEQ ID NO: 55, 149-152, and 154, and the VL comprises an amino acid sequence selected from the group consisting of SEQ ID NO: 56, 156-158, 160, and 232-238, optionally wherein the VH and VL, respectively, comprise the amino acid sequences of (a) SEQ ID NO: 149 and 156; (b) SEQ ID NO: 149 and 157; (c) SEQ ID NO: 149 and 158; (d) SEQ ID NO: 150 and 156; (e) SEQ ID NO: 150 and 157; (f) SEQ ID NO: 150 and 158; (g) SEQ ID NO: 151 and 156; (h) SEQ ID NO: 151 and 157; (i) SEQ ID NO: 151 and 158; (j) SEQ ID NO: 152 and 156; (k) SEQ ID NO: 149 and 160; (l) SEQ ID NO: 152 and 160; (m) SEQ ID NO: 154 and 156; (n) SEQ ID NO: 154 and 160; (o) SEQ ID NO:152 and 232; (p) SEQ ID NO:152 and 233; (q) SEQ ID NO:152 and 234; (r) SEQ ID NO:152 and 235; (s) SEQ ID NO:152 and 236; (t) SEQ ID NO:152 and 237 or (u) SEQ ID NO:152 and 238.

5. An antibody or antigen-binding fragment thereof having specificity to a human CDH17 protein, wherein

(i) the antibody or antigen-binding fragment thereof comprises a heavy chain variable region comprising heavy chain complementarity determining regions HCDR1, HCDR2, and HCDR3, and a light chain variable region comprising light chain complementarity determining regions LCDR1, LCDR2, and LCDR3,
wherein:

the set of HCDR1, HCDR2, and HCDR3 is selected from antibody 69E3H11 in Table 2A, or CDR sets derived from antibody 69E3H11 in Table 2A with one, two, or three amino acid addition, deletion and/or substitution in one or more of the CDRs, and
the set of LCDR1, LCDR2, and LCDR3 are selected from antibody 69E3H11 in Table 2B or CDR sets derived from antibody 69E3H11 in Table 2B with one, two, or three amino acid addition, deletion and/or substitution in one or more of the CDRs, or

(ii) the antibody or antigen-binding fragment thereof compete with the antibody or antigen-binding fragment thereof of any one of the preceding claims.

6. The antibody or antigen-binding fragment thereof of any one of the preceding claims, wherein

(i) the antibody or an antigen-binding fragment thereof is selected from the group consisting of a full length antibody, Fab, Fab', F(ab')2, Fd, Fvs, single-chain Fvs (scFv), single-chain antibodies, disulfide-linked Fvs (sdFv), a nanobody, a domain antibody, an isolated CDR and a bivalent domain antibody;
(ii) the antibody or an antigen-binding fragment thereof
further compresses a heavy chain constant region, a light chain constant region, an Fc region, or the combination thereof, optionally wherein the light chain constant region is a kappa or lambda chain constant region; and/or
(iii) the antibody or antigen-binding fragment thereof is of an isotype of IgG, IgM, IgA, IgE or IgD, optionally wherein the isotype is IgG1, IgG2, IgG3 or IgG4.

7. An antibody-drug conjugate comprising the antibody or antigen-binding fragment thereof of any one of claims 1-6, and a drug which is optionally connected to the antibody or antigen-binding fragment thereof through a linker, optionally wherein

(i) the linker is connected to the antibody or antigen-binding fragment thereof of any one of claims 1-6 via chemical conjugation or enzymatic conjugation;
(ii) the linker is a cleavable linker, or a non-cleavable linker, optionally wherein the cleavable linker is a chemically sensitive linker or enzyme-cleavable linker; wherein the non-cleavable linker comprises thioether or maleimidecaproyl (MC), more optionally wherein the chemically sensitive linker is a pH-sensitive linker or glutathione-sensitive disulfide linker; wherein the enzyme-cleavable linker is a peptide based linker or β-glucuronide linker; or
(iii) the linker comprises succinimidyl-4-(N-maleimidomethyl) cyclohexane-1-carboxylate (SMCC), sulfo-SMCC, p-carboxycyclo hexylmethylmaleimide, maleimide-caproyl (MC)-Valine-citrulline (VC)- para-aminobenzyloxy-carbamoyl (PABC), CL2A, maleimide-caproyl (MC)-glycine-glycine-phenylalanine-glycine (GGFG), MC, or maleimide propoyl (MP)-PEG8-Valine-alanine (VA)-PABC.

8. The antibody-drug conjugate of any one of claim 7, wherein the drug is selected from the group consisting of a cytotoxin, a therapeutic peptide and polypeptide, optionally wherein the drug is selected from the group consisting of auristatins, maytansinoids, benzodiazepines, tubulysins, duocarmycin, camptothecin, calicheamicins, exatecans, irinotecans (SN38), doxorubicin, anthracycline, the pyrrolobenzodiazepenes (PBD), TLR agonist, STING agonists, pseudomonas aeruginosa exotoxin PE38, diphtheria toxin, staphylococcus aureus enterotoxin A/E-120, antibacterial

antibiotic, shigatoxin, ricin, and urease, more optionally wherein the drug is monomethyl auristatin E (MMAE), monomethyl auristatin F (MMAF), maytansine, mertansine (DM1), ravtansine (DM4), tublysin A, DX-8951f, DXd, 7-ethyl-10-hydroxycamptothecin (SN-38), DGN462, Amberstatin269, anthramycin, SG3199 / SCX, IRDye®700DX, TLR7/8 agonist, diABZI STING agonist-2, or any derivative thereof.

**9.** The antibody-drug conjugate of any one of claims 7-8, wherein the drug and the linker, collectively, is ozogamicin, vedotin, mafodotin, emtansine, soravtansine, ravtansine, mertansine, deruxtecan, govitecan, or tesirine, optionally wherein the drug and linker, collectively, comprise (a) vedotin having Formula I:

(Mc-vc-PABC-MMAE);

Formula I

(b) deruxtecan having Formula II:

(Mc-GGFG-DXd)

Formula II

(c) ozogamicin having Formula III:

Formula III

;

(d) mafodotin having Formula IV:

Formula IV

;

(e) emtansine having Formula V:

Formula V

;

(f) govitecan having Formula VI:

Formula VI ;

or

(g) tesirine having Formula VII:

Formula VII .

**10.** A composition comprising the antibody or antigen-binding fragment thereof of any one of claims 1-6 or the antibody-drug conjugate of any one of claims 7-9, and a pharmaceutically acceptable carrier.

**11.** An isolated cell comprising one or more polynucleotide encoding the antibody or antigen-binding fragment thereof of any one of claims 1-6.

**12.** A polynucleotide encoding one or more chains of the antibody or antigen-binding fragment thereof of any one of claims 1-6.

**13.** The antibody or antigen-binding fragment thereof of any one of claims 1-6, the antibody-drug conjugate of any one of claims 7-9, or the composition of claim 10 for use in the treatment of cancer in a patient in need thereof.

**14.** The antibody or antigen-binding fragment thereof, the antibody-drug conjugate, or the composition of claim for use of claim 13, wherein the cancer is selected from the group consisting of bladder cancer, breast cancer, colorectal cancer, endometrial cancer, esophageal cancer, head and neck cancer, kidney cancer, leukemia, liver cancer, lung cancer, lymphoma, melanoma, pancreatic cancer, prostate cancer, and thyroid cancer, and/or
wherein the treatment further comprises administering to the patient an additional therapy for treating said cancer, optionally wherein said additional therapy is an immunotherapy, a chemotherapy or a radiotherapy.

**15.** A method of detecting expression of CDH17 in a sample, comprising contacting the sample with the antibody or antigen-binding fragment thereof of any one of claims 1-6 under conditions for the antibody or antigen-binding fragment thereof to bind to the CDH17, and detecting the binding which indicates expression of CDH17 in the sample.

A
Binding to hCDH17-his protein
OD450
0
1
2
3
0.001
0.01
0.1
1
10
100
1000
Conc.nM
20C3E8
29D2D7
29H8D3
120B10C5
143H10E4
152A1D12
155B11C6
Isotype
B
Binding to hCDH17-his protein
OD450
0
1
2
3
0.001
0.01
0.1
1
10
100
1000
Conc.nM
16C18
61C7F12
67A11B11
69E3H11
95F2C2F12
103G6G1
Isotype

FIG. 1A-B

A
Binding to hCDH16-his protein
OD450
2.5
2.0
1.5
1.0
0.5
0.0
0.001
0.01
0.1
1
10
100
1000
Conc.nM
20C3E8
29D2D7
29H8D3
120B10C5
134C7B1
143H10E4
152A1D12
155B11C6
Isotype
B
Binding to hCDH16-his protein
OD450
Conc.nM
16C18
61C7F12
67A11B11
69E3H11
95F2C2F12
103G6G1
Isotype

FIG. 2A-B

A
Binding to HEK293-hCDH17 cells
Geometric Mean
60000
40000
20000
0
0.001
0.01
0.1
1
10
100
1000
Conc.nM
20C3E8
29D2D7
29H8D3
120B10C5
143H10E4
152A1D12
155B11C6
Isotype
B
Binding to HEK293-hCDH17 cells
16C18
61C7F12
67A11B11
69E3H11
95F2C2F12
103G6G1
Isotype
C
Binding to HEK293 cells
D
Binding to HEK293 cells

FIG. 3A-D

A
Binding to AsPC-1 cells
Geometric Mean
Conc.nM
20C3E8
29D2D7
29H8D3
120B10C5
143H10E4
152A1D12
155B11C6
Isotype
C
Binding to HCT-8 cells
Geometric Mean
Conc.nM
20C3E8
29D2D7
29H8D3
120B10C5
143H10E4
152A1D12
155B11C6
Isotype
E
Binding to AGS cells
Geometric Mean
Conc.nM
20C3E8
29D2D7
29H8D3
120B10C5
143H10E4
152A1D12
155B11C6
Isotype
B
Binding to AsPC-1 cells
Geometric Mean
Conc.nM
16C18
61C7F12
67A11B11
69E3H11
95F2C2F12
103G6G1
Isotype
D
Binding to HCT-8 cells
Geometric Mean
Conc.nM
16C18
61C7F12
67A11B11
69E3H11
95F2C2F12
103G6G1
Isotype
F
Binding to AGS cells
Geometric Mean
Conc.nM
16C18
61C7F12
67A11B11
69E3H11
95F2C2F12
103G6G1
Isotype

FIG. 4A-F

A
Binding to HEK293-cynoCDH17 cells
Geometric Mean
30000
20000
10000
0
0.001
0.01
0.1
1
10
100
1000
Conc.nM
20C3E8
29D2D7
29H8D3
120B10C5
143H10E4
152A1D12
155B11C6
Isotype
B
Binding to HEK293-cynoCDH17 cells
Geometric Mean
30000
20000
10000
0
0.001
0.01
0.1
1
10
100
1000
Conc.nM
16C18
61C7F12
67A11B11
69E3H11
95F2C2F12
103G6G1
Isotype

FIG. 5A-B

A
Binding to AsPC-1 cells
Geometric Mean
15000
10000
5000
0
0.001
0.01
0.1
1
10
100
Conc.nM
29H8D3-z1
29H8D3-z2
29H8D3-z3
29H8D3-z4
29H8D3-z5
29H8D3-z6
29H8D3
Isotype
B
Binding to AsPC-1 cells
Geometric Mean
15000
10000
5000
0
0.001
0.01
0.1
1
10
100
Conc.nM
29H8D3-z7
29H8D3-z8
29H8D3-z9
29H8D3-z10
29H8D3-z11
29H8D3-z12
29H8D3-z13
29H8D3-z14
29H8D3-z15
29H8D3-z16
29H8D3

FIG. 6A-B

C
ELISA binding to human CDH17-his
OD450nm
Conc. (nM)
29H8D3-z12
Isotype mAb
EC50 (nM)
0.187
D
ELISA binding to rhesus CDH17-his
OD450nm
Conc. (nM)
29H8D3-z12
Isotype mAb
EC50 (nM)
0.163
E
ELISA binding to cyno CDH17-hFc
OD450nm
Conc. (nM)
29H8D3-z12
Isotype mAb
EC50 (nM)
0.0169
F
ELISA binding to rat CDH17-his
OD450nm
Conc. (nM)
29H8D3-z12
Isotype mAb
G
ELISA binding to mouse CDH17-his
OD450nm
Conc. (nM)
29H8D3-z12
Isotype mAb
H
ELISA binding to human CDH16-his
OD450nm
Conc. (nM)
29H8D3-z12
Isotype mAb
I
ELISA binding to human CDH9-his
OD450nm
Conc. (nM)
29H8D3-z12
Isotype mAb
J
ELISA binding to human CDH10-his
OD450nm
Conc. (nM)
29H8D3-z12
Isotype mAb
K
ELISA binding to human CDH3-his
OD450nm
Conc. (nM)
29H8D3-z12
Isotype mAb
L
ELISA binding to human CDH6-his
OD450
Conc. (nM)
29H8D3-z12
Isotype mAb
M
Cell binding on HEK293-rhesus CDH17 cells
MFI (Geometric Mean)
Conc. (nM)
29H8D3-z12
Isotype mAb
EC50 (nM)
0.225
N
Cell binding on HEK293-cyno CDH17 cells
MFI (Geometric Mean)
Conc. (nM)
29H8D3-z12
Isotype mAb

FIG. 6C-N

A
Binding to HEK293-hCDH17 cells
Geometric Mean
80000
60000
40000
20000
0
0.001
0.01
0.1
1
10
100
1000
Conc.nM
29D2D7-z1
29D2D7-z2
29D2D7-z3
29D2D7-z4
29D2D7-z5
29D2D7-z6
29D2D7-z7
29D2D7-z8
29D2D7-z9
29D2D7
Isotype
B
Binding to HEK293-hCDH17 cells
Geometric Mean
80000
60000
40000
20000
0
0.001
0.01
0.1
1
10
100
1000
Conc.nM
29D2D7-z10
29D2D7-z11
29D2D7-z12
29D2D7-z13
29D2D7-z14
29D2D7-z15
29D2D7-z18
29D2D7-z19
29D2D7-z24
29D2D7-z25
29D2D7

FIG. 7A-B

Binding to HEK293-hCDH17 cells
Geometric Mean
60000
40000
20000
0
0.001
0.01
0.1
1
10
100
1000
Conc.nM
61C7F12-z1
61C7F12-z2
61C7F12-z3
61C7F12-z4
61C7F12-z5
61C7F12-z6
61C7F12-z7
61C7F12-z8
61C7F12-z9
61C7F12-z10
61C7F12


FIG. 8

Binding to HEK293-hCDH17 cells
50000
40000
30000
20000
10000
0
Geometric Mean
0.001
0.01
0.1
1
10
100
1000
Conc.nM
69E3H11-z6
69E3H11-z7
69E3H11-z8
69E3H11-z9
69E3H11-z10
69E3H11-z12
69E3H11-z14
69E3H11
Isotype

FIG. 9

A
Binding to HCT-8 cells
Geometric Mean
10000
8000
6000
4000
2000
0
0.001
0.01
0.1
1
10
100
1000
Conc.nM
143H10E4-z1
143H10E4-z2
143H10E4-z3
143H10E4-z4
143H10E4-z5
143H10E4-z6
143H10E4
Isotype
B
Binding to HCT-8 cells
Geometric Mean
10000
8000
6000
4000
2000
0
0.001
0.01
0.1
1
10
100
1000
Conc.nM
143H10E4-z7
143H10E4-z8
143H10E4-z9
143H10E4-z10
143H10E4-z11
143H10E4-z12
143H10E4
Isotype

FIG. 10A-B

A
Binding to AGS cells
Geometric Mean
4000
3000
2000
1000
0
0.001
0.01
0.1
1
10
100
1000
Conc.nM
152A1D12-z1
152A1D12-z2
152A1D12-z3
152A1D12-z4
152A1D12-z5
152A1D12-z6
152A1D12-z7
152A1D12-z8
152A1D12-z9
152A1D12
Isotype
B
Binding to AGS cells
Geometric Mean
4000
3000
2000
1000
0
0.001
0.01
0.1
1
10
100
1000
Conc.nM
152A1D12-z10
152A1D12-z11
152A1D12-z12
152A1D12-z13
152A1D12-z14
152A1D12-z15
152A1D12-z16
152A1D12-z17
152A1D12-z18
152A1D12
Isotype

FIG. 11A-B

A
Binding to AGS cells
Geometric Mean
4000
3000
2000
1000
0
0.001
0.01
0.1
1
10
100
1000
Conc.nM
155B11C6-z1
155B11C6-z2
155B11C6-z3
155B11C6-z4
155B11C6-z5
155B11C6-z6
155B11C6-z7
155B11C6-z8
155B11C6-z9
155B11C6
Isotype
B
Binding to AGS cells
Geometric Mean
4000
3000
2000
1000
0
0.001
0.01
0.1
1
10
100
1000
Conc.nM
155B11C6-z10
155B11C6-z11
155B11C6-z12
155B11C6
Isotype

FIG. 12A-B

A
Binding to HCT-8 cells
Geometric Mean
8000
6000
4000
2000
0
0.001
0.01
0.1
1
10
100
1000
Conc.nM
29D2D7-z19p1
29D2D7-z19p2
29D2D7-z19p3
29D2D7-z19
29D2D7
Isotype
B
Binding to HCT-8 cells
Geometric Mean
Conc.nM
29D2D7-z19p3
29D2D7-z19p4
29D2D7-z19p5
29D2D7-z19
29D2D7
Isotype
C
Binding to HCT-8 cells
Geometric Mean
5000
4000
3000
2000
1000
0
Conc.nM
29H8D3-z12p9
29H8D3-z12p10
29H8D3-z12p13
29H8D3-z12p14
29H8D3-z12p15
29H8D3-z12p16
29H8D3-z12
Isotyp
e
D
Binding to HCT-8 cells
Geometric Mean
Conc.nM
29H8D3-z12p17
29H8D3-z12p21
29H8D3-z12p22
29H8D3-z12
Isotype
E
Binding to HCT-8 cells
Geometric Mean
10000
8000
6000
4000
2000
0
Conc.nM
29H8D3-z12p1
29H8D3-z12p3
29H8D3-z12p4
29H8D3-z12p7
29H8D3-z12
29H8D3
Isotype

FIG. 13A-E

F
Binding to LoVo cells
Geometric Mean
5000
4000
3000
2000
1000
0
0.001
0.01
0.1
1
10
100
1000
Conc.nM
69E3H11-z12
69E3H11-z12a19
69E3H11-z12a20
69E3H11-z12a21
69E3H11-z12a26
69E3H11-z12a27
69E3H11-z12a28
G
Binding to HCT-8 cells
Geometric Mean
5000
4000
3000
2000
1000
0
0.001
0.01
0.1
1
10
100
1000
Conc.nM
69E3H11-z12
69E3H11-z12a19
69E3H11-z12a20
69E3H11-z12a21
69E3H11-z12a26
69E3H11-z12a27
69E3H11-z12a28
H
Binding to AsPC-1 cells
Geometric Mean
15000
10000
5000
0
0.001
0.01
0.1
1
10
100
1000
Conc.nM
69E3H11-z12
69E3H11-z12a27
69E3H11-z12a42
Isotype
I
Binding to HCT-8 cells
Geometric Mean
5000
4000
3000
2000
1000
0
0.001
0.01
0.1
1
10
100
1000
Conc.nM
69E3H11-z12
69E3H11-z12a27
69E3H11-z12a42
Isotype

FIG. 13F-I

A
Internalization on AsPC-1 cells
MFI per well(pHAb Reactive Dyes)
3000000
2000000
1000000
0
0
5
10
15
20
25
30
35
40
45
50
55
60
Time (h)
16C8
20C3E8
29D2D7
29H8D3
61C7F12
67A11B11
69E3H11
95F2C2F12
103G6G1
120B10C5
143H10E4
152A1D12
155B11C6
Isotype
B
Internalization on LoVo cells
MFI per well(pHAb Reactive Dyes)
100000
80000
60000
40000
20000
0
0
5
10
15
20
25
30
35
40
45
50
55
60
Time (h)
16C8
20C3E8
29D2D7
29H8D3
61C7F12
67A11B11
69E3H11
95F2C2F12
103G6G1
120B10C5
143H10E4
152A1D12
155B11C6
Isotype

FIG. 14A-B

A
Internalization rate on HCT-8 cells
150000
100000
50000
0
MFI per well(pHAb Reactive Dyes)
0
10
20
30
40
50
Time (h)
29H8D3
29H8D3-z3
29H8D3-z6
29H8D3-z9
29H8D3-z12
Isotype
B
Internalization rate on HCT-8 cells
150000
100000
50000
0
MFI per well(pHAb Reactive Dyes)
0
10
20
30
40
50
Time (h)
155B11C6
155B11C6-z3
155B11C6-z4
155B11C6-z7
Isotype
C
Internalization rate on HCT-8 cells
150000
100000
50000
0
MFI per well(pHAb Reactive Dyes)
0
10
20
30
40
50
Time (h)
152A1D12
152A1D12-z13
152A1D12-z17
152A1D12-z18
Isotype
D
Internalization on HCT-8 cells
50000
40000
30000
20000
10000
0
MFI per well(pHAb Reactive Dyes)
0
10
20
30
Time (h)
29H8D3
29H8D3-z12
29H8D3-z12p1
29H8D3-z12p3
29H8D3-z12p4
29H8D3-z12p7
29H8D3-z12p10
29H8D3-z12p17
29H8D3-z12p21
29H8D3-z12p22
Isotype
E
Internalization on HCT-8 cells
50000
40000
30000
20000
10000
0
MFI per well(pHAb Reactive Dyes)
0
5
10
15
20
25
30
Time (h)
29D2D7
29D2D7-z19
29D2D7-z19p3
Isotype

FIG. 15A-E

A

Mc-vc-PABC-MMAE (Vedotin)

B

Mc-GGFG-DXd (Deruxtecan)

FIG. 16A-B

D

Formula IV

C

Formula III

E

Formula V

FIG. 16C-E

G

Formula VII

F

Formula VI

FIG. 16F-G

A
vc-MMAE labeled α-H IgG secondary antibody based killing
RFU
55000
44000
33000
22000
11000
0
0.00001
0.0001
0.001
0.01
0.1
1
10
Conc.nM
16C18
20C3E8
29D2D7
29H8D3
Isotype
B
vc-MMAE labeled α-H IgG secondary antibody based killing
RFU
55000
44000
33000
22000
11000
0
0.00001
0.0001
0.001
0.01
0.1
1
10
Conc.nM
61C7F12
67A11B11
69E3H11
95F2C2F12
103G6G1
Isotype
C
vc-MMAE labeled α-H IgG secondary antibody based killing
RFU
55000
44000
33000
22000
11000
0
0.00001
0.0001
0.001
0.01
0.1
1
10
Conc.nM
120B10C5
143H10E4
152A1D12
155B11C6
Isotype

FIG. 17A-C

A
CDH17-vc-MMAE cytotoxicity on AsPC1 cells
RFU
1250000
1000000
750000
500000
250000
0
0.0001 0.001 0.01 0.1 1 10 100
Conc(nM)
29D2D7-vc-MMAE
29H8D3-vc-MMAE
29H8D3-z3-vc-MMAE
61C7F12-vc-MMAE
143H10E4-vc-MMAE
152A1D12-vc-MMAE
155B11C6-vc-MMAE
Isotype-vc-MMAE
B
CDH17-vc-MMAE cytotoxicity on LoVo cells
RFU
250000
200000
150000
100000
50000
0
0.0001 0.001 0.01 0.1 1 10 100
Conc(nM)
29D2D7-vc-MMAE
29H8D3-vc-MMAE
29H8D3-z3-vc-MMAE
61C7F12-vc-MMAE
143H10E4-vc-MMAE
152A1D12-vc-MMAE
155B11C6-vc-MMAE
Isotype-vc-MMAE
C
CDH17-vc-MMAE cytotoxicity on AGS cells
RFU
60000
40000
20000
0
0.0001 0.001 0.01 0.1 1 10 100
Conc(nM)
29D2D7-vc-MMAE
29H8D3-vc-MMAE
29H8D3-z3-vc-MMAE
61C7F12-vc-MMAE
143H10E4-vc-MMAE
152A1D12-vc-MMAE
155B11C6-vc-MMAE
Isotype-hIgG1-MMAE

FIG. 18A-C

A
Binding to AsPC-1 cells
MFI (geometric mean)
50000
40000
30000
20000
10000
0
0.001 0.01 0.1 1 10 100 1000
Conc. (nM)
29H8D3-z12-DXd
29D2D7-z19p3-DXd
29H8D3-z12
29D2D7-z19p3
Isotype-DXd
Isotype mAb
B
Binding to AGS cells
MFI (geometric mean)
10000
8000
6000
4000
2000
0
0.001 0.01 0.1 1 10 100 1000
Conc. (nM)
29H8D3-z12-DXd
29D2D7-z19p3-DXd
29H8D3-z12
29D2D7-z19p3
Isotype-DXd
Isotype mAb
C
Binding to KATO III cells
MFI (geometric mean)
400
300
200
100
0
0.001 0.01 0.1 1 10 100 1000
Conc. (nM)
29H8D3-z12-DXd
29D2D7-z19p3-DXd
29H8D3-z12
29D2D7-z19p3
Isotype-DXd
Isotype mAb
D
Binding to HT-29 cells
MFI (geometric mean)
400
300
200
100
0
0.001 0.01 0.1 1 10 100 1000
Conc. (nM)
29H8D3-z12-DXd
29D2D7-z19p3-DXd
29H8D3-z12
29D2D7-z19p3
Isotype-DXd
Isotype mAb

FIG. 19A-D

A
Internalization rate on HCT-8 cells
MFI per well(pHAb Reactive Dyes)
Time (h)
29H8D3-z12-DXd
29H8D3-z12
Isotype
B
24h internalization on HCT-8 cells
Geometric Mean
29H8D3-z12-DXd
29H8D3-z12
Isotype
4°C
37°C
C
Internalization rate on AGS cells
MFI per well(pHAb Reactive Dyes)
Time (h)
29H8D3-z12-DXd
29H8D3-z12
Isotype mAb
D
Internalization rate on AsPC1 cells
MFI per well(pHAb Reactive Dyes)
Time (h)
29H8D3-z12-DXd
29H8D3-z12
Isotype mAb
E
Internalization rate on SW480-hCDH17 cells
MFI per well(pHAb Reactive Dyes)
Time (h)
29H8D3-z12-DXd
29H8D3-z12
Isotype mAb
F
Internalization rate on SW480 cells
MFI per well(pHAb Reactive Dyes)
Time (h)
29H8D3-z12-DXd
29H8D3-z12
Isotype mAb

FIG. 20A-F

A
6 days killing on HCT-8 cells
29H8D3-DXd
29H8D3-z3-DXd
Isotype-DXd
RFU
100000
80000
60000
40000
20000
0
0.0001
0.01
1
100
Conc.nM
B
6 days killing on SW620-hCDH17 cells
29H8D3-DXd
29H8D3-z3-DXd
Isotype-DXd
RFU
400000
300000
200000
100000
0
0.0001
0.01
1
100
Conc.nM
C
120h killing on MDA-MB-468-hCDH17 cells
29H8D3-DXd
29H8D3-z3-DXd
Isotype-DXd
RFU
300000
200000
100000
0
0.0001
0.01
1
100
Conc.nM
D
6 days killing on SW480-hCDH17 cells
29H8D3-DXd
29H8D3-z3-DXd
Isotype-DXd
RFU
300000
200000
100000
0
0.0001
0.01
1
100
Conc.nM

FIG. 21A-D

A
6-days killing on HCT-8 cells
29H8D3-z12-DXd
29D2D7-z19p3-DXd
Isotype-DXd
RFU
Conc. (nM)
B
6-days killing on AGS cells
29H8D3-z12-DXd
29D2D7-z19p3-DXd
RLU
Conc. (nM)
C
6-days killing on MDA-MB-468-CDH17 cells
29H8D3-z12-DXd
29D2D7-z19p3-DXd
RFU
Conc. (nM)
D
6-days killing on SW480-CDH17 cells
29H8D3-z12-DXd
29D2D7-z19p3-DXd
Isotype-DXd
RLU
Conc. (nM)
E
6 days killing on SK-CO-1 cells
29H8D3-z12-DXd
RLU
Conc.(nM)
F
6 days killing on SW620-hCDH17 cells
29H8D3-z12-DXd
RLU
Conc. (nM)
G
6 days killing on LS1034 cells
29H8D3-z12-DXd
RLU
Conc. (nM)
H
6 days killing on Caco-2 cells
29H8D3-z12-DXd
RLU
Conc. (nM)
I
6-days killing on RKO cells
29H8D3-z12-DXd
RLU
Conc. (nM)

FIG. 22A-I

A
Bystander killing on GFP+ SW480-hCDH17 and RFP+ RKO cells
T+ cells /untreated T+ cells%
or T- cells /untreated T- cells %
120
90
60
30
0
Medium
29H8D3-z12-DXd
CDH17+ cells in coculture
CDH17- cells in coculture
CDH17- cells only
B
Bystander killing on GFP+ SW480-hCDH17 and SW480- WT cells
T+ cells /untreated T+ cells%
or T- cells /untreated T- cells %
120
90
60
30
0
Medium
29H8D3-z12-DXd
CDH17+ cells in coculture
CDH17- cells in coculture
CDH17- cells only
C
Bystander killing on GFP+ SW480-hCDH17 and MDA-MB-468-WT cells
T+ cells /untreated T+ cells%
or T- cells /untreated T- cells %
120
90
60
30
0
Medium
29H8D3-z12-DXd
CDH17+ cells in coculture
CDH17- cells in coculture
CDH17- cells only

FIG. 23A-C

A

LoVo transplanted CDX model
BALB/c nude mice (n=6 mice)

Tumor Volume (mm³)
0
200
400
600
800
1000
0
5
10
15
20
Days Post Treatment

Vehicle i.v. D1
Isotype-vc-MMAE, 3 mg/kg i.v. D1
29D2D7-vc-MMAE, 3 mg/kg, i.v. D1
29H8D3-vc-MMAE, 3 mg/kg i.v. D1
29H8D3-z3-vc-MMAE 3 mg/kg i.v. D1
143H10E4-vc-MMAE, 3 mg/kg i.v. D1
152A1D12-vc-MMAE, 3 mg/kg i.v. D1
155B11C6-vc-MMAE, 3 mg/kg i.v. D1

| No. | Groups (N=6) | TGI % |
|---|---|---|
| 1 | Vehicle | / |
| 2 | Isotype-vc-MMAE | 18.96 |
| 3 | 29D2D7-vc-MMAE | 94.69 |
| 4 | 29H8D3-vc-MMAE | 80.45 |
| 5 | 29H8D3-z3-vc-MMAE | 90.63 |
| 6 | 143H10E4-vc-MMAE | 80.97 |
| 7 | 152A1D12-vc-MMAE | 84.87 |
| 8 | 155B11C6-vc-MMAE | 93.66 |

FIG. 24A

B

Vehicle
29D2D7-vc-MMAE
29H8D3-vc-MMAE
29H8D3-z3-vc-MMAE
Isotype-vc-MMAE
143H10E4-vc-MMAE
152A1D12-vc-MMAE
155B11C6-vc-MMAE
Tumor volume (mm³)
Days after administration

FIG. 24B

A
LS1034 CDX model
CB-17 SCID Mice n=4
Tumor Volume (mm3)
Days after administration
Vehicle
29H8D3-z12-DXd, 3mg/kg
29H8D3-z12-DXd, 10mg/kg
TGI=68.45%
TGI=106.07%
B
Vehicle
Tumor volume (mm3)
Days after administration
29H9D3-12-DXd 3mg/kg
Tumor volume (mm3)
Days after administration
29H8D3-z12-DXd 10mg/kg
Tumor volume (mm3)
Days after administration
C
LS1034 CDX model
CB-17 SCID Mice n=4
Body Weight Change (%)
Days after administration
Vehicle
29H8D3-z12-DXd, 3mg/kg
29H8D3-z12-DXd, 10mg/kg

FIG. 25A-C

A
CRC PDX model 1
n=4
Tumor volume (mm³)
Days post administration
H-score 255, 3+
CRC PDX model 2
n=4
Vehicle Control;
29H8D3-z12-DXd, 10mg/kg, i.v.
29D2D7-z19p3-DXd,10mg/kg,i.v.
H-score 105, 2+
CRC PDX model 3
n=4
H-score 230, 3+
CRC PDX model 4
n=4
H-score 165, 2+

| Category | Model 1 | Model 2 | Model 3 | Model 4 |
|---|---|---|---|---|
| CDH17 H-score | 255, 3+ | 105, 2+ | 230, 3+ | 165, 2+ |
| SOC resistance | Yes | | Yes | |
| *TP53 mutation* | *V173M* | *P278S* | *V73Gfs*50* | *G245D* |
| *KRAS mutation* | | *G12D* | | |
| *Gene amplification* | | | *ERBB2* | |
| TGI % at the end of study | | | | |
| 29H8D3-z12-DXd | 107.2 | 104.4 | 98.8 | 108.6 |
| 29D2D7-z19p3-DXd | 113.0 | 103.3 | 108.0 | 113.3 |

FIG. 26A

Vehicle Control
29H8D3-z12-DXd, 10mg/kg, i.v.
29D2D7-z19p3-DXd, 10mg/kg, i.v.
CRC PDX model 2
n=4
CRC PDX model 4
n=4
Relative Change of Body Weight(%)
Days post administration

B
CRC PDX model 1
n=4
CRC PDX model 3
n=4
Relative Change of Body Weight(%)
Days post administration

FIG. 26B

CRC PDX model 1
n=4
Tumor volume (mm³)
1800
1500
1200
900
600
300
0
0
7
14
21
28
Days post administration
Vehicle
29H8D3-z12-DXd;3mg/kg
29H8D3-z12-DXd;10mg/kg
H-score 255, 3+
400X
CRC PDX model 2
n=4
Tumor volume (mm³)
2500
2000
1500
1000
500
0
Days post administration
H-score 105, 2+
400X
CRC PDX model 3
n=4
Tumor volume (mm³)
Days post administration
H-score 165, 2+
400X

Tumor growth inhibition (TGI) summary (Day 21)

| Groups | CRC PDX model 1 | CRC PDX model 2 | CRC PDX model 3 |
|---|---|---|---|
| 29H8D3-z12-DXd, 3 mpk | 79.0 % | 66.5 % | 54.5 % |
| 29H8D3-z12-DXd, 10 mpk | 100.2 % | 98.6 % | 98.1% |

**FIG. 27**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005035572 A
- WO 03035694 A
- WO 0029004 A
- WO 02051870 A
- US 5892019 A
- US 4816567 A
- US 5693762 A
- US 5773001 A
- US 7659241 B
- US 7498298 B
- US 5208020 A
- US 10195288 B
- US 8420086 B
- US 9889207 B
- US 6150584 A
- US 6458592 B
- US 6420140 B


### Non-patent literature cited in the description


- *Nature*, 1989, vol. 341, 544-546
- *Dev Comp Immunol*, 2006, vol. 30, 43-56
- *Trend Biochem Sci*, 2001, vol. 26, 230-235
- *Trends Biotechnol*, 2003, vol. 21, 484-490
- *Febs Lett*, 1994, vol. 339, 285-290
- **HAMERS-CASTERMAN et al.** *Nature*, 1993, vol. 363, 446-448
- **KABAT, E et al.** Sequences of Proteins of Immunological Interest. *U.S. Department of Health and Human Services*, 1983
- **CHOTHIA** ; **LESK**. *J. Mol. Biol.*, 1987, vol. 196, 901-917
- **KABAT et al.** Sequences of Proteins of Immunological Interest. *U.S. Dept. of Health and Human Services*, 1983
- **CHOTHIA et al.** *J. Mol. Biol.*, 1987, vol. 196, 901-917
- **KABAT et al.** Sequence of Proteins of Immunological Interest. *U.S. Dept. of Health and Human Services*, 1983
- **BURTON**. *Molec. Immunol.*, 1985, vol. 22, 161-206
- **ROUX et al.** *J. Immunol*, 1998, vol. 161, 4083
- **ALTSCHUL S. F. et al.** *J. Mol. Biol.*, 1990, vol. 215, 403-410
- **STEPHEN F. et al.** *Nucleic Acids Res.*, 1997, vol. 25, 3389-3402
- **HIGGINS D. G. et al.** *Methods in Enzymology*, 1996, vol. 266, 383-402
- **LARKIN M. A. et al.** *Bioinformatics (Oxford, England)*, 2007, vol. 23 (21), 2947-8
- **JONES et al.** *Nature*, 1986, vol. 321, 522-525
- **RIECHMANN et al.** *Nature*, 1988, vol. 332, 323-329
- **PRESTA**. *Curr. Op. Struct. Biol.*, 1992, vol. 2, 593-596
- **RIECHMANN et al.** *Nature*, 1988, vol. 332, 323-327
- **VERHOEYEN et al.** *Science*, 1988, vol. 239, 1534-1536
- **MARKS et al.** *Rio/Technology*, 1992, vol. 10, 779-783
- **BARBAS et al.** *Proc Nat. Acad. Sci. USA*, 1994, vol. 91, 3809-3813
- **SCHIER et al.** *Gene*, 1995, vol. 169, 147-155
- **YELTON et al.** *J. Immunol.*, 1995, vol. 155, 1994-2004
- **JACKSON et al.** *J. Immunol.*, 1995, vol. 154 (7), 331 0-15 9
- **HAWKINS et al.** *J. Mol. Biol.*, 1992, vol. 226, 889-896
- **FU et al.** *Signal Transduction and Targeted Therapy*, 2022, vol. 7, 93
- **KHONGORZUL et al.** *Mol Cancer Res*, 2020, vol. 18 (1)